# EUROPEAN PATENT APPLICATION

(11) **EP 4 403 547 A2**
(43) Date of publication of application: **24.07.2024**
(21) Application number: 24178775.3
(22) Date of filing: 27.05.2021
(51) Int. Cl.: C07C 69/30

(54) **PROCESS FOR PRODUCING HIGH-GRADE TRICAPRYLIN**

(30) Priority: 21.05.2021 WO PCT/EP2021/063583
(62) Divisional of application: 21728906.5
(71) Applicant: IOI Oleo GmbH, 58453 Witten (DE)
(72) Inventor: LOCHMANN, Dirk, 58453 Witten (DE); REYER, Sebastian, 58453 Witten (DE); STEHR, Michael, 58453 Witten (DE)
(74) Representative: Von Rohr Patentanwälte Partnerschaft mbB

(57) **Abstract**

The present invention relates to a process for producing high-grade tricaprylin and to the tricaprylin thus prepared as well as to its respective uses and applications.

## Description

The present invention relates to the technical field of fatty acid polyol esters, particularly fatty acid glycerol esters, and related metabolisms and medical therapy concepts of related diseases.

Especially, the present invention relates to a process for producing high-grade tricaprylin (which is synonymously also called trioctanoylglyceride, tricaprylyl glycerol, glycerol trioctanoin, tricapryloyl glycerol, octanoic acid-1,2,3-propanetriyl-ester, glycerin tricaprylate, tricaprylyl glycerin, glycerol tricaprylate, glycerol trioctanoate, glyceryl tricaprylate, caprylic triglyceride, tricaprilin, tricaprylyl glycerol etc.) as well as to the inventive reaction product thus obtainable or thus prepared (i.e. tricaprylin) and to its respective uses and applications, especially in pharmaceutical compositions, such as drugs or medicaments, or in food, in food or nutritive compositions in food products and/or in medical foods, as well as to its further applications or uses.

Furthermore, the present invention relates to pharmaceutical compositions, especially drugs or medicaments, comprising the inventive reaction product (i.e. tricaprylin) obtainable or produced according to the inventive process, usually together with a physiologically acceptable excipient, as well as to their respective applications or uses.

Moreover, the present invention relates to food, nutritive or food compositions, food products and/or medical foods, especially to food supplements, functional foods, novel foods, food additives, dietary foods, power snacks, appetite suppressants and strength and/or endurance sports supplements, which comprise the inventive reaction product (i.e tricaprylin) obtainable or produced according to the inventive process, as well as to their applications or uses.

Further, the present invention relates to the use of the inventive reaction product (i.e. tricaprylin) as an additive or aid, especially as a carrier or excipient, a solubilizer, a release agent, a surface-treatment agent, a transport agent, a lubricant, hydrophobic agent, a film-forming or protective agent and a viscosity regulator, preferably as a carrier or excipient, in pharmaceutical compositions, especially drugs or medicaments, in food, nutritive or food compositions, food products and/or medical foods as well as in cosmetic compositions.

Finally, the present invention also relates to the use a nucleophilic agent for purifying tricaprylin.

In the human energy metabolism, glucose is the short-term available energy carrier, which is metabolized into energy in the mitochondria by releasing water and carbon dioxide. The glycogen stores of the liver are already emptied during the sleep period during the night. However, especially the human central nervous system (CNS) and the heart require a permanent energy supply.

The physiological alternative to glucose, which is mainly available to the central nervous system, are the so-called ketone bodies (synonymously also called keto bodies).

The term ketone body is especially a collective term for three compounds, which are formed mainly in catabolic metabolic states (such as hunger, reduction diets or low-carbohydrate diets) and may lead to ketosis. The term ketone bodies includes especially the three compounds acetoacetate (synonymously also referred to as acetacetate) and acetone as well as 3-hydroxybutyric acid (hereinafter also synonymously referred to as beta-hydroxybutyric acid or BHB or 3-BHB) or its salt (i.e. 3-hydroxybutyrate or beta-hydroxybutyrate), the latter being the most important of the three aforementioned compounds. 3-Hydroxybutyric acid or its salt occurs physiologically as the (R)-enantiomer, i.e. as (R)-3-hydroxybutyric acid (synonymously also called (3R)-3-hydroxybutyric acid to emphasize the centre of chirality in the 3-position) or its salt.

These ketone bodies are also provided physiologically in large amounts from lipids stored in the body by lipolysis during fasting or starvation and replace the energy source glucose almost completely.

The ketone bodies are formed in the liver from acetyl coenzyme A (= acetyl-CoA), which originates from beta-oxidation; they represent a transportable form of the acetyl coenzyme A in the human body. However, in order to utilize the ketone bodies, the brain and muscles must first adapt by expressing enzymes that are required to convert ketone bodies back into acetyl coenzyme A. Especially in times of hunger, the ketone bodies contribute a considerable amount to energy production. For example, after some time the brain is able to get by with only a third of the daily amount of glucose.

Physiologically, the ketone bodies are synthesized from two molecules of activated acetic acid in the form of acetyl coenzyme A, the normal intermediate product of fatty acid degradation, which is extended using a further acetyl coenzyme A unit and the enzyme HMG-CoA-synthase to the intermediate product 3-hydroxy-3-methyl-glutaryl-CoA (HMG-CoA), wherein finally the HMG-CoA-lyase cleaves off the acetoacetate. These three steps take place exclusively in the mitochondria of the liver (i.e. Lynen cycle), wherein 3-hydroxybutyrate is finally formed in the cytosol by the D-beta-hydroxybutyrate dehydrogenase. HMG-CoA is also an end product of the degradation of the amino acid leucine, while acetoacetate is formed during the degradation of the amino acids phenylalanine and tyrosine.

Spontaneous decarboxylation turns acetoacetate into acetone; it can occasionally be perceived in the breath of diabetics and dieters. It cannot be further used by the body. However, the proportion of acetone in the ketone bodies is small.

Acetoacetate is thus reductively converted into the physiologically relevant form of 3-hydroxybutyric acid or 3-hydroxybutyrate, but can also decompose into the physiologically unusable acetone with the release of carbon dioxide, which is detectable and olfactory perceptible in severe ketosis, a ketoacidosis (e. g. in diabetes mellitus type 1 patients without insulin substitution), in the urine and in the exhaled air.

3-Hydroxybutyric acid is currently used and marketed in the weight training sector as a sodium, magnesium or calcium salt.

However, 3-hydroxybutyric acid is not known or only in very small quantities to humans in evolutionary terms, since plants do not produce 3-hydroxybutyric acid and 3-hydroxybutyric acid in the animal organism only occurs in dead emaciated animals in ketosis, so that 3-hydroxybutyric acid causes nausea when administered orally. 3-Hydroxybutyric acid in the form of free acid and its salts also taste very bitter and can cause severe vomiting and nausea.

Moreover, patients, especially newborns, but also adults cannot permanently tolerate large amounts of salts of 3-hydroxybutyric acid, as these compounds can have a kidney-damaging effect.

In addition, the plasma half-life of 3-hydroxybutyric acid and its salts is so short that even if several grams are taken, the ketosis lasts only for about three to four hours, i.e. patients cannot benefit continuously from a therapy with 3-hydroxybutyric acid or its salts, especially at night. In case of metabolic diseases this can lead to life-threatening situations.

Therefore, in the case of the therapy of such metabolic diseases, fatty acid polyol esters, particularly fatty acid glycerol esters, and in particular so-called medium-chain triglycerides (i.e. so-called MCTs or MCT fats/oils) are currently used for ketogenic therapy, i.e. particularly the metabolic conversion of caproic, caprylic and capric acid (i.e. saturated linear C₆-, C₈- and C₁₀-fatty acids) from the corresponding triglycerides is intended.

Medium-chain triglycerides (synonymously also called MCTs, MCT fats, MCT oils or the like) are triglycerides with two or three fatty acids having a linear saturated aliphatic tail of five to twelve carbon atoms, especially six to twelve carbon atoms, i.e. medium-chain fatty acids (MCFAs). Rich food sources for commercial extraction of MCTs include palm kernel oil and coconut oil.

Thus, according to this definition, medium-chain triglycerides (MCTs) are triglycerides containing medium-chain fatty acids. Medium-chain fatty acids particularly and inter alia include caproic (C 6:0), caprylic (C 8:0), capric (C 10:0) and lauric (C 12:0) acids. These acids are saturated fatty acids, which are mainly found in tropical vegetable fats such as coconut oil (approximately 60 %) and palm kernel oil (approximately 55 %) and in butter. To a small extent, they are also found in milk fat (approximately 10 %). Pure MCT oil does not occur in nature but only in mixtures with other triglycerides. MCTs have a slightly lower calorific value or a lower food energy (i.e. 3475 kJ / 100 g) if compared to fat (3852 kJ / 100 g) and also a lower smoke-point if compared to conventional long-chain fats (i.e. LCTs = long-chain triglycerides).

MCT fats are industrially obtained by hydrolysis of coconut oil and palm kernel oil, fractionation of the medium-chain fatty acids and subsequent esterification with glycerol. A pure MCT oil is colourless to yellowish, neutral in odour and taste and of very low viscosity. It is declared as a vegetable oil (i.e. neutral oil or oleum neutrale).

The composition of MCT fats according to the German Pharmacopoeia, 10th Edition, 1991 (i.e. Deutsches Arzneibuch 10 or DAB 10) and corresponding European Pharmacopoeia (Ph. Eur., particularly Ph. Eur. 10.0, Monograph No. 0686 "Triglycerides, medium-chain [Triglycerida saturata media]") is as follows: 50 to 80 % of caprylic acid Cs, 25 to 45 % of capric acid C₁₀, at most 3 % of lauric acid C₁₂ and at most 2 % of caproic acid C₆ (cf. also: Hagers Handbuch der Pharmazeutischen Praxis [Hager's Handbook of Pharmaceutical Practice], 5th Edition, ISBN 978-3-642-63389-8, pages 1059 seq.).

MCTs find a wide range of uses and dosage forms. MCT fats have been produced industrially since 1955 and are used in a variety of applications due to their specific properties. With the GRAS application (generally recognized as safe petition) accepted by the U.S. Food and Drug Administration (FDA) in 1994, MCTs gained interest not least in the food sector. Due to their physical characteristics, the industrially obtained MCT fats also find application in the production of cosmetics (e.g. in ointments, creams, bath oils etc.) and pharmaceuticals (e.g. in tablets, coated tablets etc.), where they may serve particularly as carriers or excipients, solubilizers, release agents, surface-treatment agents, transport agents, lubricants, hydrophobic agents, film-forming and protective agents and viscosity regulators. Furthermore, due to their metabolic peculiarities, they are also used in the form of dietetic foods (e.g. MCT oil and MCT margarine) and products for artificial nutrition (e.g. enteral and parenteral). These products are categorized as dietetic foods for special medical purposes (e.g. balanced diets) according to the German Dietary Products Ordinance (i.e. so-called German "Diätverordnung").

Also, the physiological properties and metabolism of MCTs is of high interest: Due to their shorter fatty acid chain length, MCT fats are relatively soluble in aqueous environments and can therefore be metabolized without bile acids. Their structure also does not require cleavage by pancreatic lipase (i.e. enzyme of the pancreas). They are transported directly in the blood to the liver, bypassing the lymphatic system, where they are preferentially oxidized if compared to conventional fats and the formation of ketone bodies is thus increased. The transport of medium-chain fatty acids (MCFAs) to the mitochondria (i.e. the site of fatty acid oxidation) occurs independently of carnitine. The tolerable daily intake varies individually and is about 50 to about 100 g or more of MCT fats. In order to avoid side-effects (such as diarrhoea, cramps, and headaches) it is common to start with about 20 g of MCT fat per day. This amount can be gradually increased by 5 to 10 g per day. When following a diet with MCT fats, it is essential to ensure the supply of fat-soluble vitamins and essential fatty acids (i.e. omega-3 and omega-6). Fat-soluble vitamins are sufficiently absorbed when MCT fats are used.

Due to their metabolic characteristics, MCT fats are a valuable component of nutritional therapy in the dietetics of various clinical indications.

Medium-chain triglycerides were first used in clinical nutrition in the 1950s for the dietary treatment of malabsorption syndrome, as they are easily soluble in an aqueous environment and are absorbed by the body more quickly than conventional long-chain fats. In cases of lymphangiectasia (i.e. dilatation of lymphatic vessels), Whipple's disease (i.e. a rare infectious disease) and chylothorax (i.e. accumulation of lymphatic fluid in pleural cavity), MCT fats can help relieve congested lymphatic vessels. In premature infants, medium-chain fats are used to achieve weight gain due to the not yet fully developed digestive system.

Since MCT fats can be metabolised independently of pancreatic enzymes and only need to be broken down by gastric lipase, their use is indicated in cases of existing exocrine pancreatic insufficiency with marked steatorrhea (i.e. fatty stools or presence of fat in feces) if enzyme supplementation does not produce the desired effect. Exocrine pancreatic insufficiency occurs, for example, in chronic pancreatitis and cystic fibrosis. Furthermore, their use is also useful in combination with pancreatic supplements. Another classical field of application for MCT fats is short bowel syndrome, in which, depending on the extent and the section of intestine removed, there are digestive disorders, especially of edible fats. If the large intestine is preserved, medium-chain fatty acids can be sufficiently absorbed and represent a suitable alternative to conventional fats. In rare inborn errors of metabolism, such as the defect of β-oxidation of long-chain (LCHAD) and very long-chain fatty acids (VLCAD), medium-chain fatty acids are an essential source of energy. MCT fats are also used in artificial nutrition (i.e. enteral and parenteral) for various diseases, mainly of the gastrointestinal tract.

Since MCT fats have a higher ketogenicity than conventional fats, they are also used in the ketogenic diet (KD), especially as a partial replacement of LCT fats. The ketogenic diet finds application e.g. in pharmacoresistant epilepsy and in some congenital rare metabolic diseases (e.g. pyruvate dehydrogenase deficiency, GLUT 1 defect). The nutrient ratio of the classic form of ketogenic diet is usually 3 to 4 parts fat and one part carbohydrate and protein. Ketogenic diet with MCT fats can be considered a promising alternative to the classical form of diet in children and adults since the higher ketogenicity of MCT fats can shift the ratio of fat to carbohydrate and protein in favour of carbohydrate. Ketogenic diet is also used in the treatment of patients with brain tumors, Parkinson's disease, migraine, Alzheimer's disease and NAFLD (i.e. non-alcoholic fatty liver disease/ hepatitis). Due to the metabolic properties of MCT fats and their lower energy content compared to conventional fats, MCT fats are also used in the field of weight management and metabolic syndrome.

The use of MCT fats in the nutritional therapy of various diseases is to be distinguished from their use in sports nutrition, where they are known as a fast energy supplier and are mainly used in endurance sports.

Medium-chain fatty acids are contraindicated in cases where there is a risk of ketoacidosis, as well as in cases of oxidation disorders of medium-chain fatty acids (MCAD deficiency or MCADD or medium-chain acyl-CoA dehydrogenase deficiency). Due to their low smoke-point, medium-chain fats have only limited heat-resistance, so that manufacturer's information and instructions in this regard should be followed to avoid increased smoke.

Specifically, one particular inventive fatty acid polyol ester, especially fatty acid glycerol ester, of special interest is tricaprylin (which is synonymously also called trioctanoylglyceride, tricaprylyl glycerol, glycerol trioctanoin, tricapryloyl glycerol, octanoic acid-1,2,3-propanetriyl-ester, glycerin tricaprylate, tricaprylyl glycerin, glycerol tricaprylate, glycerol trioctanoate, glyceryl tricaprylate, caprylic triglyceride, tricaprilin, tricaprylyl glycerol etc.) Tricaprylin is the triglyceride of caprylic acid which is usually manufactured by esterification of caprylic acid with glycerin. Tricaprylin occurs as a clear, colourless to pale-yellow liquid. It forms crystals from acetone/ethanol (95 %). Tricaprylin is odourless.

Tricaprylin is used e.g. in pharmaceutical preparations, for example as a neutral carrier or excipient, as an absorption promoter and as a solubilizer for active drugs. It is also used as an oily phase to prepare water-in-oil-in-water multiple emulsions for incorporating water-soluble drugs and also for obtaining stable microcapsules. Tricaprylin also acts as a vehicle for topical creams and lotions as well as cosmetic preparations. It is also used as a penetration-enhancing lipid base with excellent emollient and skin-smoothing properties. Owing to its non-greasy components and low viscosity, it has very good spreadability. In spite of being skin-permeable, tricaprylin does not obstruct natural skin respiration and hence it is used in baby oils, massage oils and face masks. It is an excellent dispersant and acts as a solubilizer, wetting agent and binder in colour cosmetics. Being readily miscible with natural oils and surfactants, tricaprylin is also used as fat component in two-phase foam baths. It is furthermore used in sunscreen creams and oils because of its compatibility with organic and inorganic filter agents. It is also used as a fixative for perfumes and fragrances.

However, as already indicated hereinabove, fatty acid polyol esters, particularly fatty acid glycerol esters, and in particular so-called medium-chain triglycerides (MCTs) and, above all, also tricaprylin have also been used as active ingredients or active compounds in pharmaceutical compositions.

Specifically, a tricaprylin-containing composition was previously marketed under the tradename Axona as a medical food for the clinical dietary management of the impairment of metabolic processes associated with mild to moderate states of Alzheimer's disease. The marketed formulation comprises tricaprylin as fractionated palm kernel oil. During digestion, the caprylic triglyceride is broken down into ketones, which provide an alternative energy source for the brain. Its use is based on the fact that the ability of the brain to use its normal energy source (i.e. glucose) is impaired in Alzheimer's disease.

Consequently, in the prior art there exists a high demand for and an increasing interest in fatty acid polyol esters, particularly fatty acid glycerol esters, such as medium-chain triglycerides (MCTs), especially tricaprylin but also others. Above all, in pharmaceutical and food industry there exists an increasing demand for high-grade fatty acid polyol esters, particularly high-grade fatty acid glycerol esters, with high degrees of active substance and low degrees of impurities, especially toxic impurities.

However, conventional production methods for manufacturing fatty acid polyol esters, particularly fatty acid glycerol esters, such as medium-chain triglycerides (MCTs), especially tricaprylin but also others, including the subsequent multistage refinement treatment (above multistage distillation at elevated temperatures), lead to relatively high degrees of impurities and by-products particularly due to high process temperatures needed and the used starting materials and reagents, particularly halogen-containing substances (e.g. originating from hydrochloric acid HCl or metal-based catalysts used in the esterification process). Especially, when triglycerides with low acid and hydroxyl values and high esterification degrees are required, the mandatory drastic esterification conditions, especially high temperatures over 180 °C and up to 230 °C, the necessary subsequent multistage refinement treatment (above multistage distillation at elevated temperatures) and the use of metal-based catalysts lead to the formation of high contents of toxic by-products and impurities, especially toxic halide-based by-products and impurities.

Above all, conventional production methods for manufacturing fatty acid polyol esters, particularly fatty acid glycerol esters, including the subsequent multistage refinement treatment (above multistage distillation at elevated temperatures), usually produce also relatively high levels of impurities and by-products, especially toxic impurities and by-products, such as, for example, genotoxic glycidol esters of fatty acids (synonymously also called glycidyl esters of fatty acids or simply GEs of fatty acids or only GEs) and nephrotoxic fatty acid esters of monochloropropanediols (synonymously also called MCPD fatty acid esters or simply MCPD esters), especially fatty acid esters of 3-monochloropropanediol (3-MCPD fatty acid esters or 3-MCPD esters) and fatty acid esters of 2-monochloropropanediol (2-MCPD fatty acid esters or 2-MCPD esters).

The following exemplary reaction scheme illustrates the formation of the three aforenamed toxic by-products, i.e. 3-MCPD fatty acid ester, 2-MCPD fatty acid ester and glycidol fatty acid ester for the triglyceride tricaprylin selected as a non-limiting example, which formation occurs when high temperatures (e.g. temperatures of 200 °C and more) are applied to the fatty acid triglyceride (e.g. tricaprylin) in the presence of chlorine-containing substances originating from the production process. The substance 3-chloro-1,2-propanediol originates from glycerine when reacting to 3-MCPD in the presence of chlorine-containing substances or chlorides, respectively.

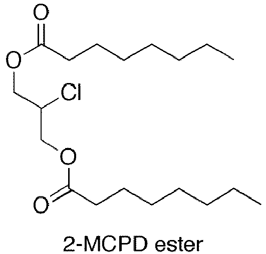

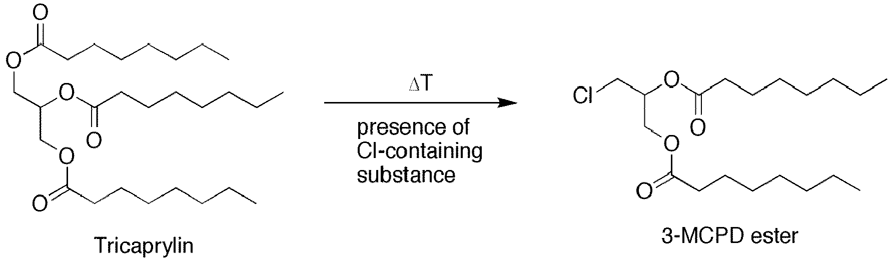

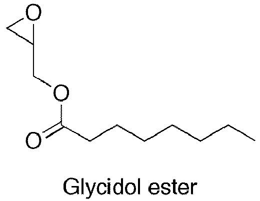

Above all with respect to pharmaceutical and nutritional applications, controlling the impurity profile is crucial and compulsory, especially for known toxic by-products such as the three aforenamed toxic by-products, i.e. 3-MCPD fatty acid ester, 2-MCPD fatty acid ester and glycidol fatty acid ester.

Although it has been well known for decades that, e.g. due to the harsh process conditions occurring during the conventional esterification/condensation methods of fats and oils, these undesired and toxic by-products are formed, no attempts have been made to avoid such harsh conditions by improving or modifying the production processes. For, it is known that mild esterification conditions using biobased catalysts and lower esterification temperatures reduce the formation of toxic by-products, such as e.g. the three aforenamed toxic by-products, i.e. 3-MCPD fatty acid ester, 2-MCPD fatty acid ester and glycidol fatty acid ester, but at the same time only lead to triglycerides with undesirably high acid and hydroxyl values and relatively low triglyceride contents at low conversion rates and yields.

Consequently, instead of trying to avoid the formation of undesired and toxic by-products, rather cost-intensive and burdensome pre- and/or post-treatment methods are effected in order to remove these undesired and toxic by-products from the desired final product obtained under harsh esterification conditions (i.e. especially high temperatures over 180 °C and up to 230 °C and use of metal-based catalysts etc.). Such burdensome post-treatment methods comprise, inter alia, e.g. the addition of strongly alkaline reagents such as KOH, multistage distillation cascades, heat-treatment with strongly alkaline aqueous solutions etc. (e.g. WO 2019/038320 A1, WO 2014/012548 A1 etc.), which post-treatment methods also affect and deteriorate the product quality as such and overall yields and efficiency. For instance, WO 2021/070209 A1 discloses a complex multistage extraction process for the purification of triglycerides, using a saline / organic solvent two-phase extraction system also with chlorinated organic solvents.

However, these post-treatment methods mostly also fail to remove last traces of these undesired and toxic by-products, which traces will then remain in the final product. This is of particular danger when applying high dosages of the fatty acid polyol esters, particularly fatty acid glycerol esters, e.g. MCTs such as tricaprylin, in pharmaceutical and nutritional applications (above all in case of parenteral application), which leads to an undesired accumulation of such traces of undesired and toxic by-products in the human body, thus also exceeding the authorised threshold levels of the undesired and toxic by-products as regulated by law.

Moreover, such cost-intensive and burdensome post-treatment methods which aim to effect a only subsequent or retroactive removal of the undesired and toxic by-products are also inefficient since these post-treatment methods often lead to an undesired entrainment of product and do also not increase or influence product quality or product purity such as the esterification degree or the residual content of other impurities (such as e.g. the total halogenated substance content or the total acid content).

For instance, the European Union (EU) has strictly limited the concentration of such toxic by-products and impurities for food-applications by respective legal regulations (e.g. EU Regulations Nos. 2020/1322 and 2018/290), especially for infant application. For example, the maximum permissible content of nephrotoxic 3-MCPD fatty acid esters in liquid infant formula, follow-on formula and foods for special medical purposes intended for infants and young children and young children formula is 15 mg / kg (i.e. 15 ppm) only while the maximum permissible content of genotoxic glycidol fatty acid esters for same formulations is even 6.0 mg / kg (i.e. 6.0 ppm) only. Consequently, the allowed exposure levels of these toxic by-products in nutritional applications are very restricted.

Accordingly, especially for monographed APIs (i.e. Active Pharmaceutical Ingredients) like e.g. MCT oils with a total daily intake of far greater than 10 g / day it is challenging to constantly stay below those maximum exposure levels. In case of parenteral application this will be even more challenging since the toxic substances may directly enter the human body.

A permanent or an even lifelong medication with fatty acid polyol esters, particularly fatty acid glycerol esters, such as medium-chain triglycerides (MCTs), especially tricaprylin but also others, can comprise daily dosages or intakes of even up to 100 g / day. For this purpose, particularly pure grades are required. In contrast to nutritional applications, in case of pharmaceutical applications patients suffering from serious diseases are taking this medication, whose state of health is impaired anyway, so that their level of tolerance is very low, also in view of toxic impurities.

Therefore, it would be desirable to conceive efficient production processes which directly produce high-grade fatty acid polyol esters, particularly fatty acid glycerol esters, such as MCTs (e.g. tricaprylin) etc., having high degrees of purity or minimum levels of by-products and impurities, respectively, as well as exhibiting at the same time high esterification degrees and good stabilities, especially storage-stabilities, and high active ingredient contents, especially without the necessity of additional cost-intensive and burdensome post-treatment operations.

Consequently, the prior art has not lacked attempts to find efficient production processes but failed to improve the efficiency and performance of existing processes. But so far, however, no efficient production processes have been conceived in the prior art. Also, access to such high-grade fatty acid polyol esters, particularly fatty acid glycerol esters, such as MCTs (e.g. tricaprylin) etc., is not or not easily possible according to the prior art.

The problem underlying the present invention is thus providing an efficient process for producing triglycerides of fatty acids (i.e. fatty acid glycerol triesters), above all tricaprylin, especially with a high purity degree, wherein the aforementioned disadvantages and/or drawbacks of the prior art should be at least partially avoided or even at least essentially overcome.

Such production process should especially make the respective fatty acid polyol esters, particularly fatty acid glycerol esters, such as MCTs (above all tricaprylin) etc., accessible in an efficient way, especially with improved qualities and without significant amounts of toxic by-products or impurities, which production process should be feasible on an industrial level, especially on a large-scale level, and without excessive and cost-intensive post-treatment as envisaged in the prior art processes.

In a completely surprising way, applicant has now found out that tricaprylin, especially with a high purity degree, can be directly and efficiently produced, above all in an economic and also industrially feasible way, when applying at least one of the following measurements in the production process: (i) preferably selective derivatization of halide-based impurities present in the resulting reaction product and/or treating or contacting the resulting reaction product or its with at least one nucleophilic agent, (ii) performing and/or running esterification at temperatures not exceeding 180 °C, (iii) using, as starting material, a glycerol or its protected form or precursor, preferably solketal (isopropylidene glycerol), which is at least essentially free of any chlorinated species, (iv) running and/or performing esterification in the absence of any metal-based catalyst. It could not be foreseen that the application of at least one of these measurements or their combination would lead to a significant improvement in the production of tricaprylin.

Thus, in order to solve the problem described hereinabove, the present invention therefore proposes - according to a **first** aspect of the present invention - a process for producing tricaprylin, especially with a high purity degree, according to Claim 1; further, especially special and/or advantageous embodiments of the inventive method are the subject-matter of the respective subclaims.

Furthermore, the present invention relates - according to a **second** aspect of the present invention - to an inventive tricaprylin obtainable or obtained by the inventive process according to the respective independent claim (i.e. Claim 23) and to the inventive tricaprylin according to the further respective independent claim (i.e. Claim 24); further, especially special and/or advantageous embodiments of this aspect of the invention are the subject-matter of the respective subclaims.

Likewise, the present invention - according to a **third** aspect of the present invention - relates to a pharmaceutical composition, especially a drug or medicament, according to the respective independent claim (Claim 26); further, especially special and/or advantageous embodiments of this aspect of the invention are the subject-matter of the respective subclaims.

Furthermore, the present invention - according to a **fourth** aspect of the present invention - also relates to an inventive tricaprylin for the prophylactic and/or therapeutic treatment or for use in the prophylactic and/or therapeutic treatment of diseases of the human or animal body according to the respective independent claims (Claim 29 and Claim 30).

Further, the present invention - according to a **fifth** aspect of the present invention - also relates to an inventive tricaprylin for the prophylactic and/or therapeutic treatment or for producing a medicament for the prophylactic and/or therapeutic treatment of diseases of the human or animal body according to the respective independent claims (Claim 31 to 33).

Furthermore, the present invention - according to a **sixth** aspect of the present invention - relates to a food and/or a nutritive or food composition and/or a food product and/or a medical food according to the respective independent claim (Claim 34); further, especially special and/or advantageous embodiments of the food and/or nutritive or food composition and/or food product and/or medical food according to the invention are the subject-matter of the respective subclaim.

Likewise, the present invention - according to a **seventh** aspect of the present invention - relates to the use of an inventive tricaprylin according to the respective independent claim (Claim 36); further, especially special and/or advantageous embodiments of the use according to the invention are the subject-matter of the respective subclaim.

Also, the present invention - according to an **eighth** aspect of the present invention - furthermore relates to the uses of an inventive tricaprylin according to the respective independent claims (Claims 38 to 42).

Finally, the present invention - according to a **ninth** aspect of the present invention - also relates to the use of a nucleophilic agent for purifying tricaprylin and/or for a preferably selective removal or derivatization of halide-based impurities present in tricaprylin according to the respective independent claim (Claim 43); further, especially special and/or advantageous embodiments of the use according to the invention are the subject-matter of the respective subclaims.

It goes without saying that following features, embodiments, advantages and the like, which are subsequently listed below only with regard to one aspect of the invention for the purpose of avoiding repetition, naturally also apply accordingly to the other aspects of the invention, without this requiring a separate mention.

Furthermore, it goes without saying that individual aspects and embodiments of the present invention are also considered disclosed in any combination with other aspects and embodiments of the present invention and, especially, any combination of features and embodiments, as it results from back references of all patent claims, is also considered extensively disclosed with regard to all resulting combinations possibilities.

With respect to all relative or percentage weight-based data provided below, especially relative quantity or weight data, it should further be noted that within the scope of the present invention these are to be selected by the person skilled in the art such that they always add up to 100 % or 100 % by weight, respectively, including all components or ingredients, especially as defined below; however, this is self-evident for the person skilled in the art.

In addition, the skilled person may, if necessary, deviate from the following range specifications without leaving the scope of the present invention.

In addition, it applies that all values or parameters or the like specified in the following can be determined or identified in principle with standardized or explicitly specified determination methods or otherwise with the determination or measurement methods that are otherwise familiar to a person skilled in the art.

Having stated this, the present invention will be described in more detail hereinafter.

The subject-matter of the present invention - according to a **first** aspect of the present invention - is thus a process for producing tricaprylin (i.e. trioctanoylglyceride or tricaprylyl glycerol), especially with a high purity degree,
wherein 1 ,2,3-propantriol (glycerol, glycerine) of formula (I)

   CH₂(OH)-CH(OH)-CH₂(OH) (I)
or its protected form or precursor, preferably solketal (isopropylidene glycerol), is used as starting material and is reacted and/or converted to tricaprylin,
so as to produce, as a reaction product, tricaprylin of formula (II)

   CH₂[O-C(O)R¹]-CH[O-C(O)R¹]-CH₂[O-C(O)R¹] (II)
wherein in formula (II) the radical R¹ represents a linear saturated aliphatic C₇-alkyl radical of formula - (CH₂)₆ - CH₃ (i.e. a n-heptylic radical),
wherein the process comprises at least one esterification step, especially an esterification step using C₈-fatty acids, especially linear saturated or mono- or poly-unsaturated C₈-fatty acids, and/or their anhydrides and/or esters (preferably C₁-C₄-alkyl esters), preferably at least one esterification step using caprylic acid (i.e. n-octylic acid or 1-heptanecarboxlic acid) of formula CH₃ - (CH₂)₆ - COOH and/or its anhydride or esters (preferably C₁-C₄-alkyl esters);
wherein the process is characterized by at least one of the following features and/or measurements (i) to (iv), especially by a combination of at least two, preferably by a combination of at least three, more preferably by a combination of all four, of the following features and/or measurements (i) to (iv):
   (i) a preferably selective derivatization of halide-based impurities, especially of MCPD fatty acid esters (fatty acid esters of monochloropropanediols, particularly MCPD caprylic acid esters) and glycidol esters of fatty acids (particularly glycidol caprylic acid esters), present in the resulting reaction product is performed and/or the resulting reaction product is treated and/or contacted with at least one nucleophilic agent, especially with a nucleophilic agent capable of removing halide-based impurities, preferably with a nucleophilic agent selected from the group consisting of hydrogen sulfites (bisulfites, HSO₃⁻), sulfites (SO₃²⁻), hydrogen thiosulfates (HS₂O₃⁻), thiosulfates (S₂O₃²⁻), hydrogen phosphites (H₂PO₃⁻), phosphites (HPO₃²⁻) and phosphines as well as combinations thereof, more preferably with hydrogen sulfites (bisulfites, HSO₃⁻), in particular for a preferably selective derivatization of halide-based impurities, especially of MCPD fatty acid esters (fatty acid esters of monochloropropanediols) and glycidol esters of fatty acids;
   (ii) the at least one esterification step is run and/or performed at temperatures not exceeding 180 °C, especially not exceeding 150 °C, particularly not exceeding 120 °C, preferably not exceeding 100 °C, more preferably not exceeding 80 °C, even more preferably not exceeding 50 °C;
   (iii) the 1,2,3-propantriol of formula (I) or its protected form, preferably solketal (isopropylidene glycerol), used as starting material is at least essentially free of any chlorinated species and particularly comprises a total chlorinated species content of at most 1 ppm, especially at most 0.5 ppm, preferably at most 0.1 ppm, based on the 1,2,3-propantriol of formula (I) or its protected form, preferably solketal (isopropylidene glycerol);
   (iv) the at least one esterification step is run and/or performed in the absence of any metal-based catalyst.

As stated above, the applicant has, quite surprisingly, discovered that tricaprylin may thus be produced in an efficient and economic way, particularly with a high purity degree, especially with a quality appropriate for pharmaceutical or clinical applications as well as nutritional applications.

Tricaprylin is thus accessible, for the first time, in a direct and efficient manner with such high purity through the production process according to the invention.

Above all, also very surprisingly, when applying at the last one of the aforenamed features and/or measurements (i) to (iv), the formation of toxic by-products and impurities, such as e.g. genotoxic glycidyl esters of fatty acids and nephrotoxic fatty acid esters of monochloropropanediols such as 3-MCPD fatty acid esters and 2-MCPD fatty acid esters can be efficiently avoided or at least minimized a priori, so that burdensome and cost-intensive post-treatment operations for their removal, as envisaged in the prior art, are not required. Any potential traces of toxic by-products and impurities are easily and efficiently removed by the aforementioned measurement (i), as will be delineated in detail in the following.

For, surprisingly, as applicant has found out in a not expected manner, when applying at least one of the features and/or measurements (i) to (iv), especially a combination of at least two, preferably a combination of at least three, more preferably a combination of all four, of the above features and/or measurements (i) to (iv), tricaprylin with a high purity degree result, i.e. irrespective of any specific reaction route for this substance.

Above all, absolutely surprisingly, when applying at least one of the features and/or measurements (i) to (iv), especially a combination of at least two, preferably a combination of at least three, more preferably a combination of all four, of the above features and/or measurements (i) to (iv), tricaprylin with a high purity degree results, which at the same time unifies a combination of five purity properties (1) to (5), namely: (1) a very low acid value (AV), (2) a very low hydroxyl value (OHV), (3) a very high triglyceride content, (4) a very low total content of MCPD fatty acid esters (fatty acid esters of monochloropropanediols) and (5) a very low total content of glycidol esters of fatty acids. Such combination of purity properties (1) to (5) was deemed incompatible or non-realizable so far in the prior art. However, as applicant has surprisingly found out, when applying at least one of the features and/or measurements (i) to (iv), especially a combination of at least two, preferably a combination of at least three, more preferably a combination of all four, of the above features and/or measurements (i) to (iv), this purity specification can be reached independently from any specific synthesis route.

In this respect, above the following finding by applicant is very surprising: In the prior art, it is mainly the high-temperature post-treatment procedure, especially multistage refinement treatment (above multistage distillation at elevated temperatures), that is deemed to be responsible for the formation of high degrees of impurities and by-products (predominantly caused by glycidol fatty acids esters and MCPD fatty acid esters); for, it is known that refinement treatment under application of heat or high temperatures leads to these toxic by-products. But, as applicant has now surprisingly found out, already the *de-novo* synthesis per se of fatty acid polyol esters, particularly fatty acid glycerol esters, such as medium-chain triglycerides (MCTs), especially tricaprylin but also others, produces high amounts of these undesired toxic by-products when - apart from the necessary subsequent multistage refinement post-treatment (above multistage distillation at elevated temperatures), which already produces higher amounts of these toxic by-products anyway - in addition drastic esterification conditions, especially high temperatures over 180 °C and up to 230 °C combined with the use of metal-based catalysts, are applied in order to achieve low acid and hydroxyl values but also high esterification degrees. Thus, the finding by applicant is completely astonishing that the avoidance intention with respect to these toxic by-products has to be started much earlier in process line (i.e. on behalf of synthesis itself and also already in the upstream selection of the purity grade of the glycerol-based starting materials).

Although much effort has been made in the last decade in finding optimized refinement conditions for natural oils and fats, in particular in palm oil, in order to reduce the content of toxic by-products, above all MCPD and glycidol fatty acid esters, there was no systematic attempt in the prior art to find adequate conditions in the case of a conventional condensation reaction for the manufacturing of synthetic triglycerides. However, a simple transfer of the teachings from the studies related to natural oil refinement is by far not sufficient since these teachings focus exclusively on an a *posteriori* or retroactive impurity reduction of by-products already formed and present in the final products but without taking into account the simultaneously existing need for a high-grade quality (i.e. the necessity of an *a priori* avoidance of the formation of these undesired toxic by-products in the origin of their formation). Furthermore, those prior teachings fail to give any root cause analysis regarding the origin of these toxic by-products, above all MCPD and glycidol fatty acid esters.

Consequently and absolutely surprisingly, as applicant has unexpectedly found out, when applying at least one of the features and/or measurements (i) to (iv), especially a combination of at least two, preferably a combination of at least three, more preferably a combination of all four, of the above features and/or measurements (i) to (iv), the abovedefined purity specification, unifying a combination of the abovenamed five purity properties (1) to (5), can be efficiently reached. Thus, according to the present invention, the aforenamed features and/or measurements, especially the aforenamed features and/or measurements (ii) to (iv), prevent the formation of toxic by-products, such as glycidol fatty acids esters and MCPD fatty acid esters, in its origin by avoiding the cause of their formation while predominantly feature and/or measurement (i) enables the efficient and selective removal of any traces of these toxic by-products nevertheless formed or entrained otherwise.

Thus, for the very first time, applicant has been able to provide a concept for producing high-purity tricaprylin with high yields and conversion rates, which concept is independent from any specific synthesis route. In other words, applicant has been able, for the very first time, to provide a respective concept which is universally for this purpose.

By a preferably selective derivatization of halide-based impurities present in the resulting reaction product and/or treatment of the resulting reaction product with at least one nucleophilic agent, respectively, by selecting relatively mild esterification conditions in the absence of any metal-based catalyst and/or by selecting the purity grade of the glycerol starting material it is surprisingly possible to yield tricaprylin exhibiting a multiple combination of the product properties of high triglyceride contents, low acid and hydroxyl values as well as low total contents of MCPD and glycidol fatty acid esters while avoiding harsh esterification conditions and burdensome and inefficient pre- and/or post-treatment procedures.

Apart from this, the inventive process is at the same time highly flexible and compatible with known prior art production processes and plants for this substance since the aforenamed features and/or measurements (i) to (iv) can be easily implemented in or used to modify known production processes and plants for this substance.

In contrast to this, the prior art production of this substance by means of conventional synthesis processes with burdensome and cost-intensive post-treatment is very complex, expensive and inefficient since prior art synthesis processes produce quite large amounts of the aforenamed toxic by-products and impurities which then have to be separated off from the respective crude product in complex and mostly multi-stage post-treatment operations. Above all, the burdensome and cost-intensive post-treatment operations can also not increase the product quality with respect to high esterification degrees and low acid degrees in the final product. Also the yields of the production process as such cannot be influenced in this way.

Within the scope of the present invention, it was possible for the first time to provide an efficiently working production process with which tricaprylin can be produced with high content degrees of this substance, high esterification degrees and very low acid degrees while at the same time being free or at least essentially free of the aforenamed toxic by-products and impurities.

In addition, the production process according to the invention can be operated economically and can also be implemented on a large scale.

Especially, the inventive production process uses commercially available starting compounds and furthermore allows a relatively simple process management even in case of large-scale implementation.

In contrast to conventional prior art production methods requiring additional complex post-treatment operations, the production process according to the invention does not use such complex post-treatment operations but nevertheless achieves excellent yields, wherein the formation of toxic by-products is minimized or even avoided at all.

In addition, the inventive process is easy to be performed and economical. Especially, the production process according to the invention, especially esterification, is usually carried out in the absence of solvents and/or without any solvent (i.e. as a reaction in mass or as a reaction in substance or as a so-called *bulk reaction*); consequently, the reaction product obtained is thus not contaminated with solvent and no solvent has to be removed and disposed of or recycled in a costly and energy-intensive manner after the process or reaction has been carried out. Furthermore, no or at least essentially no toxic by-products are formed. To sum up, as stated hereinbefore, the present invention thus refers to a process for producing tricaprylin (i.e. trioctanoylglyceride or tricaprylyl glycerol), especially with a high purity degree,
wherein 1 ,2,3-propantriol (glycerol, glycerine) of formula (I)

   CH₂(OH) - CH(OH) - CH₂(OH) (I)
or its protected form or precursor, preferably solketal (isopropylidene glycerol), is used as starting material and is reacted and/or converted to tricaprylin,
so as to produce, as a reaction product, tricaprylin of formula (II)

   CH₂[O-C(O)R¹]-CH[O-C(O)R¹]-CH₂[O-C(O)R¹] (II)
wherein in formula (II) the radical R¹ represents a linear saturated aliphatic C₇-alkyl radical of formula - (CH₂)₆ - CH₃ (i.e. a n-heptylic radical),
wherein the process comprises at least one esterification step, especially an esterification step using C₈-fatty acids, especially linear saturated or mono- or poly-unsaturated C₈-fatty acids, and/or their anhydrides and/or esters (preferably C₁-C₄-alkyl esters), preferably at least one esterification step using caprylic acid (i.e. n-octylic acid or 1-heptanecarboxlic acid) of formula CH₃ - (CH₂)₆ - COOH and/or its anhydride or esters (preferably C₁-C₄-alkyl esters).

As also stated hereinabove already, the inventive process is characterized by at least one of the following features and/or measurements (i) to (iv), especially by a combination of at least two, preferably at least three, of the following features and/or measurements (i) to (iv), which features and/or measurements (i) to (iv) will be explained in more detail hereinafter, namely:
(i) a preferably selective derivatization of halide-based impurities, especially of MCPD fatty acid esters (fatty acid esters of monochloropropanediols, particularly MCPD caprylic acid esters) and glycidol esters of fatty acids (particularly glycidol caprylic acid esters), present in the resulting reaction product is performed and/or the resulting reaction product is treated and/or contacted with at least one nucleophilic agent, especially with a nucleophilic agent capable of removing halide-based impurities, preferably with a nucleophilic agent selected from the group consisting of hydrogen sulfites (bisulfites, HSO₃⁻), sulfites (SO₃²⁻), hydrogen thiosulfates (HS₂O₃⁻), thiosulfates (S₂O₃²⁻), hydrogen phosphites (H₂PO₃⁻), phosphites (HPO₃²⁻) and phosphines as well as combinations thereof, more preferably with hydrogen sulfites (bisulfites, HSO₃⁻), in particular for a preferably selective derivatization of halide-based impurities, especially of MCPD fatty acid esters (fatty acid esters of monochloropropanediols) and glycidol esters of fatty acids;
(ii) the at least one esterification step is run and/or performed at temperatures not exceeding 180 °C, especially not exceeding 150 °C, particularly not exceeding 120 °C, preferably not exceeding 100 °C, more preferably not exceeding 80 °C, even more preferably not exceeding 50 °C;
(iii) the 1,2,3-propantriol of formula (I) or its protected form, preferably solketal (isopropylidene glycerol), used as starting material is at least essentially free of any chlorinated species and particularly comprises a total chlorinated species content of at most 1 ppm, especially at most 0.5 ppm, preferably at most 0.1 ppm, based on the 1,2,3-propantriol of formula (I) or its protected form, preferably solketal (isopropylidene glycerol);
(iv) the at least one esterification step is run and/or performed in the absence of any metal-based catalyst.

As to feature and/or measurement (i), the following is to be emphasized and should be taken into account: According to said feature and/or measurement (i), it is envisaged that a preferably selective derivatization of halide-based impurities, especially of MCPD fatty acid esters (fatty acid esters of monochloropropanediols, particularly MCPD caprylic acid esters) and glycidol esters of fatty acids (particularly glycidol caprylic acid esters), present in the resulting reaction product is performed and/or the resulting reaction product is treated and/or contacted with at least one nucleophilic agent, especially with a nucleophilic agent capable of removing halide-based impurities, preferably with a nucleophilic agent selected from the group consisting of hydrogen sulfites (bisulfites, HSO₃⁻), sulfites (SO₃²⁻), hydrogen thiosulfates (HS₂O3⁻), thiosulfates (S₂O₃²⁻), hydrogen phosphites (H₂PO₃⁻), phosphites (HPO₃²⁻) and phosphines as well as combinations thereof, more preferably with hydrogen sulfites (bisulfites, HSO₃⁻), in particular for a preferably selective derivatization of halide-based impurities, especially of MCPD fatty acid esters (fatty acid esters of monochloropropanediols, particularly MCPD caprylic acid esters) and glycidol esters of fatty acids (particularly glycidol caprylic acid esters).

As applicant has surprisingly found out, when using the aforementioned nucleophilic agents, a preferably selective derivatization of halide-based impurities, especially of MCPD fatty acid esters (fatty acid esters of monochloropropanediols, particularly MCPD caprylic acid esters) and glycidol esters of fatty acids (particularly glycidol caprylic acid esters), is effected. This is even more surprising since these halide-based impurities are usually present in very low amounts only, especially only in traces; nevertheless, the aforedescribed treatment with the aforedefined nucleophilic agents usually leads to a selective derivatization of these substances according to the following reaction scheme, which derivatives can then be easily removed or eliminated, i.e. separated off from the final reaction product (see below explanations).

The following reaction scheme shows, as a merely illustrating but not limiting example, the selective derivatization of halide-based impurities, namely the selective derivatization of a MCPD fatty acid ester (above reaction), on the one hand, and the selective derivatization of a glycidol fatty acid ester (below reaction), on the other hand, wherein sodium hydrogen sulfite (sodium bisulfite, NaHSOs) is used (as a merely illustrating but not limiting example) as a nucleophilic agent and the radical R, independently of each other, denotes a linear saturated C₇-alkyl radical. As may be seen from this reaction scheme, the nucleophilic agent replaces, via a so-called nucleophilic substitution, preferably via a so-called S_{N}2-like reaction mechanism, the organically bonded chlorine atom in the MCPD fatty acid ester and the chlorine atom is removed as sodium chloride, which can be easily separated off with the water-based phase together with the substitution product (above reaction). As may be seen from this reaction scheme, the nucleophilic agent can also react with the glycidol fatty acid ester under ring opening to yield also a water-soluble product (below reaction). Separation of the purified triglyceride product, after separation of the water-based phase, can e.g. be effected via distillation (e.g. short-path distillation).

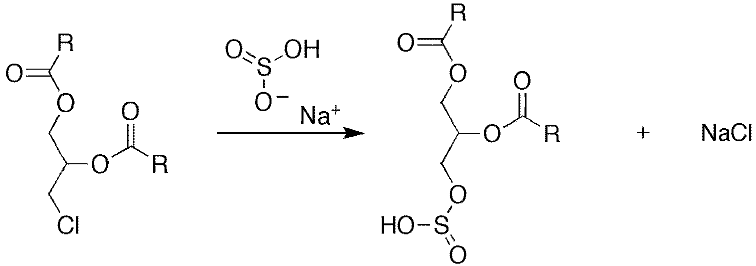

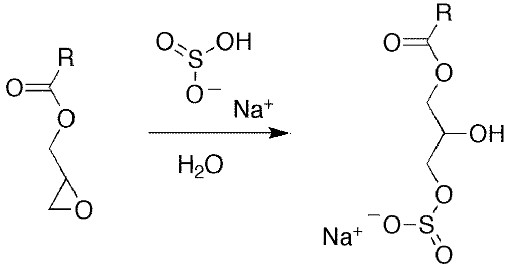

As shown by the above reaction scheme, the halide-based impurities (i.e. MCPD fatty acid esters and glycidol fatty acid esters) can be efficiently removed by selective reaction with the nucleophilic agent (here: e.g. bisulfite = hydrogen sulfite). The halide-based impurities are selectively converted (even if present in traces only) into water-soluble molecules which can then be easily removed, especially by e.g. washing out with water or short-path distillation.

Thus, by the inventive feature and/or measurement (i), halide-based impurities, especially MCPD and glycidol fatty acid esters, can be selectively removed by derivatization, however, without affecting or influencing the other relevant product properties, such as e.g. acid value (AV), hydroxyl value (OHV) and triglyceride content.

Thus, above feature and/or measurement (i) is an efficient means in order to selectively remove toxic halide-based impurities such as e.g. genotoxic glycidol esters of fatty acids and nephrotoxic fatty acid esters of MCPD. The resulting derivatization products can be easily removed, i.e. by washing out or by short-path distillation (wherein the derivatized products usually remain or are kept in the distillation residue (sump) in case of short-path distillation). As also shown in the above reaction scheme, the chlorine atom is preferably converted into harmless sodium chloride, which is easily washed out with the water phase.

Generally, , it is preferred that the above feature and/or measurement (i) is performed as a one-stage or single-step treatment. However, particularly in case of higher degrees of impurifications, it is also possible to perform the treating and/or contacting of the tricaprylin to be purified with the nucleophilic agent repeatedly, especially in two or more cycles and/or as a multistage process. Nevertheless, it is preferred to perform the treating and/or contacting of the tricaprylin to be purified with the nucleophilic agent in one stage (i.e. without any repeated treating and/or contacting); particularly in case of higher degrees of impurifications, it is also possible, as an alternative embodiment, to use higher amounts or concentrations of the nucleophilic agent instead of performing a repeated treating and/or contacting.

Consequently, by the inventive feature and/or measurement (i), the selective elimination or removal even of low amounts, preferably traces, of toxic halide-based impurities is possible in an efficient and selective way, namely without influencing other required and desired properties of the resulting product (such as e.g. low acid values and low hydroxyl values as well as high triglyceride contents).

As to feature and/or measurement (ii), the following is to be emphasized and should be taken into account: According to said feature and/or measurement (ii), it is envisaged that the at least one esterification step is run and/or performed at temperatures not exceeding 180 °C, especially not exceeding 150 °C, particularly not exceeding 120 °C, preferably not exceeding 100 °C, more preferably not exceeding 80 °C, even more preferably not exceeding 50 °C.

Surprisingly, as applicant has found out, the limitation of the temperatures during the at least one esterification step is also an efficient means in order to avoid the formation of toxic impurities, especially MCPD and glycidol fatty acid esters, during esterification. Nevertheless, high conversion rates, high yields as well as high esterification levels, particularly also high triglyceride contents with low acid and hydroxyl values, are reached on behalf of the inventive process, especially when taking into consideration other features and/or measurements, particularly when applying the synthesis paths as described hereinafter.

Consequently, the inventive process renders it possible, for the first time, to run the at least one esterification step under relatively mild conditions, especially for the purpose of avoiding the formation of toxic side-products of the aforementioned kind, while at the same time ensuring high esterification levels, especially high triglyceride contents, and excellent conversion rates and yields.

Above all in combination with at least one of the other aforementioned features and/or measurements (i), (iii) and/or (iv), the inventive feature and/or measurement (ii) leads to excellent results.

As to feature and/or measurement (iii), the following is to be emphasized and should be taken into account: According to said feature and/or measurement (iii), it is envisaged that the 1 ,2,3-propantriol of formula (I) or its protected form, preferably solketal (isopropylidene glycerol), used as starting material is at least essentially free of any chlorinated species and particularly comprises a total chlorinated species content of at most 1 ppm, especially at most 0.5 ppm, preferably at most 0.1 ppm, based on the 1,2,3-propantriol of formula (I) or its protected form, preferably solketal (isopropylidene glycerol).

As applicant has surprisingly found out, the presence of chlorinated species, especially the so-called total chlorinated species content, has a crucial influence on the formation of toxic side-products in the final reaction product, namely with respect to the formation of toxic MCPD and glycidol fatty acid esters.

Thus, the selection of the starting materials in the form of 1,2,3-propantriol or its protective form, preferably solketal, as to their purity grades very surprisingly has a key role in avoiding the formation of toxic side-products (e.g. MCPD and glycidol fatty acid esters) during esterification. By selecting these starting material as being at least essentially free of any chlorinated species, particularly with the indicated total chlorinated species contents, the formation of these toxic side-products is efficiently prevented or at least minimized to a maximum extent.

In the prior art, it was completely unknown and not realized at all so far that the content of chlorinated species in the starting materials in the form of 1 ,2,3-propantriol or its protected form (e.g. solketal) has such decisive influence on the purity degree of the resulting final reaction product (i.e. the tricaprylin).

Starting products in the form of 1,2,3-propantriol or its protected form, preferably solketal, with the required purities are in general available as marketed products.

1 ,2,3-propantriol with the required purity degree as used in the present invention may be obtained e.g. from petrochemical source originating from epichlorine-hydrine or from oleochemical source originating from e.g. hydrolytic splitting of natural fats and oils. The petrochemically or oleochemically derived glycerols with appropriate purity degrees and qualities are obtained mainly via purification by physical techniques (e.g. distillation and membrane-filtration such as membrane-ultrafiltration).

As experimental data by applicant surprisingly show, in contrast to the chlorinated species content of the glycerol or its protected derivative (e.g. solketal) as starting materials, the chlorinated species content of the remaining starting materials, i.e. the C₈-fatty acids, especially linear saturated or mono- or poly-unsaturated C₈-fatty acids, and/or their anhydrides and/or esters (preferably C₁-C₄-alkyl esters), preferably caprylic acid and/or its anhydride or esters (preferably C₁-C₄-alkyl esters), as also used for the at least esterification step, does not play such a key role. Esterification performed with caprylic acid and/or its anhydride and/or esters comprising total chlorinated species contents in the range of from 20 ppm to 100 ppm do not lead to the formation of toxic MCPD and glycidol esters during esterification provided that, at the same time, 1,2,3-propantriol or its protective form, preferably solketal, with the inventive purity grades (i.e. total chlorinated species content of at most 1 ppm, especially at most 0.5 ppm, preferably at most 0.1 ppm) is used. In turn, as further experimental data by applicant also surprisingly show, 1,2,3-propantriol or its protective form, preferably solketal, comprising total chlorinated species contents in the range of from 20 ppm to 100 ppm lead to the significant formation of toxic MCPD and glycidol fatty acid esters during esterification even if the remaining starting materials, i.e. the C₈-fatty acids, especially linear saturated or mono- or poly-unsaturated C₈-fatty acids, and/or their anhydrides and/or esters, preferably caprylic acid and/or its anhydride or esters, comprise high purity grades (i.e. total chlorinated species content of at most 1 ppm, especially at most 0.5 ppm, preferably at most 0.1 ppm). Without being bound to any specific theory, this surprising phenomenon might possibly be explained by the fact that 1,2,3-propantriol or its protective form, preferably solketal, at least predominantly comprises the chlorinated species in organically bonded form where they contribute to form MCPD and glycidol fatty acid esters during esterification, whereas in case of the C₈-fatty acids, especially linear saturated or mono- or poly-unsaturated Cs-fatty acids, and/or their anhydrides and/or esters, preferably caprylic acid and/or its anhydride or esters, these substances might at least predominantly comprise entrained chlorinated species in an inorganic form, especially as inorganic salts such as NaCl, which cannot contribute to form MCPD and glycidol fatty acid esters during esterification at all (or at most to a negligible extent only).

Consequently, on the whole, the specific selection of the aforenamed glycerol-based and solketal-based starting materials with respect to their purity degree, especially their content of chlorinated species, and the minimization of the total chlorinated species content in these starting materials also plays a key role for the purity and quality of the final reaction product.

As to feature and/or measurement (iv), the following is to be emphasized and should be taken into account: According to said feature and/or measurement (iv), it is envisaged that the at least one esterification step is run and/or performed in the absence of any metal-based catalyst.

Performing the at least one esterification step in the absence of any metal-based catalyst also is a key issue in the inventive process since such metal-based catalysts often introduce or entrain significant amounts of undesired halides, especially chlorides. The thus introduced or entrained halides, especially chlorides, may then lead to the undesired formation of toxic side-products, especially MCPD and glycidol fatty acid esters, during esterification. By avoiding such metal-based catalyst during esterification, the uncontrolled and undesired introduction or entrainment of halide-based impurities is thus efficiently avoided or prevented.

Furthermore, in contrast to enzyme-based catalysts used during esterification according to the inventive process according to particular embodiments, metal-based catalysts (such as e.g. tetrabutyl titanate) require relative high temperatures (e.g. between 190 °C and 230 °C) for esterification, thus leading to the undesired and uncontrolled formation of the aforementioned toxic side-products, especially MCPD and glycidol fatty acid esters.

Consequently, also the inventive feature and/or measurement (iv) significantly contributes to obtaining the final reaction product with the desired characteristics with respect to an excellent purity degree and other properties, such as e.g. low acid values, low hydroxyl values and high triglyceride contents.

Thus, on the whole, according to the present invention, the process of the present invention produces tricaprylin with a high purity degree.

Particularly, according to the process of the present invention, the reaction product, especially tricaprylin of formula (II)

CH₂[O-C(O)R¹]-CH[O-C(O)R¹]-CH₂[O-C(O)R¹] (II)

wherein in formula (II) the radical R¹ represents a linear saturated aliphatic C₇-alkyl radical of formula - (CH₂)₆ - CH₃ (i.e. a n-heptylic radical),
is characterized by and/or exhibits the following properties (1) to (5) in their combination and/or fulfils the following product specification:
   (1) acid value (AV), especially determined according to Ph. Eur. 2.5.1 (European Pharmacopoeia 10.0): ≤ 0.5 mg KOH / g, especially ≤ 0.2 mg KOH / g, preferably ≤ 0.1 mg KOH / g; and
   (2) hydroxyl value (OHV), especially determined according to Ph. Eur. 2.5.3 (European Pharmacopoeia 10.0): ≤ 0.5 mg KOH / g, especially ≤ 0.2 mg KOH / g, preferably ≤ 0.1 mg KOH / g; and
   (3) triglyceride content, especially determined according to Ph. Eur. 2.2.28 (European Pharmacopoeia 10.0): ≥ 99 %, especially ≥ 99.2 %, preferably ≥ 99.5 %, each based on the reaction product; and
   (4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.5 ppm, especially ≤ 0.2 ppm, preferably ≤ 0.1 ppm, each based on the reaction product; and
   (5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.5 ppm, especially ≤ 0.2 ppm, preferably ≤ 0.1 ppm, each based on the reaction product.

Particularly, in other words, according to the present invention, the process is performed such and/or the features and/or measurements (i) to (iv), especially the combination of at least two, preferably at least three, of the features and/or measurements (i) to (iv), are applied and/or performed with the proviso that the reaction product, especially tricaprylin of formula (II)

CH₂[O-C(O)R¹]-CH[O-C(O)R¹]-CH₂[O-C(O)R¹] (II)

wherein in formula (II) the radical R¹ represents a linear saturated aliphatic C₇-alkyl radical of formula - (CH₂)₆ - CH₃ (i.e. a n-heptylic radical),
is characterized by and/or exhibits the following properties (1) to (5) in their combination and/or fulfils the following product specification:
   (1) acid value (AV), especially determined according to Ph. Eur. 2.5.1 (European Pharmacopoeia 10.0): ≤ 0.5 mg KOH / g, especially ≤ 0.2 mg KOH / g, preferably ≤ 0.1 mg KOH / g; and
   (2) hydroxyl value (OHV), especially determined according to Ph. Eur. 2.5.3 (European Pharmacopoeia 10.0): ≤ 0.5 mg KOH / g, especially ≤ 0.2 mg KOH / g, preferably ≤ 0.1 mg KOH / g; and
   (3) triglyceride content, especially determined according to Ph. Eur. 2.2.28 (European Pharmacopoeia 10.0): ≥ 99 %, especially ≥ 99.2 %, preferably ≥ 99.5 %, each based on the reaction product; and
   (4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.5 ppm, especially ≤ 0.2 ppm, preferably ≤ 0.1 ppm, each based on the reaction product; and
   (5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.5 ppm, especially ≤ 0.2 ppm, preferably ≤ 0.1 ppm, each based on the reaction product.

Surprisingly, the inventive reaction product unifies the aforementioned five properties (1) to (5), which have been deemed so far incompatible with one another in their overall combination. For, according to prior art methods, when increasing the triglyceride content and lowering acid and hydroxyl values, usually high total contents of MCPD and glycidol fatty acid esters are effected due to drastic esterification conditions; in turn, when lowering the total contents of MCPD and glycidol fatty acid esters, according to the prior art, relatively low triglyceride contents with high acid and hydroxyl values are reached.

In contrast to this prior art, the present invention renders it, for the first time, possible to provide a combination of all aforementioned five properties (1) to (5) in one and the same product. Consequently, the inventive reaction products resulting from the inventive process are highly suitable for food and pharmaceutical applications, even in parenteral applications with high dosages and/or concentrations.

According to the present invention, the inventive reaction product resulting from the process of the present invention has a very low acid value (AV), namely an acid value (AV), especially determined according to Ph. Eur. 2.5.1 (European Pharmacopoeia 10.0), ≤ 0.5 mg KOH / g, especially ≤ 0.2 mg KOH / g, preferably ≤ 0.1 mg KOH / g. The parameter of the so-called acid value indicates the residual free acids originating from esterification. The lower the acid value, the better the product quality. Especially, very low acid values as envisaged by the present invention lead to a high stability of the reaction product, especially an excellent long-term stability and/or storage stability as well as an excellent shelf-life stability, of the resulting reaction product (i.e. tricaprylin).

As indicated hereinabove, according to the present invention, the acid value (AV) is usually determined according to Ph. Eur. 2.5.1 (European Pharmacopoeia 10.0). The acid value (synonymously also called neutralization number or acid number or acidity) is the mass of potassium hydroxide (KOH) in milligram that is required to neutralize one gram of chemical substance; the acid number particularly is a measure of the number of carboxylic acid groups in a chemical compound (here: tricaprylin). The indicated standard Ph. Eur. 2.5.1 is based on a titration method.

Apart from the parameter of the acid value (AV), also the hydroxyl value (OHV) of the inventive reaction product obtained from the process of the present invention plays a key role. According to the present invention, the inventive reaction product has also a very low hydroxyl value (OHV), namely a hydroxyl value (OHV), especially determined according to Ph. Eur. 2.5.3 (European Pharmacopoeia 10.0), ≤ 0.5 mg KOH / g, especially ≤ 0.2 mg KOH / g, preferably ≤ 0.1 mg KOH / g. In the present case, the hydroxyl value (OHV) is a measure for the esterification degree and indicates very high esterification levels in the inventive reaction products.

According to the present invention, the hydroxyl value (OHV) is especially determined according to Ph. Eur. 2.5.3 (European Pharmacopoeia 10.0). The hydroxyl value determined according to this method is defined as the number of milligrams of potassium hydroxide (KOH) required to neutralize the acetic acid taken up in acetylation of one gram of a chemical substance that contains free hydroxyl groups (here: tricaprylin). Thus, the hydroxyl value is a measure of a content of free hydroxyl groups in a chemical substance (here: tricaprylin), usually expressed in units of the mass of potassium hydroxide (KOH) in milligrams equivalent to the hydroxyl content of one gram of the chemical substance. The indicated determination method Ph. Eur. 2.5.3 (European Pharmacopoeia 10.0) comprises acetylation of the free hydroxyl groups of the reaction product (here: tricaprylin) with acetic anhydride in pyridine solvent, wherein, after completion of the reaction, water is added and the remaining unreacted acetic anhydride is converted to acetic acid and measured by titration with potassium hydroxide.

Thus, in case of the inventive reaction product, the tricaprylin of the present invention, apart from a very low acid value (AV), also comprise, at the same time, very low hydroxyl values (OHV), as indicated hereinabove, corresponding to high esterification degrees.

Furthermore, as a third parameter and/or characteristic (3), the inventive reaction product exhibit a very high triglyceride content, namely a triglyceride content especially determined according to Ph. Eur. 2.2.28 (European Pharmacopoeia 10.0), ≥ 99 %, especially ≥ 99.2 %, preferably ≥ 99.5 %, each based on the reaction product. These extremely high values for the triglyceride content also indicate the high purity grade of the inventive reaction product. The triglyceride content of the inventive products is particularly determined according to Ph. Eur. 2.2.28 (European Pharmacopoeia 10.0). This method is based on determination via gas chromatography.

Apart from the parameters and/or characteristics of the low acid value (AV), low hydroxyl value (OHV) and high triglyceride content, the inventive reaction product is also characterized by extremely low total contents of MCPD fatty acid esters and glycidol fatty acid esters: The total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]), is ≤ 0.5 ppm, especially ≤ 0.2 ppm, preferably ≤ 0.1 ppm, each based on the reaction product and; the total content of glycidol esters of fatty acids, especially determined according to method of DGF C-VI 18 (10) (Einheitsmethode der Deutschen Gesellschaft für Fettwissenschaft e.V. [Standard method of German Society for Fat Science]), is ≤ 0.5 ppm, especially ≤ 0.2 ppm, preferably ≤ 0.1 ppm, each based on the reaction product.

The indicated determination method of DGF C-VI 18 (10) allows a reliable quantification of these toxic impurities. Above all, the inventive reaction product resulting from the process of the present invention combines these very low total contents of toxic esters with high triglyceride content levels and low acid and hydroxide values, as indicated hereinabove.

Consequently, the fivefold combination of the aforementioned characteristics and/or parameters (1) to (5) is a unique feature of the reaction product obtained from the process of the present invention and delimits this reaction product from prior art reaction products of same kind. Thus, for the very first time, the process of the present invention allows producing tricaprylin with high purity grades, indicated by low acid values and low hydroxyl values, while at the same time providing very high triglyceride contents and extremely low total contents of toxic MCPD and glycidol fatty acid esters.

According to a particular embodiment, the present invention relates to a process for producing tricaprylin (i.e. trioctanoylglyceride or tricaprylyl glycerol), especially with a high purity degree, especially to the process as described or defined hereinbefore,
wherein 1 ,2,3-propantriol (glycerol, glycerine) of formula (I)

   CH₂(OH) - CH(OH) - CH₂(OH) (I)
or its protected form or precursor, preferably solketal (isopropylidene glycerol), is used as starting material and is reacted and/or converted to tricaprylin,
so as to produce, as a reaction product, tricaprylin of formula (II)

   CH₂[O-C(O)R¹]-CH[O-C(O)R¹]-CH₂[O-C(O)R¹] (II)
wherein in formula (II) the radical R¹ represents a linear saturated aliphatic C₇-alkyl radical of formula - (CH₂)₆ - CH₃ (i.e. a n-heptylic radical),
wherein the process comprises at least one esterification step, especially an esterification step using C₈-fatty acids, especially linear saturated or mono- or poly-unsaturated C₈-fatty acids, and/or their anhydrides and/or esters (preferably C₁-C₄-alkyl esters), preferably at least one esterification step using caprylic acid (i.e. n-octylic acid or 1-heptanecarboxlic acid) of formula CH₃ - (CH₂)₆ - COOH and/or its anhydride or esters (preferably C₁-C₄-alkyl esters);
wherein the process is characterized by at least one of the following features and/or measurements (i) to (iv), especially by a combination of at least two, preferably by a combination of at least three, more preferably by a combination of all four, of the following features and/or measurements (i) to (iv):
   (i) a preferably selective derivatization of halide-based impurities, especially of MCPD fatty acid esters (fatty acid esters of monochloropropanediols, particularly MCPD caprylic acid esters) and glycidol esters of fatty acids (particularly glycidol caprylic acid esters), present in the resulting reaction product is performed and/or the resulting reaction product is treated and/or contacted with at least one nucleophilic agent, especially with a nucleophilic agent capable of removing halide-based impurities, preferably with a nucleophilic agent selected from the group consisting of hydrogen sulfites (bisulfites, HSO₃⁻), sulfites (SO₃²⁻), hydrogen thiosulfates (HS₂O₃⁻), thiosulfates (S₂O₃²⁻), hydrogen phosphites (H₂PO₃⁻), phosphites (HPO₃²⁻) and phosphines as well as combinations thereof, more preferably with hydrogen sulfites (bisulfites, HSO₃⁻), in particular for a preferably selective derivatization of halide-based impurities, especially of MCPD fatty acid esters (fatty acid esters of monochloropropanediols) and glycidol esters of fatty acids;
   (ii) the at least one esterification step is run and/or performed at temperatures not exceeding 180 °C, especially not exceeding 150 °C, particularly not exceeding 120 °C, preferably not exceeding 100 °C, more preferably not exceeding 80 °C, even more preferably not exceeding 50 °C;
   (iii) the 1,2,3-propantriol of formula (I) or its protected form, preferably solketal (isopropylidene glycerol), used as starting material is at least essentially free of any chlorinated species and particularly comprises a total chlorinated species content of at most 1 ppm, especially at most 0.5 ppm, preferably at most 0.1 ppm, based on the 1,2,3-propantriol of formula (I) or its protected form, preferably solketal (isopropylidene glycerol);
   (iv) the at least one esterification step is run and/or performed in the absence of any metal-based catalyst,
      wherein the process is performed such and/or the features and/or measurements (i) to (iv), especially the combination of at least two, preferably at least three, of the features and/or measurements (i) to (iv), are applied and/or performed with the proviso that the reaction product, especially tricaprylin of above formula (II), is characterized by and/or exhibits the following properties (1) to (5) in their combination and/or fulfils the following product specification:
      (1) acid value (AV), especially determined according to Ph. Eur. 2.5.1 (European Pharmacopoeia 10.0): ≤ 0.5 mg KOH / g, especially ≤ 0.2 mg KOH / g, preferably ≤ 0.1 mg KOH / g; and
      (2) hydroxyl value (OHV), especially determined according to Ph. Eur. 2.5.3 (European Pharmacopoeia 10.0): ≤ 0.5 mg KOH / g, especially ≤ 0.2 mg KOH / g, preferably ≤ 0.1 mg KOH / g; and
      (3) triglyceride content, especially determined according to Ph. Eur. 2.2.28 (European Pharmacopoeia 10.0): ≥ 99 %, especially ≥ 99.2 %, preferably ≥ 99.5 %, each based on the reaction product; and
      (4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.5 ppm, especially ≤ 0.2 ppm, preferably ≤ 0.1 ppm, each based on the reaction product; and
      (5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.5 ppm, especially ≤ 0.2 ppm, preferably ≤ 0.1 ppm, each based on the reaction product.

According to a further particular embodiment of the present invention, the inventive process is performed such and/or the features and/or measurements (i) to (iv), especially the combination of at least two, preferably at least three, of the features and/or measurements (i) to (iv), are applied and/or performed with the proviso that the reaction product, especially tricaprylin of above formula (II), is characterized by and/or exhibits the following properties (1) to (5) in their combination and/or fulfils the following product specification:
(1) acid value (AV), especially determined according to Ph. Eur. 2.5.1 (European Pharmacopoeia 10.0): ≤ 0.1 mg KOH / g; and
(2) hydroxyl value (OHV), especially determined according to Ph. Eur. 2.5.3 (European Pharmacopoeia 10.0): ≤ 0.1 mg KOH / g; and
(3) triglyceride content, especially determined according to Ph. Eur. 2.2.28 (European Pharmacopoeia 10.0): ≥ 99.5 %, especially ≥ 99.6 %, preferably ≥ 99.8 %, each based on the reaction product; and
(4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.1 ppm based on the reaction product; and
(5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.1 ppm based on the reaction product.

Such reaction product, especially the tricaprylin of above general formula (II), is particularly appropriate for applications requiring high purity grades and at the same time high active ingredient levels, e.g. for nutritional and above all pharmaceutical applications and uses (e.g. even for parenteral applications in high dosages and/or with high concentrations).

As stated hereinabove, the inventive process comprises at least one esterification step, especially using C₈-fatty acids, especially linear saturated or mono- or poly-unsaturated C₈-fatty acids, and/or their anhydrides and/or esters (preferably C₁-C₄-alkyl esters), preferably using caprylic acid (i.e. n-octylic acid or 1-heptanecarboxlic acid) of formula CH₃ - (CH₂)₆ - COOH and/or its anhydride or esters (preferably C₁-C₄-alkyl esters).

According to a particular embodiment of the inventive process, in the at least one esterification step caprylic acid of formula CH₃ - (CH₂)₆ - COOH and/or its anhydride or esters (preferably C₁-C₄-alkyl esters) is used.

As already delineated hereinabove, according to the inventive process, there is provided and/or produced, as a reaction product, tricaprylin of formula (II)

CH₂[O-C(O)R¹]-CH[O-C(O)R¹]-CH₂[O-C(O)R¹] (II)

wherein in formula (II) the radical R¹ represents a linear saturated aliphatic C₇-alkyl radical of formula - (CH₂)₆ - CH₃ (i.e. a n-heptylic radical).

In this respect, according to a particular embodiment of the inventive process, it is envisaged
that the reaction product is tricaprylin; and/or
that in above formula (II) the radical R¹ represents a linear saturated aliphatic C₇-alkyl radical; and/or
that in the at least one esterification step octanoic acid (caprylic acid) and/or its anhydride and/or esters (preferably C₁-C₄-alkyl esters) is used.

As already delineated hereinabove, according to the present invention, the inventive process is characterized by at least one of the aforedefined features and/or measurements (i) to (iv), especially by a combination of at least two, preferably by a combination of at least three, more preferably by a combination of all four, of the aforedefined features and/or measurements (i) to (iv).

In this respect, according to a particular embodiment of the inventive process, it is envisaged that the inventive process is characterized by a combination of at least two, preferably by a combination of at least three, more preferably by a combination of all four, of the aforedefined features and/or measurements (i) to (iv).

According to another particular embodiment of the inventive process, it is envisaged that the inventive process is characterized by a combination of at least two, preferably by a combination of at least three, of the aforedefined features and/or measurements (ii) to (iv), optionally in further combination with aforedefined feature and/or measurement (i).

Furthermore, according to yet another particular embodiment of the inventive process, it is envisaged that the inventive process is characterized by a combination of aforedefined feature and/or measurement (i) with at least two of the aforedefined features and/or measurements (ii) to (iv).

Finally, according to a further particular embodiment of the inventive process, it is envisaged that the inventive process is characterized by a combination of all of aforedefined features and/or measurements (i) to (iv).

As already delineated hereinabove, according to the present invention, when applying aforedefined features and/or measurements (i) and/or (ii) and/or (iii) and/or (iv), tricaprylin, especially with a high purity degree, is obtained in accordance with the above-reference product specification.

According to a particular embodiment of the inventive process, it is particularly preferred that the whole and/or overall process, especially also including optional pre-treatment and/or post-treatment steps if any, is run and/or performed at temperatures not exceeding 180 °C, especially not exceeding 160 °C, particularly not exceeding 150 °C, preferably not exceeding 120 °C.

In other words, according to this particular embodiment of the inventive process, it is particularly preferred that the process is characterized by further feature and/or measurement (v):
(v) the whole and/or overall process, especially also including optional pre-treatment and/or post-treatment steps if any, is run and/or performed at temperatures not exceeding 180 °C, especially not exceeding 160 °C, particularly not exceeding 150 °C, preferably not exceeding 120 °C.

By this measurement (v), the formation of toxic by-products, such as e.g. MCPD or glycidol fatty acid esters can be efficiently prevented since such formation usually occurs at temperatures higher than these indicated values.

According to another particular embodiment of the inventive process, it is particularly preferred that the whole and/or overall process, especially also including optional pre-treatment and/or post-treatment steps if any, is run and/or performed in the absence of any metal-based catalyst.

In other words, according to this particular embodiment of the inventive process, it is particularly preferred that the process is characterized by further feature and/or measurement (vi):
(vi) the whole and/or overall process, especially also including optional pre-treatment and/or post-treatment steps if any, is run and/or performed in the absence of any metal-based catalyst.

Performing the whole and/or overall process, especially also including optional pre-treatment and/or post-treatment steps if any, in the absence of any metal-based catalyst is also a key issue in the inventive process since such metal-based catalysts often introduce or entrain significant amounts of undesired halides, especially chlorides. The thus introduced or entrained halides, especially chlorides, may then lead to the undesired formation of toxic side-products, especially MCPD and glycidol fatty acid esters, during esterification. By avoiding such metal-based catalyst over the whole and/or overall process, especially also including optional pre-treatment and/or post-treatment steps if any, the uncontrolled and undesired introduction or entrainment of halide-based impurities is thus efficiently avoided or prevented.

As already delineated hereinabove, the process of the present invention comprises at least one esterification step.

In general, the at least one esterification step may be performed and/or carried out as a one-stage esterification or, alternatively, as a two-stage or multistage esterification.

According to a particular embodiment of the inventive process, the at least one esterification step may be performed and/or carried out as a one-stage esterification, using anhydrides of C₈-fatty acids, especially caprylic acid anhydride, to be reacted with 1,2,3-propantriol of formula (I) or its protected form or precursor, preferably solketal. Alternatively, according to another particular embodiment of the inventive process, the at least one esterification step may also be performed and/or carried out as a two-stage or multistage esterification (i.e. in a first stage using C₈-fatty acids, especially caprylic acid, and/or their esters to be reacted with the 1,2,3-propantriol of formula (I) or its protected form or precursor, preferably solketal, followed in a subsequent stage by further esterification, especially further esterification of free and/or remaining OH-groups, preferably using anhydrides of C₈-fatty acids, especially caprylic acid anhydride).

In general, according to a particular embodiment of the inventive process, it is preferred when the at least one esterification step is performed and/or carried out in the absence of solvents and/or without any solvent. This means that the at least one esterification step is carried out as a reaction/esterification in mass or as a reaction/esterification in substance or as a so-called *bulk reaction* (i.e. *bulk esterification*). This has the advantage that the reaction products obtained are not contaminated with any solvent and that no solvent has to be removed and disposed of or recycled in a costly and energy-intensive manner after the at least one esterification step has been carried out. Surprisingly, the at least one esterification step nevertheless proceeds with high conversion and yields and at least essentially without any significant by-product formation. In addition, avoiding the use of any solvents during the at least one esterification step also facilitates the recycling of the esterification catalyst if used and above all ensures that the recycling of the esterification catalyst is possible without its reactivation since solvents might otherwise deactivate or even poison such esterification catalyst.

The temperature ranges to be applied for the at least one esterification step may vary over a wide range. Usually, according to the present invention, it is preferred when the at least one esterification step is performed and/or carried out at moderate temperatures, i.e. at temperatures in the range of from 5 °C to 95 °C, especially in the range of from 10 °C to 85 °C, preferentially in the range of from 15 °C to 80 °C, more preferably in the range of from 20 °C to 75 °C, even more preferably in the range of from 25 °C to 70 °C. This moderate temperature regime applied for the at least one esterification step efficiently prevents the formation of particularly toxic by-products and ensures an efficient esterification. However, the skilled person may, if necessary or in a single or exceptional case, deviate from these range specifications without leaving the scope of the present invention.

Also the pressure ranges to be applied for the at least one esterification step may vary over a wide range. In general, according to the present invention, it is preferred when the at least one esterification step may be performed and/or carried out at a pressure in the range of from 0.0001 bar to 10 bar, especially in the range of from 0.001 bar to 5 bar, preferentially in the range of from 0.01 bar to 2 bar, more preferably in the range of from 0.05 bar to 1 bar, even more preferably at about 1 bar. This pressure regime applied for the at least one esterification step ensures an efficient esterification. However, the skilled person may, if necessary or in a single or exceptional case, deviate from these range specifications without leaving the scope of the present invention.

Basically, according to the present invention, it is possible that either the at least one esterification step is performed and/or carried out in the absence of any catalyst or, alternatively, that the at least one esterification step is performed and/or carried out at least partially in the presence of an enzyme as a catalyst and/or at least partially in the presence of an enzyme-based catalyst. In case that an enzyme as a catalyst and/or an enzyme-based catalyst is used, it is preferred that the catalyst is recycled after the at least one esterification step; this increases efficiency and process economy, especially when the inventive process is performed on a large-scale or industrial level.

In the present description, the wording or formulation "enzyme (used) as a catalyst" and "enzyme-based catalyst" are utilized as synonyms.

In this respect, according to a particular embodiment of the inventive process, the at least one esterification step may be performed and/or carried out in the absence of any catalyst when using anhydrides of C₈-fatty acids, especially caprylic acid anhydride, to be reacted with 1,2,3-propantriol of formula (I) or its protected form or precursor, preferably solketal. Alternatively, according to another and alternative particular embodiment of the inventive process, the at least one esterification step may also be performed and/or carried out at least partially in the presence of an enzyme as a catalyst and/or at least partially in the presence of an enzyme-based catalyst when using C₈-fatty acids, especially caprylic acid, and/or their esters to be reacted with the 1,2,3-propantriol of formula (I) or its protected form or precursor, preferably solketal; as delineated hereinbefore, in case that an enzyme as a catalyst and/or an enzyme-based catalyst is used, it is preferred that the catalyst is recycled after the at least one esterification step.

As already delineated hereinabove, according to a particular embodiment of the present invention, the at least one esterification step may be performed at least partially in the presence of an enzyme as a catalyst and/or at least partially in the presence of an enzyme-based catalyst (preferably in the absence of solvents and/or without any solvent as also indicated hereinabove). This ensures a high esterification efficiency, especially high conversion rates and yields with excellent esterification degrees and low acid and hydroxyl values at mild esterification conditions.

In general, when the at least one esterification step is performed at least partially in the presence of an enzyme as a catalyst and/or at least partially in the presence of an enzyme-based catalyst, the enzyme may especially be selected from synthetases (ligases), catalases, esterases, lipases and combinations thereof.

Especially, the enzyme may be derived from Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar and Pseudomonas sp. and combinations thereof, preferably from Candida antarctica, Mucor miehei (Rhizomucor miehei) and Thermomyces lanuginosus, more preferably from Candida Antarctica. These species lead to particularly good esterification results.

Furthermore, the enzyme may especially be used in immobilized form, especially immobilized on a carrier, preferentially on a polymeric carrier, preferably on a polymeric organic carrier, more preferably with hydrophobic properties, even more preferably on a poly(meth)acrylic resin-based carrier. This significantly improves handling and use as well as recycling of the enzyme as a catalyst.

Especially, as already delineated hereinabove, for the above reasons it is preferred when the enzyme is recycled after the at least one esterification step.

In general, when the at least one esterification step is performed at least partially in the presence of an enzyme as a catalyst and/or at least partially in the presence of an enzyme-based catalyst, the temperature ranges to be applied for the at least one esterification step may vary over a wide range. Especially, in such case, the at least one esterification step may be performed and/or carried out at temperatures in the range of from 5 °C to 95 °C, especially in the range of from 10 °C to 85 °C, preferentially in the range of from 15 °C to 80 °C, more preferably in the range of from 20 °C to 75 °C, even more preferably in the range of from 25 °C to 70 °C. However, the skilled person may, if necessary or in a single or exceptional case, deviate from these range specifications without leaving the scope of the present invention.

Also, when the at least one esterification step is performed at least partially in the presence of an enzyme as a catalyst and/or at least partially in the presence of an enzyme-based catalyst, the enzyme amounts to be applied for the at least one esterification step may vary over a wide range. Especially, in such case, the enzyme is used in amounts, based on the total amount of all starting compounds, in the range of from 0.0001 to 25 % by weight, especially in the range of from 0.001 to 20 % by weight, preferentially in the range of from 0.01 to 15 % by weight, preferably in the range of from 0.05 to 10 % by weight. However, the skilled person may, if necessary or in a single or exceptional case, deviate from these range specifications without leaving the scope of the present invention.

Especially, according to a particular embodiment of the present invention, the enzyme may be used in amounts, based on the total amount of all starting compounds, in the range of from 1 • 10⁻⁶ to 5 mol-%, especially in the range of from 1 • 10⁻⁵ to 2.5 mol-%, preferentially in the range of from 1 • 10⁻⁴ to 1.5 mol-%, preferably in the range of from 1 • 10⁻³ to 1 mol-%. However, the skilled person may, if necessary or in a single or exceptional case, deviate from these range specifications without leaving the scope of the present invention.

Moreover, when the at least one esterification step is performed at least partially in the presence of an enzyme as a catalyst and/or at least partially in the presence of an enzyme-based catalyst, also the pressure ranges to be applied for the at least one esterification step may vary over a wide range. Especially, in such case, the at least one esterification step may be performed and/or carried out at a pressure in the range of from 0.0001 bar to 10 bar, especially in the range of from 0.001 bar to 5 bar, preferentially in the range of from 0.01 bar to 2 bar, more preferably in the range of from 0.05 bar to 1 bar, even more preferably at about 1 bar. However, the skilled person may, if necessary or in a single or exceptional case, deviate from these range specifications without leaving the scope of the present invention.

As also already delineated hereinabove, the present invention is based on a universal concept / process for producing high-purity tricaprylin with high yields and high conversion rates, which concept / process is independent from any specific synthesis route. Thus, applicant's concept is universal to be applied for this purpose.

Consequently, a multitude of synthesis routes may be applied within the scope of the present invention, which possible synthesis routes will be explained in more detail hereinafter.

According to a particular embodiment of the present invention, the reaction product obtained or obtainable from the process of the present invention may be produced e.g. via one of the following synthesis routes (A) to (F):
(A) According to a (first) Synthesis Route (A), 1,2,3-propantriol (glycerol, glycerine) of formula (I)

   CH₂(OH)-CH(OH)-CH₂(OH) (I)

   is first reacted, preferably in the presence of an enzyme as a catalyst and/or in the presence of an enzyme-based catalyst (especially as defined hereinabove), with caprylic acid or its ester, preferably so as to produce a diglyceride, more preferably a 1,3-diglyceride,
   followed by subsequent reaction, preferably in the absence of any catalyst, with caprylic acid anhydride,
   so as to produce the reaction product of formula (II) as defined hereinabove, especially tricaprylin;
   or else
(B) according to a (second) Synthesis Route (B), 1,2,3-propantriol (glycerol, glycerine) of formula (I)

   CH₂(OH)-CH(OH)-CH₂(OH) (I)

   is first reacted, preferably in the presence of an enzyme as a catalyst and/or in the presence of an enzyme-based catalyst (especially as defined hereinabove), with caprylic acid or its ester, preferably so as to produce a diglyceride, more preferably a 1,3-diglyceride,
   followed by subsequent reaction, preferably in the presence of an enzyme as a catalyst and/or in the presence of an enzyme-based catalyst (more preferably an enzyme for regioselective esterification of the 2-position of 1,3-diglyceride, such as e.g. *Pseudomonas fluorescens* lipase or *Burkholderia cepacia* lipase), with caprylic acid or its ester,
   so as to produce the reaction product of formula (II) as defined hereinabove, especially tricaprylin;
   or else
(C) according to a (third) Synthesis Route (C), 1,2,3-propantriol (glycerol, glycerine) of formula (I)

   CH₂(OH)-CH(OH)-CH₂(OH) (I)

   is reacted, preferably in the absence of any catalyst, with caprylic acid anhydride,
   so as to produce the reaction product of formula (II) as defined hereinabove, especially tricaprylin;
   or else
(D) according to a (fourth) Synthesis Route (D), a protected form or precursor of 1,2,3-propantriol of above formula (I), preferably solketal (isopropylidene glycerol), is reacted, preferably in the absence of any catalyst, with caprylic acid anhydride,
   especially wherein a deprotection reaction, especially ring-opening (preferably acid-induced) and optionally removal of acetone, is performed after monoesterification of the free OH-group of the protected form or precursor of 1,2,3-propantriol of above formula (I), followed by further esterification with caprylic acid anhydride,
   so as to produce the reaction product of formula (II) as defined hereinabove, especially tricaprylin;
   or else
(E) according to a (fifth) Synthesis Route (E), a protected form or precursor of 1,2,3-propantriol of above formula (I), preferably solketal (isopropylidene glycerol), is reacted, preferably in the presence of an enzyme as a catalyst and/or in the presence of an enzyme-based catalyst (especially as defined hereinabove), with caprylic acid or its ester, followed by deprotection reaction, especially ring-opening (preferably acid-induced) and removal of acetone, preferably so as to produce a monoglyceride, more preferably a terminal monoglyceride and/or 1-monoglyceride,
   followed by subsequent reaction, preferably in the absence of any catalyst, with caprylic acid anhydride,
   so as to produce the reaction product of formula (II) as defined hereinabove, especially tricaprylin;
   or else
(F) according to a (sixth) Synthesis Route (F), 1,2,3-propantriol (glycerol, glycerine) of formula (I)

   CH₂(OH)-CH(OH)-CH₂(OH) (I)

   is reacted, optionally in the presence of an enzyme as a catalyst and/or in the presence of an enzyme-based catalyst (especially as defined hereinabove), with at least one linear mono- or poly-unsaturated (preferably mono-unsaturated) aliphatic C₈-fatty acid and/or its esters and/or anhydrides, preferably with at least one linear mono-unsaturated aliphatic C₈-fatty acid and/or its esters and/or anhydrides, preferably so as to produce triglycerides of linear mono- or poly-unsaturated (preferably mono-unsaturated) aliphatic C₈-fatty acids,
   followed by subsequent hydrogenation reaction, preferably in the presence of at least one hydrogenation catalyst, preferably so as to hydrogenate the double bonds of the linear mono- or poly-unsaturated (preferably mono-unsaturated) aliphatic C₈-fatty acid radicals of the triglycerides,
   so as to produce the reaction product of formula (II) as defined hereinabove, especially tricaprylin.

The above-described (first) Synthesis Route (A) may be illustrated, in a non-limiting way and for purposes of visualization only, by the following simplified reaction scheme, showing the production of the inventive reaction product tricaprylin.

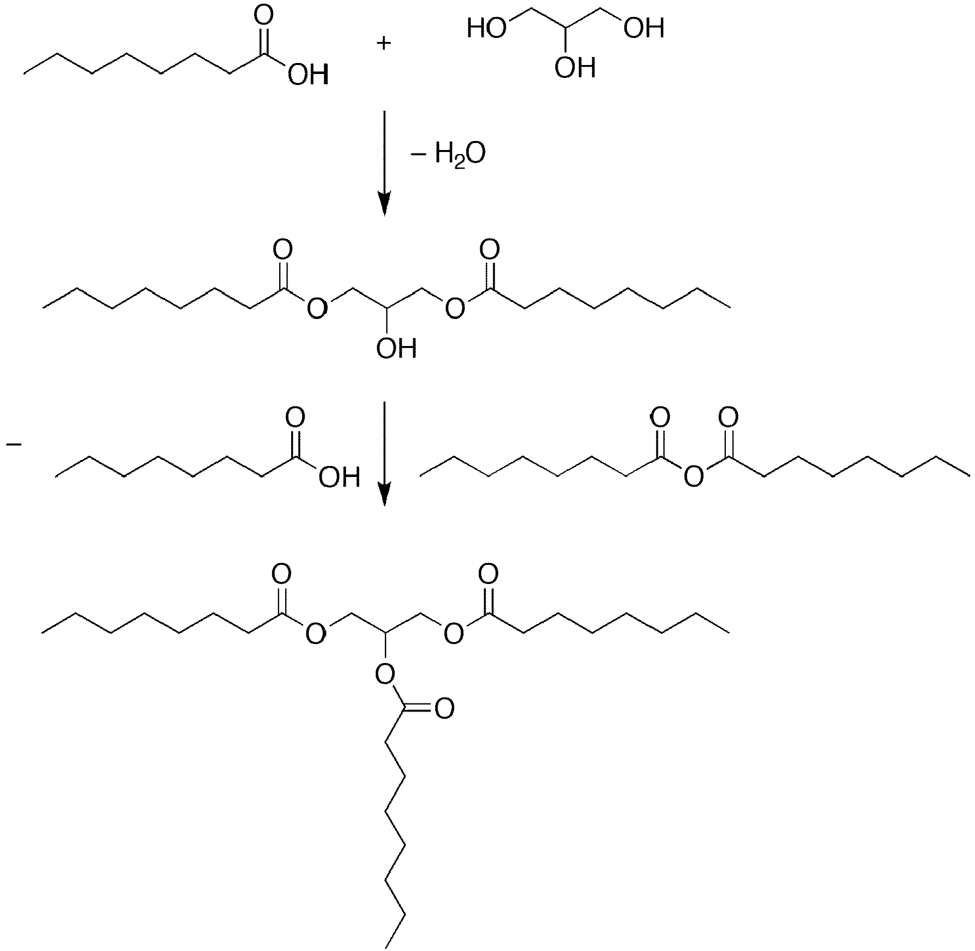

The above-described (second) Synthesis Route (B) may be illustrated, in a non-limiting way and for purposes of visualization only, by the following simplified reaction scheme, showing the production of the inventive reaction product tricaprylin.

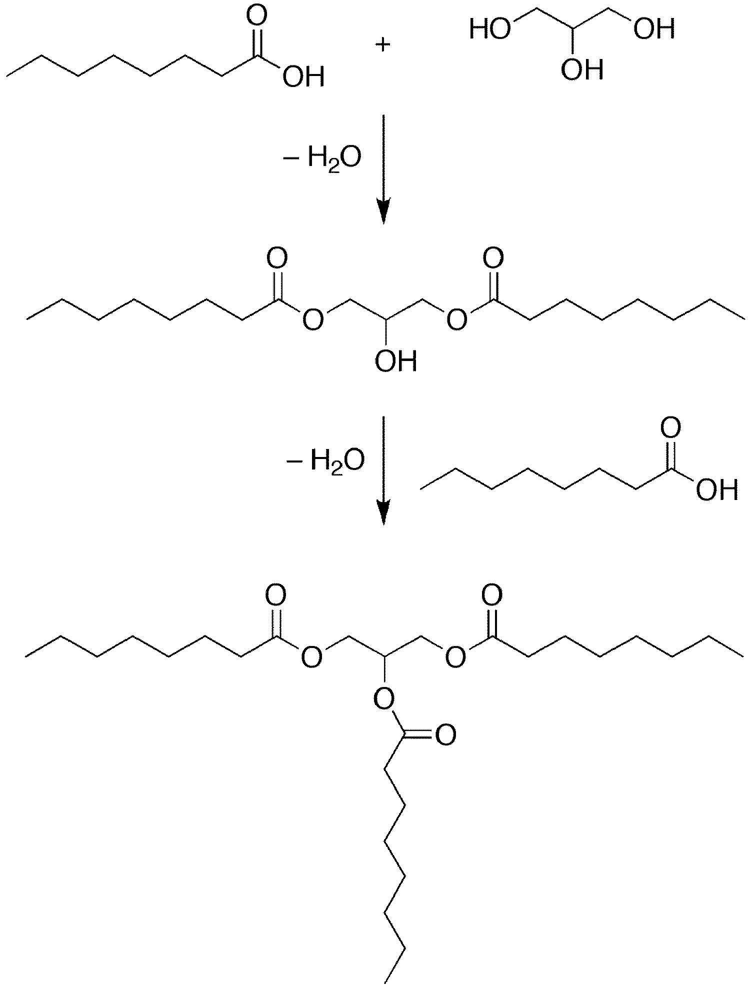

The above-described (third) Synthesis Route (C) may be illustrated, in a non-limiting way and for purposes of visualization only, by the following simplified reaction scheme, showing the production of the inventive reaction product tricaprylin.

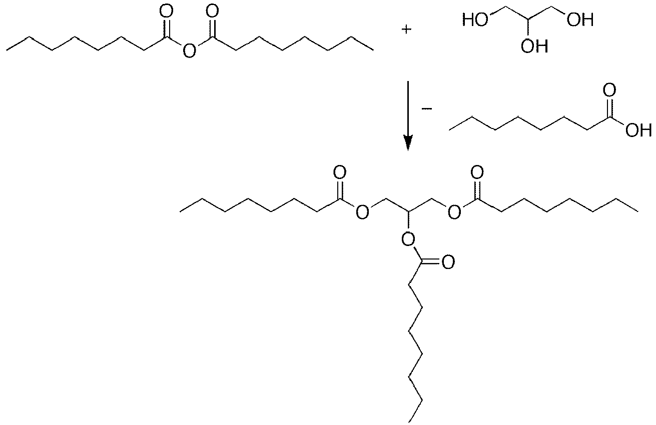

The above-described (fourth) Synthesis Route (D) may be illustrated, in a non-limiting way and for purposes of visualization only, by the following simplified reaction scheme, showing the production of the inventive reaction product tricaprylin.

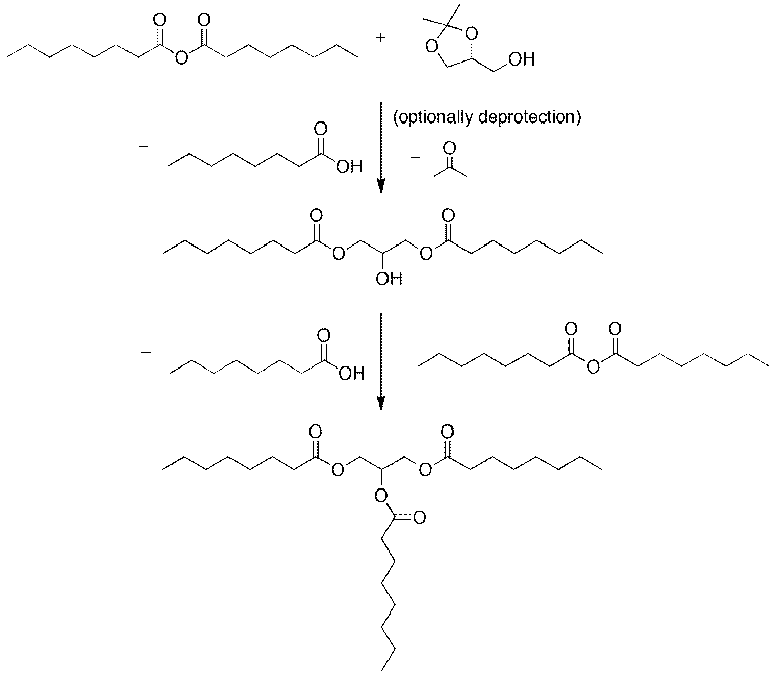

The above-described (fifth) Synthesis Route (E) may be illustrated, in a non-limiting way and for purposes of visualization only, by the following simplified reaction scheme, showing the production of the inventive reaction product tricaprylin.

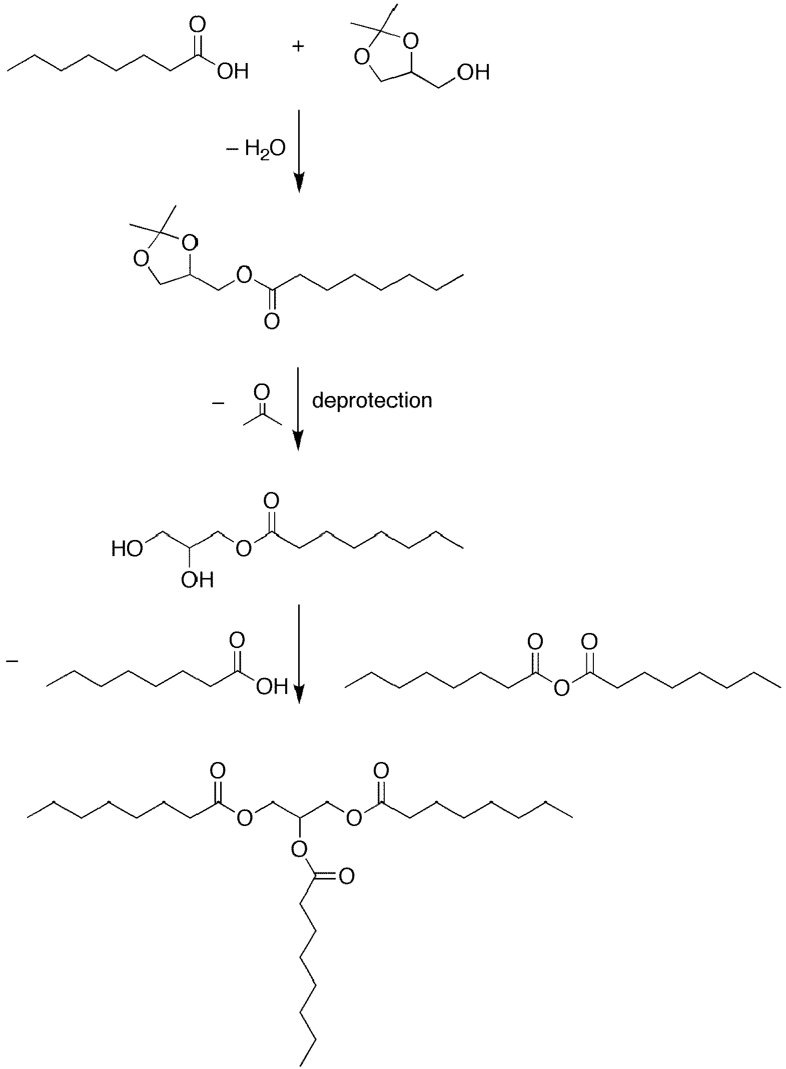

The above-described (sixth) Synthesis Route (F) may be illustrated, in a non-limiting way and for purposes of visualization only, by the following simplified reaction scheme, showing the production of the inventive reaction product tricaprylin.

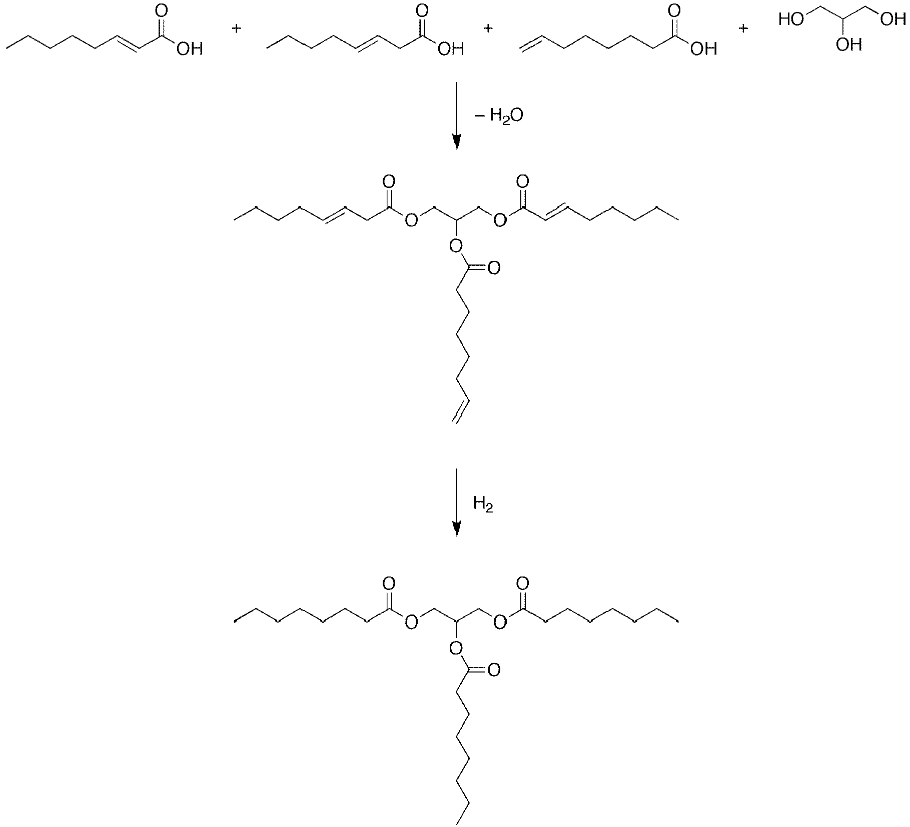

On the whole, applicant has been able to provide a universal concept and process, respectively, for producing high-purity tricaprylin with high yields and high conversion rates, which concept / process is independent from any specific synthesis route. Thus, applicant's concept is universal to be applied for this purpose. The inventive process is at the same time highly flexible and compatible with known prior art production processes and plants for these substances since the aforenamed inventive features and/or measurements (i) to (iv) can be easily implemented in or used to modify known production processes and plants for these substances.

Above all, the inventive process produces tricaprylin with a high purity degree, which at the same time unifies a combination of at least five purity properties, namely: (1) a very low acid value (AV), (2) a very low hydroxyl value (OHV), (3) a very high triglyceride content, (4) a very low total content of MCPD fatty acid esters and (5) a very low total content of glycidol esters of fatty acids, wherein this purity specification can be reached independently from any specific synthesis route. Such combination of purity properties (1) to (5) was deemed incompatible or non-realizable so far; however, as applicant has surprisingly found out, the inventive process renders such combination of purity properties possible for the very first time.

A further subject-matter - according to a **second** aspect of the present invention - is the reaction product obtained or obtainable by the process according to the invention. In other words, according this aspect of the present invention, the present invention relates to a tricaprylin obtainable or obtained by the process according to the present of the present invention as delineated and defined hereinabove.

Especially, a subject-matter of the present invention - according to this aspect of the present invention - is a tricaprylin, especially a tricaprylin obtainable or obtained by the process according to the present of the present invention,
wherein the tricaprylin corresponds to formula (II)

   CH₂[O-C(O)R¹]-CH[O-C(O)R¹]-CH₂[O-C(O)R¹] (II)
wherein in formula (II) the radical R¹ represents a linear saturated aliphatic C₇-alkyl radical of formula - (CH₂)₆ - CH₃ (i.e. a n-heptylic radical);
wherein the tricaprylin of formula (II) is characterized by and/or exhibits the following properties (1) to (5) in their combination and/or fulfils the following product specification:
   (1) acid value (AV), especially determined according to Ph. Eur. 2.5.1 (European Pharmacopoeia 10.0): ≤ 0.5 mg KOH / g, especially ≤ 0.2 mg KOH / g, preferably ≤ 0.1 mg KOH / g; and
   (2) hydroxyl value (OHV), especially determined according to Ph. Eur. 2.5.3 (European Pharmacopoeia 10.0): ≤ 0.5 mg KOH / g, especially ≤ 0.2 mg KOH / g, preferably ≤ 0.1 mg KOH / g; and
   (3) triglyceride content, especially determined according to Ph. Eur. 2.2.28 (European Pharmacopoeia 10.0): ≥ 99 %, especially ≥ 99.2 %, preferably ≥ 99.5 %, each based on the tricaprylin; and
   (4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.5 ppm, especially ≤ 0.2 ppm, preferably ≤ 0.1 ppm, each based on the tricaprylin; and
   (5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.5 ppm, especially ≤ 0.2 ppm, preferably ≤ 0.1 ppm, each based on the tricaprylin.

Especially, a subject-matter of the present invention is a tricaprylin, especially according to the above definition,
wherein the tricaprylin of formula (II) is characterized by and/or exhibits the following properties (1) to (5) in their combination and/or fulfils the following product specification:
(1) acid value (AV), especially determined according to Ph. Eur. 2.5.1 (European Pharmacopoeia 10.0): ≤ 0.1 mg KOH / g; and
(2) hydroxyl value (OHV), especially determined according to Ph. Eur. 2.5.3 (European Pharmacopoeia 10.0): ≤ 0.1 mg KOH / g; and
(3) triglyceride content, especially determined according to Ph. Eur. 2.2.28 (European Pharmacopoeia 10.0): ≥ 99.5 %, especially ≥ 99.6 %, preferably ≥ 99.8 %, each based on the tricaprylin; and
(4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.1 ppm based on the tricaprylin; and
(5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.1 ppm based on the tricaprylin.

The inventive reaction product, i.e. tricaprylin, is linked to and unifies a multitude of advantageous and particular properties and characteristics, which have already been delineated hereinabove in detail in context with the inventive process; thus, in order to avoid unnecessary repetitions, reference may be made to these above explanations.

Above all, the inventive reaction product, i.e. the inventive tricaprylin, is particularly appropriate for applications requiring high purity grades and at the same time high active ingredient levels, e.g. for nutritional and above all also for pharmaceutical applications and uses (e.g. even for parenteral applications in high dosages and/or with high concentrations).

Furthermore, the inventive reaction product, i.e. the inventive tricaprylin, unifies the aforementioned five properties (1) to (5), which have been deemed so far incompatible with one another in their overall combination. For, according to prior art methods, when increasing the triglyceride content and lowering acid and hydroxyl values, usually high total contents of MCPD and glycidol fatty acid esters are effected due to drastic esterification conditions; in turn, when lowering the total contents of MCPD and glycidol fatty acid esters, according to the prior art, relatively low triglyceride contents with high acid and hydroxyl values are reached. In contrast to this prior art, the present invention renders it, for the first time, possible to provide a combination of all aforementioned five properties (1) to (5) in one and the same product. Consequently, the inventive reaction product (i.e. tricaprylin) resulting from the inventive process is highly suitable for food and pharmaceutical applications, even in parenteral applications with high dosages and/or concentrations.

As to further details relating to the second aspect of the invention, for avoidance of unnecessary repetition, reference may be made to the above explanations and remarks relating to the first aspect of the present invention (i.e. process of the present invention), which explanations and remarks accordingly apply *mutatis mutandis* also to this second aspect of the present invention.

Likewise, the present invention - according to a **third** aspect of the present invention - relates to a pharmaceutical composition, especially a drug or medicament, comprising the inventive tricaprylin according to the above definition, especially together with a physiologically acceptable excipient.

Especially, according to this aspect of the invention, the present invention relates to a pharmaceutical composition for the prophylactic and/or therapeutic treatment or for use in the prophylactic and/or therapeutic treatment of diseases of the human or animal body, especially diseases associated with a disorder of the energy metabolism, especially ketone body metabolism, such as especially craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, fat metabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, especially ulcerative colitis and Crohn's disease, lyosomal storage diseases such as sphingolipidosis, especially Niemann-Pick disease, diabetes mellitus, migraine and migraine headaches, epilepsy, especially infantile spasms as well as effects or side-effects of chemotherapy.

In particular, according to this aspect of the invention, the present invention relates to a pharmaceutical composition for the prophylactic and/or therapeutic treatment or for use in the prophylactic and/or therapeutic treatment of diseases of the human body selected among Alzheimer's disease, especially mild to moderate forms of Alzheimer's disease; migraine and migraine headaches; as well as epilepsy, especially infantile spasms.

As to further details relating to the third aspect of the invention, for avoidance of unnecessary repetition, reference may be made to the above explanations and remarks relating to the first and second aspects of the present invention, which explanations and remarks accordingly apply *mutatis mutandis* also to this third aspect of the present invention.

Furthermore, the present invention - according to a **fourth** aspect of the present invention - also relates to the inventive tricaprylin according to the above definition for the prophylactic and/or therapeutic treatment or for use in the prophylactic and/or therapeutic treatment of diseases of the human or animal body, especially diseases associated with a disorder of the energy metabolism, especially ketone body metabolism, such as especially craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, fat metabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, especially ulcerative colitis and Crohn's disease, lyosomal storage diseases such as sphingolipidosis, especially Niemann-Pick disease, diabetes mellitus, migraine and migraine headaches, epilepsy, especially infantile spasms as well as effects or side-effects of chemotherapy.

Especially, according to this aspect of the invention, the present invention relates to the inventive tricaprylin according to the above definition for the prophylactic and/or therapeutic treatment or for use in the prophylactic and/or therapeutic treatment of diseases of the human body selected among Alzheimer's disease, especially mild to moderate forms of Alzheimer's disease; migraine and migraine headaches; as well as epilepsy, especially infantile spasms.

As to further details relating to the fourth aspect of the invention, for avoidance of unnecessary repetition, reference may be made to the above explanations and remarks relating to the first, second and third aspects of the present invention, which explanations and remarks accordingly apply *mutatis mutandis* also to this fourth aspect of the present invention.

Further, the present invention - according to a **fifth** aspect of the present invention - also relates to the inventive use of the inventive tricaprylin according to the above definition for the prophylactic and/or therapeutic treatment or for producing a medicament for the prophylactic and/or therapeutic treatment of diseases of the human or animal body, especially diseases associated with a disorder of the energy metabolism, especially ketone body metabolism, such as especially craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, fat metabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, especially ulcerative colitis and Crohn's disease, lyosomal storage diseases such as sphingolipidosis, especially Niemann-Pick disease, diabetes mellitus, migraine and migraine headaches, epilepsy, especially infantile spasms as well as effects or side-effects of chemotherapy.

Especially, according to this aspect of the invention, the present invention relates to the inventive use of the inventive tricaprylin according to the above definition for the prophylactic and/or therapeutic treatment or for producing a medicament for the prophylactic and/or therapeutic treatment of diseases of the human body selected among Alzheimer's disease, especially mild to moderate forms of Alzheimer's disease; migraine and migraine headaches; as well as epilepsy, especially infantile spasms.

In particular, according to this aspect of the invention, the present invention relates to the inventive use of the inventive tricaprylin according to the above definition for the prophylactic and/or therapeutic treatment or for producing a medicament for the prophylactic and/or therapeutic treatment of or for the application for catabolic metabolic states, such as hunger, diets or low-carbohydrate nutrition.

As to further details relating to the fifth aspect of the invention, for avoidance of unnecessary repetition, reference may be made to the above explanations and remarks relating to the first, second, third and fourth aspects of the present invention, which explanations and remarks accordingly apply *mutatis mutandis* also to this fifth aspect of the present invention.

Furthermore, the present invention - according to a **sixth** aspect of the present invention - relates to a food and/or a nutritive or food composition and/or a food product and/or a medical food comprising the inventive tricaprylin according to the above definition.

Especially, according to this aspect of the invention, the food and/or the nutritive or food composition and/or the food product and/or the medical food may be e.g. a dietary supplement, a functional food, a novel food, a food additive, a food supplement, a dietary food, a power snack, an appetite suppressant or a strength and/or endurance sports supplement.

As to further details relating to the sixth aspect of the invention, for avoidance of unnecessary repetition, reference may be made to the above explanations and remarks relating to the first, second, third, fourth and fifth aspects of the present invention, which explanations and remarks accordingly apply *mutatis mutandis* also to this sixth aspect of the present invention.

Likewise, the present invention - according to a **seventh** aspect of the present invention - relates to the inventive use of the inventive tricaprylin according to the above definition in a food and/or in a nutritive or food composition and/or in a food product and/or in a medical food.

Especially, according to this aspect of the invention, the food and/or the nutritive or food composition and/or the food product and/or the medical food may be e.g. a dietary supplement, a functional food, a novel food, a food additive, a food supplement, a dietary food, a power snack, an appetite suppressant or a strength and/or endurance sports supplement.

As to further details relating to the seventh aspect of the invention, for avoidance of unnecessary repetition, reference may be made to the above explanations and remarks relating to the first, second, third, fourth, fifth and sixth aspects of the present invention, which explanations and remarks accordingly apply *mutatis mutandis* also to this seventh aspect of the present invention.

Also, the present invention - according to an **eighth** aspect of the present invention - furthermore relates to the inventive use of the inventive tricaprylin according to the above definition as an additive or aid, especially as a carrier or excipient, a solubilizer, a release agent, a surface-treatment agent, a transport agent, a lubricant, hydrophobic agent, a film-forming or protective agent or a viscosity regulator, preferably as a carrier or excipient, in pharmaceutical compositions, especially drugs or medicaments, in food, nutritive or food compositions, food products and/or medical foods as well as in cosmetic compositions.

Especially, according to this aspect of the invention, the present invention relates to the inventive use of the inventive tricaprylin according to the above definition as a carrier or excipient in pharmaceutical compositions, especially drugs or medicaments.

Furthermore, according to this aspect of the invention, the present invention relates also to the inventive use of the inventive tricaprylin according to the above definition as an active substance (i.e. active ingredient) in pharmaceutical compositions, especially drugs or medicaments, in food, nutritive or food compositions, food products and/or medical foods or in cosmetic compositions, especially together with an excipient, particularly together with a physiologically acceptable excipient.

Moreover, according to this aspect of the invention, the present invention relates to the inventive use of the inventive tricaprylin according to the above definition as an active substance (i.e. active ingredient, API) in pharmaceutical compositions.

Further, according to this aspect of the invention, the present invention also relates to the inventive use of the inventive tricaprylin according to the above definition as an active substance (i.e. active ingredient, API) in pharmaceutical compositions, especially drugs or medicaments, especially together with a physiologically acceptable excipient.

As to further details relating to the eighth aspect of the invention, for avoidance of unnecessary repetition, reference may be made to the above explanations and remarks relating to the first, second, third, fourth, fifth, sixth and seventh aspects of the present invention, which explanations and remarks accordingly apply *mutatis mutandis* also to this eighth aspect of the present invention.

Finally, the present invention - according to a **ninth** aspect of the present invention - also relates to the use of a nucleophilic agent for purifying a tricaprylin, especially tricaprylin of formula (II) as defined hereinabove, and/or for a preferably selective removal or derivatization of halide-based impurities present in a tricaprylin, especially a tricaprylin of formula (II) as defined hereinabove.

Especially, according to this aspect of the invention, the nucleophilic agent may be e.g. a nucleophilic agent capable of removing halide-based impurities, preferably selected from the group consisting of hydrogen sulfites (bisulfites, HSO₃⁻), sulfites (SO₃²⁻), hydrogen thiosulfates (HS₂O₃⁻), thiosulfates (S₂O₃²⁻), hydrogen phosphites (H₂PO₃⁻), phosphites (HPO₃²⁻) and phosphines as well as combinations thereof, more preferably hydrogen sulfites (bisulfites, HSO₃⁻).

Usually, according to a particular embodiment according to this aspect of the invention, the tricaprylin to be purified is treated and/or contacted with the nucleophilic agent.

Generally, it is preferred that the treating and/or contacting of the tricaprylin to be purified with the nucleophilic agent is performed as a one-stage or single-step treatment. However, particularly in case of higher degrees of impurifications, it is also possible to perform the treating and/or contacting of the tricaprylin to be purified with the nucleophilic agent repeatedly, especially in two or more cycles and/or as a multistage process. Nevertheless, it is preferred to perform the treating and/or contacting of the tricaprylin to be purified with the nucleophilic agent in one stage (i.e. without any repeated treating and/or contacting); particularly in case of higher degrees of impurifications, it is also possible, as an alternative embodiment, to use higher amounts or concentrations of the nucleophilic agent instead of performing a repeated treating and/or contacting.

Especially, according to another particular embodiment according to this aspect of the invention, the nucleophilic agent is used for purifying tricaprylin from halide-based, especially chlorine-based impurities and/or for removing halide-based, especially chlorine-based impurities from tricaprylin, particularly chlorine-based impurities selected from the group consisting of MCPD fatty acid esters (fatty acid esters of monochloropropanediols), glycidol esters of fatty acids and combinations thereof.

In particular, according to yet another particular embodiment according to this aspect of the invention, the halide-based impurities are chlorine-based impurities, especially chlorine-based impurities selected from the group consisting of MCPD fatty acid esters (fatty acid esters of monochloropropanediols), glycidol esters of fatty acids and combinations thereof.

Particularly, according to a further particular embodiment according to this aspect of the invention, the tricaprylin to be purified is usually treated and/or contacted with the nucleophilic agent, especially with a preferably water-based solution of the nucleophilic agent.

Especially, the tricaprylin to be purified is treated and/or contacted with the nucleophilic agent under reaction conditions, especially at reaction temperatures and/or for reaction durations, sufficient to remove the impurities.

In particular, the tricaprylin to be purified is treated and/or contacted with the nucleophilic agent at temperatures in the range of from 10 °C to 175 °C, preferably in the range of from 25 °C to 120 °C, more preferably in the range of from 50 °C to 100 °C, and/or for a duration in the range of from 0.001 to 50 hours, preferably in the range of from 0.01 to 30 hours, more preferably in the range of from 0.1 to 20 hours.

Thus, on the whole, nucleophilic agents, such as e.g. hydrogen sulfites (bisulfites, HSO₃⁻), sulfites (SO₃²⁻), hydrogen thiosulfates (HS₂O₃⁻), thiosulfates (S₂O₃²⁻), hydrogen phosphites (H₂PO₃⁻), phosphites (HPO₃²⁻) and phosphines as well as combinations thereof, are an excellent reactant agent for purifying tricaprylin, especially for removing halide-based impurities, especially chlorine-based impurities, from tricaprylin, particularly chlorine-based impurities selected from the group consisting of MCPD fatty acid esters (fatty acid esters of monochloropropanediols), glycidol esters of fatty acids and combinations thereof (i.e. via selective derivatization of halide-based impurities).

As to further details relating to the ninth aspect of the invention, for avoidance of unnecessary repetition, reference may be made to the above explanations and remarks relating to the first, second, third, fourth, fifth, sixth, seventh and eighth aspects of the present invention, which explanations and remarks accordingly apply *mutatis mutandis* also to this ninth aspect of the present invention.

Further embodiments, modifications and variations of the present invention are readily recognizable or realizable by a person skilled in the art when reading the description, without leaving the scope of the present invention.

The present invention is illustrated by the following examples, which are not intended to limit the present invention in any way, but only to explain the exemplary and non-limiting implementation and configuration of the present invention.

### EXAMPLES:

### Examples of production

The process in accordance with the invention is illustrated by the following examples, wherein the different modes of synthesis have been applied, as will be described in detail hereinafter.

In all inventive Examples, the used starting materials 1 ,2,3-propantriol (glycerol, glycerine) and solketal (isopropylidene glycerol), respectively, are each at least essentially free of any chlorinated species, i.e. each comprise a total chlorinated species content of at most 0.1 ppm (based on the 1,2,3-propantriol or solketal, respectively). Furthermore, the total concentration of the sum of MCPD fatty acid esters and glycidol fatty acid esters is under the detection limit (≤ 0.1 ppm of total MCPD and glycidol esters in sum, based on glycerol or solketal, respectively).

Furthermore, in all inventive Examples, esterification is run at temperatures not exceeding 100 °C, preferably not exceeding 80 °C.

Also, in all inventive Examples, esterification is performed in the absence of any metal-based catalyst.

Finally, all inventive Examples include selective derivatization, i.e. chemical treatment with nucleophilic agents, in order to remove any traces of halide-based impurities, especially of MCPD fatty acid esters and glycidol fatty acid esters. In all inventive Examples, product purity requirements are met even without such treatment; however, such selective derivatization, i.e. chemical treatment with nucleophilic agents, allows even increase of purity grade since even present traces of these impurities are eliminated selectively.

### 1. Synthesis Route (A)

Tricaprylin is produced according to above-described Synthesis Route (A). For this purpose, the following procedure is applied:
Caprylic acid (i.e. saturated linear C₈-fatty acid or octanoic acid) is reacted with glycerol in the presence of an immobilized enzyme as a catalyst (CALB lipase on polymer support, derived from *Candida antarctica,* e. g. Novozym^{®} 435 from Sigma-Aldrich or Merck or Lipozym^{®} 435 from Strem Chemicals, Inc.; 1 % by weight, based on the reaction mixture) at 80 °C under vacuum (< 100 mbar). Caprylic acid is used in amounts of up to 300 mol-%, preferably of up to 100 mol-%, based on the OH-groups of the glycerol. Then the excess of caprylic acid is removed using a short-path-distillation (<< 180 °C, preferably 120 °C to 130 °C; << 0,1 mbar, preferably < 2 mbar). A dicaprylin, especially 1,3-dicaprylin (1,3-diglyceride of caprylic acid), is obtained.

Subsequently the dicaprylin is reacted with caprylic acid anhydride at temperatures below 180°C, preferably between 80 °C and 150 °C, until the hydroxyl value (OHV) is below 0.1 mg KOH / g. The resulting product is treated in a usual way to remove the caprylic acid formed as by-product (i.e. short-path distillation or the like).

The resulting tricaprylin product is analyzed as follows:
- acid value (AV) determined according to Ph. Eur. 2.5.1 (European Pharmacopoeia 10.0): ≤ 0.1 mg KOH / g,
- hydroxyl value (OHV) determined according to Ph. Eur. 2.5.3 (European Pharmacopoeia 10.0): ≤ 0.1 mg KOH / g,
- triglyceride content determined according to Ph. Eur. 2.2.28 (European Pharmacopoeia 10.0): ≥ 99.4 % based on the reaction product (i.e. tricaprylin),
- total content of MCPD fatty acid esters (fatty acid esters of monochloropropanediols) determined according to method of DGF C-VI 18 (10) (Einheitsmethode der Deutschen Gesellschaft für Fettwissenschaft [Standard method of German Society for Fat Science]): 0.14 ppm based on the reaction product (i.e. tricaprylin),
- total content of glycidol esters of fatty acids determined according to method of DGF C-VI 18 (10) (Einheitsmethode der Deutschen Gesellschaft für Fettwissenschaft e.V. [Standard method of German Society for Fat Science]): 0.12 ppm based on the reaction product (i.e. tricaprylin).

The tricaprylin product is then further subjected to a treatment with sodium hydrogen sulfite (NaHSO₃) in the form of a water-based solution (80 °C, 6 hours) as a nucleophilic agent (selective derivatization of halide-based impurities, i.e. MCPD fatty acid esters and glycidol fatty acid esters). After separating the water-based phase and drying the resulting tricaprylin product, the product quality as a whole is further increased since the triglyceride content is increased to values ≥ 99.5 % and the total content of MCPD fatty acid esters and that of glycidol esters of fatty acids is each below the detection limit (i.e. ≤ 0.1 ppm each). Optionally, the thus treated product may also be further treated with bleaching earth and/or activated carbon, optionally followed by deodorization (e.g. using pressurized steam). The resulting tricaprylin product, after treatment with sodium hydrogen sulfite (NaHSOs) and optional further treatments, is analyzed as follows:
- acid value (Ph. Eur. 2.5.1): ≤ 0.1 mg KOH / g,
- hydroxyl value (Ph. Eur. 2.5.3): ≤ 0.1 mg KOH / g,
- triglyceride content (Ph. Eur. 2.2.28): ≥ 99.5 % based on tricaprylin,
- total content of MCPD fatty acid esters (DGF C-VI 18 (10)): ≤ 0.1 ppm based on tricaprylin,
- total content of glycidol esters of fatty acids (DGF C-VI 18 (10)): ≤ 0.1 ppm based on tricaprylin.

Comparable results are reached when, instead of sodium hydrogen sulfite (NaHSOs), also other nucleophilic agents are used (i.e. sodium hydrogen thiosulfate NaHS₂O₃, sodium hydrogen phosphite NaH₂PO₃ and phosphines). The obtained results are comparable.

The aforedescribed production procedure is repeated in an analogous manner, however, using different starting materials, namely the respective C₁-C₄-esters of caprylic acid. In each approach comparable results are obtained.

In an analogous manner, also triheptanoin, on the one hand, and a medium-chain triglyceride (MCT) on the basis of caproic acid (C₆) / caprylic acid (Cs) / capric acid (C₁₀), on the other hand, are produced each with comparable results (characterization after treatment with hydrogen sulfite both for triheptanoin and for MCT each: acid value: ≤ 0.1 mg KOH / g; hydroxyl value: ≤ 0.1 mg KOH / g; triglyceride content: ≥ 99.5 %; total content of MCPD fatty acid esters: ≤ 0.1 ppm; total content of glycidol esters: ≤ 0.1 ppm).

### Comparative Procedure (not of the invention)

In a non-inventive manner, Synthesis Route (A) for tricaprylin is performed with the following deviations: The glycerol used as starting material comprises a total chlorinated species content of about 30 ppm. The esterification catalyst used is a metal-based catalyst on the basis of tetrabutyl titanate (TBT). Esterification is performed in the temperature range between 190 °C and 230 °C.

The resulting tricaprylin is analyzed as follows: acid value (Ph. Eur. 2.5.1): > 0.6 mg KOH / g, hydroxyl value (Ph. Eur. 2.5.3): > 0.7 mg KOH / g, triglyceride content (Ph. Eur. 2.2.28): 97.5 % based on tricaprylin, total content of MCPD fatty acid esters (DGF C-VI 18 (10)): 0.9 ppm based on tricaprylin, total content of glycidol esters of fatty acids (DGF C-VI 18 (10)): 0.7 ppm based on tricaprylin.

Subsequent treatment with sodium hydrogen sulfite (NaHSOs) in the form of a water-based solution (80 °C, 6 hours) can lower both the total content of MCPD fatty acid esters and total content of glycidol esters of fatty acids to values of about 0.4 ppm each, however, whereas not improving or influencing the parameters of acid value, hydroxyl value and triglyceride content.

### 2. Synthesis Route (B)

Tricaprylin is produced according to above-described Synthesis Route (B). For this purpose, the following procedure is applied:
Caprylic acid (i.e. saturated linear C₈-fatty acid or octanoic acid) is reacted with glycerol in the presence of an immobilized enzyme as a catalyst (CALB lipase on polymer support, derived from *Candida antarctica,* e. g. Novozym^{®} 435 from Sigma-Aldrich or Merck or Lipozym^{®} 435 from Strem Chemicals, Inc.; 1 % by weight, based on the reaction mixture) at 80 °C under vacuum (< 100 mbar). Caprylic acid is used in amounts of up to 300 mol-%, preferably of up to 100 mol-%, based on the OH-groups of the glycerol. Then the excess of caprylic acid is removed using a short-path-distillation (<< 180 °C, preferably 120 °C to 130 °C; << 0,1 mbar, preferably < 2 mbar). A dicaprylin, especially 1,3-dicaprylin (1,3-diglyceride of caprylic acid), is obtained.

Subsequently the dicaprylin is reacted with caprylic acid in the presence of an enzyme, especially an enzyme for regioselective esterification of the 2-position if 1,3-diglyceride (e.g. *Pseudomonas fluorescens* lipase in a first approach and, in an alternative second approach, *Burkholderia cepacia* lipase, each immobilized on chitosan) at 50 °C until the hydroxyl value (OHV) is below 0.1 mg KOH / g.

The resulting tricaprylin product is analyzed as follows:
- acid value (Ph. Eur. 2.5.1): ≤ 0.1 mg KOH / g,
- hydroxyl value (Ph. Eur. 2.5.3): ≤ 0.1 mg KOH / g,
- triglyceride content (Ph. Eur. 2.2.28): ≥ 99.5 % based on tricaprylin,
- total content of MCPD fatty acid esters (DGF C-VI 18 (10)): 0.17 ppm based on tricaprylin,
- total content of glycidol esters of fatty acids (DGF C-VI 18 (10)): 0.19 ppm based on tricaprylin.

The tricaprylin product is then further subjected to a treatment with sodium hydrogen sulfite (NaHSOs) in the form of a water-based solution (80 °C, 6 hours) as a nucleophilic agent (selective derivatization of halide-based impurities, i.e. MCPD fatty acid esters and glycidol fatty acid esters). After separating the water-based phase and drying the resulting tricaprylin product, the product quality as a whole is further increased since the total content of MCPD fatty acid esters and that of glycidol esters of fatty acids is each below the detection limit (i.e. ≤ 0.1 ppm each). Optionally, the thus treated product may also be further treated with bleaching earth and/or activated carbon, optionally followed by deodorization (e.g. using pressurized steam).

The resulting tricaprylin product, after treatment with sodium hydrogen sulfite (NaHSOs) and optional further treatments, is analyzed as follows:
- acid value (Ph. Eur. 2.5.1): ≤ 0.1 mg KOH / g,
- hydroxyl value (Ph. Eur. 2.5.3): ≤ 0.1 mg KOH / g,
- triglyceride content (Ph. Eur. 2.2.28): ≥ 99.5 % based on tricaprylin,
- total content of MCPD fatty acid esters (DGF C-VI 18 (10)): ≤ 0.1 ppm based on tricaprylin,
- total content of glycidol esters of fatty acids (DGF C-VI 18 (10)): ≤ 0.1 ppm based on tricaprylin.

The aforedescribed production procedure is repeated in an analogous manner, however, using different starting materials, namely the respective C₁-C₄-esters of caprylic acid. In each approach comparable results are obtained.

In an analogous manner, also triheptanoin, on the one hand, and a medium-chain triglyceride (MCT) on the basis of caproic acid (C₆) / caprylic acid (Cs) / capric acid (C₁₀), on the other hand, are produced each with comparable results (characterization after treatment with hydrogen sulfite both for triheptanoin and for MCT each: acid value: ≤ 0.1 mg KOH / g; hydroxyl value: ≤ 0.1 mg KOH / g; triglyceride content: ≥ 99.5 %; total content of MCPD fatty acid esters: ≤ 0.1 ppm; total content of glycidol esters: ≤ 0.1 ppm).

### 3. Synthesis Route (C)

Tricaprylin is produced according to above-described Synthesis Route (C). For this purpose, the following procedure is applied:
Caprylic acid anhydride (i.e. linear saturated C₈-fatty acid anhydride or octanoic acid anhydride) is reacted with glycerol below 180 °C, preferably between 80 °C and 150 °C, until the hydroxyl value (OHV) is below 0.1 mg KOH / g. Then, the formed caprylic acid (by-product) is removed using a short-path-distillation (<< 180 °C, preferably 120 °C to 130 °C; << 0,1 mbar, preferably < 2 mbar).

The resulting tricaprylin product is analyzed as follows:
- acid value (Ph. Eur. 2.5.1): ≤ 0.1 mg KOH / g,
- hydroxyl value (Ph. Eur. 2.5.3): ≤ 0.1 mg KOH / g,
- triglyceride content (Ph. Eur. 2.2.28): ≥ 99.5 % based on tricaprylin,
- total content of MCPD fatty acid esters (DGF C-VI 18 (10)): 0.16 ppm based on tricaprylin,
- total content of glycidol esters of fatty acids (DGF C-VI 18 (10)): 0.12 ppm based on tricaprylin.

The tricaprylin product is then further subjected to a treatment with sodium hydrogen sulfite (NaHSOs) in the form of a water-based solution (80 °C, 6 hours) as a nucleophilic agent (selective derivatization of halide-based impurities, i.e. MCPD fatty acid esters and glycidol fatty acid esters). After separating the water-based phase and drying the resulting tricaprylin product, the product quality as a whole is further increased since the total content of MCPD fatty acid esters and that of glycidol esters of fatty acids is each below the detection limit (i.e. ≤ 0.1 ppm each). Optionally, the thus treated product may also be further treated with bleaching earth and/or activated carbon, optionally followed by deodorization (e.g. using pressurized steam).

The resulting tricaprylin product, after treatment with sodium hydrogen sulfite (NaHSOs) and optional further treatments, is analyzed as follows:
- acid value (Ph. Eur. 2.5.1): ≤ 0.1 mg KOH / g,
- hydroxyl value (Ph. Eur. 2.5.3): ≤ 0.1 mg KOH / g,
- triglyceride content (Ph. Eur. 2.2.28): ≥ 99.5 % based on tricaprylin,
- total content of MCPD fatty acid esters (DGF C-VI 18 (10)): ≤ 0.1 ppm based on tricaprylin,
- total content of glycidol esters of fatty acids (DGF C-VI 18 (10)): ≤ 0.1 ppm based on tricaprylin.

In an analogous manner, also triheptanoin, on the one hand, and a medium-chain triglyceride (MCT) on the basis of caproic acid (C₆) / caprylic acid (C₈) / capric acid (C₁₀), on the other hand, are produced each with comparable results (characterization after treatment with hydrogen sulfite both for triheptanoin and for MCT each: acid value: ≤ 0.1 mg KOH / g; hydroxyl value: ≤ 0.1 mg KOH / g; triglyceride content: ≥ 99.5 %; total content of MCPD fatty acid esters: ≤ 0.1 ppm; total content of glycidol esters: ≤ 0.1 ppm).

### 4. Synthesis Route (D)

Tricaprylin is produced according to above-described Synthesis Route (D). For this purpose, the following procedure is applied:
Solketal and caprylic acid anhydride (i.e. linear saturated C₈-fatty acid anhydride or octanoic acid anhydride) are combined in a flask and the system is evacuated. The mixture is reacted for 6 hours at temperatures below 180 °C, preferably between 80 °C and 150 °C. Subsequently, the caprylic acid formed as a by-product is distilled off at < 20 mbar and 130 °C. A solketal monoester of caprylic acid is obtained with a purity of < 95 %.

Subsequently, the solketal monoester of caprylic acid is subjected to an acid-induced deprotection reaction (i.e. ring-opening). Either diluted sulfuric acid or diluted phosphoric acid is used to adjust the pH value preferably to about 1 and the acetone formed as a by-product from deprotection is removed.

Then, the resulting glycerol monoester of caprylic acid (i.e. monoglyceride of caprylic acid) is further reacted with caprylic acid anhydride for 90 min at 80 °C to yield the triglyceride (i.e. tricaprylin). After the reaction is completed, the caprylic acid formed as a by-product is distilled off at < 20 mbar and 130 °C.

The resulting tricaprylin product is analyzed as follows:
- acid value (Ph. Eur. 2.5.1): ≤ 0.1 mg KOH / g,
- hydroxyl value (Ph. Eur. 2.5.3): ≤ 0.1 mg KOH / g,
- triglyceride content (Ph. Eur. 2.2.28): ≥ 99.2 % based on tricaprylin,
- total content of MCPD fatty acid esters (DGF C-VI 18 (10)): 0.15 ppm based on tricaprylin,
- total content of glycidol esters of fatty acids (DGF C-VI 18 (10)): 0.13 ppm based on tricaprylin.

The tricaprylin product is then further subjected to a treatment with sodium hydrogen sulfite (NaHSOs) in the form of a water-based solution (80 °C, 6 hours) as a nucleophilic agent (selective derivatization of halide-based impurities, i.e. MCPD fatty acid esters and glycidol fatty acid esters). After separating the water-based phase and drying the resulting tricaprylin product, the product quality as a whole is further increased since the triglyceride content is increased to values ≥ 99.5 % and the total content of MCPD fatty acid esters and that of glycidol esters of fatty acids is each below the detection limit (i.e. ≤ 0.1 ppm each). Optionally, the thus treated product may also be further treated with bleaching earth and/or activated carbon, optionally followed by deodorization (e.g. using pressurized steam). The resulting tricaprylin product, after treatment with sodium hydrogen sulfite (NaHSOs) and optional further treatments, is analyzed as follows:
- acid value (Ph. Eur. 2.5.1): ≤ 0.1 mg KOH / g,
- hydroxyl value (Ph. Eur. 2.5.3): ≤ 0.1 mg KOH / g,
- triglyceride content (Ph. Eur. 2.2.28): ≥ 99.5 % based on tricaprylin,
- total content of MCPD fatty acid esters (DGF C-VI 18 (10)): ≤ 0.1 ppm based on tricaprylin,
- total content of glycidol esters of fatty acids (DGF C-VI 18 (10)): ≤ 0.1 ppm based on tricaprylin.

In an analogous manner, also triheptanoin, on the one hand, and a medium-chain triglyceride (MCT) on the basis of caproic acid (C₆) / caprylic acid (Cs) / capric acid (C₁₀), on the other hand, are produced each with comparable results (characterization after treatment with hydrogen sulfite both for triheptanoin and for MCT each: acid value: ≤ 0.1 mg KOH / g; hydroxyl value: ≤ 0.1 mg KOH / g; triglyceride content: ≥ 99.5 %; total content of MCPD fatty acid esters: ≤ 0.1 ppm; total content of glycidol esters: ≤ 0.1 ppm).

### 5. Synthesis Route (E)

Tricaprylin is produced according to above-described Synthesis Route (E). For this purpose, the following procedure is applied:
Solketal and caprylic acid (i.e. linear saturated C₈-fatty acid or octanoic acid) are mixed with an immobilized enzyme as a catalyst (CALB lipase on polymer support, derived from *Candida antarctica,* e. g. Novozym^{®} 435 from Sigma-Aldrich or Merck or Lipozym^{®} 435 from Strem Chemicals, Inc.; 1 % by weight, based on the reaction mixture) and reacted for 6 hours at 80 °C under N₂-atmosphere. Then the enzyme is filtered off. A solketal monoester of caprylic acid is obtained.

Subsequently, the solketal monoester of caprylic acid is subjected to an acid-induced deprotection reaction (i.e. ring-opening). Either diluted sulfuric acid or diluted phosphoric acid is used to adjust the pH value preferably to about 1 and the acetone formed as a by-product from deprotection is removed.

Then, the resulting glycerol monoester of caprylic acid (i.e. monoglyceride of caprylic acid) is further reacted with caprylic acid anhydride for 90 min at 80 °C to yield the triglyceride (i.e. tricaprylin). After the reaction is completed, the caprylic acid formed as a by-product is distilled off at < 20 mbar and 130 °C.

The resulting tricaprylin product is analyzed as follows:
- acid value (Ph. Eur. 2.5.1): ≤ 0.1 mg KOH / g,
- hydroxyl value (Ph. Eur. 2.5.3): ≤ 0.1 mg KOH / g,
- triglyceride content (Ph. Eur. 2.2.28): ≥ 99.4 % based on tricaprylin,
- total content of MCPD fatty acid esters (DGF C-VI 18 (10)): 0.15 ppm based on tricaprylin,
- total content of glycidol esters of fatty acids (DGF C-VI 18 (10)): 0.12 ppm based on tricaprylin.

The tricaprylin product is then further subjected to a treatment with sodium hydrogen sulfite (NaHSOs) in the form of a water-based solution (80 °C, 6 hours) as a nucleophilic agent (selective derivatization of halide-based impurities, i.e. MCPD fatty acid esters and glycidol fatty acid esters). After separating the water-based phase and drying the resulting tricaprylin product, the product quality as a whole is further increased since the triglyceride content is increased to values ≥ 99.5 % and the total content of MCPD fatty acid esters and that of glycidol esters of fatty acids is each below the detection limit (i.e. ≤ 0.1 ppm each). Optionally, the thus treated product may also be further treated with bleaching earth and/or activated carbon, optionally followed by deodorization (e.g. using pressurized steam). The resulting tricaprylin product, after treatment with sodium hydrogen sulfite (NaHSOs) and optional further treatments, is analyzed as follows:
- acid value (Ph. Eur. 2.5.1): ≤ 0.1 mg KOH / g,
- hydroxyl value (Ph. Eur. 2.5.3): ≤ 0.1 mg KOH / g,
- triglyceride content (Ph. Eur. 2.2.28): ≥ 99.5 % based on tricaprylin,
- total content of MCPD fatty acid esters (DGF C-VI 18 (10)): ≤ 0.1 ppm based on tricaprylin,
- total content of glycidol esters of fatty acids (DGF C-VI 18 (10)): ≤ 0.1 ppm based on tricaprylin.

In an analogous manner, also triheptanoin, on the one hand, and a medium-chain triglyceride (MCT) on the basis of caproic acid (C₆) / caprylic acid (Cs) / capric acid (C₁₀), on the other hand, are produced each with comparable results (characterization after treatment with hydrogen sulfite both for triheptanoin and for MCT each: acid value: ≤ 0.1 mg KOH / g; hydroxyl value: ≤ 0.1 mg KOH / g; triglyceride content: ≥ 99.5 %; total content of MCPD fatty acid esters: ≤ 0.1 ppm; total content of glycidol esters: ≤ 0.1 ppm).

### 6. Synthesis Route (F)

Tricaprylin is produced according to above-described Synthesis Route (F). For this purpose, the following procedure is applied:
2-Octenoic acid is esterified with glycerol (≤ 0,1 ppm of total sum of MCPD fatty acid esters and glycidol esters; ≤ 0,1 ppm total chlorinated species content), using an excess of 2-octenoic acid (≤ 50 mol-%, preferably ≤ 30mol-%, based on the OH-groups of the glycerol) in the presence of an immobilized enzyme as a catalyst (CALB lipase on polymer support, derived from *Candida antarctica,* e. g. Novozym^{®} 435 from Sigma-Aldrich or Merck or Lipozym^{®} 435 from Strem Chemicals, Inc.; ≤ 0,5 % by weight, preferably ≤ 0,1 % by weight, based on the reaction mixture) at 70 °C under vacuum (≤ 100 mbar). The water formed as a reaction by-product is continuously withdrawn. Esterification is performed until a hydroxyl value (OHV) below 0,1 mg KOH / g is reached.

Then, the excess of 2-octenoic acid is removed using a short-path-distillation (<< 180 °C, << 10 mbar, preferably 120 to 130 °C, < 2 mbar). The triglyceride of 2-octenoic acid is obtained.

Subsequently, the triglyceride of 2-octenoic acid is hydrogenated using a usual hydrogenation catalyst (i.e. transition metal catalyst, e.g. Pd, Pt, Ru, Rh, Ni etc., preferably on a catalyst support) with concentrations of ≤ 10 % by weight, based on the triglyceride of 2-octenoic acid. Hydrogenation is carried out at temperatures between 50 °C and 180 °C, preferably between 80 °C and 120 °C, and at a hydrogen pressure below 50 bar, preferably between 5 bar and 30 bar. Hydrogenation is continued until the iodine value of the reaction mixture is below 1.0 g iodine / 100 g. Afterwards, the catalyst is filtered out and the reaction product is chemically washed/treated and then treated with bleaching earth/activated carbon which is afterwards filtered out. The chemical washing/treatment is performed as described hereinabove with sodium hydrogen sulfite (NaHSOs) in the form of a water-based solution (80 °C, 6 hours).

Subsequently, the product is deodorized using pressurized steam (HPW-steam) to remove any residual traces of 2-octenoic acid at temperatures between 120 °C and below 180 °C and at a pressure of ≤ 10 mbar.

The resulting tricaprylin product after treatment with sodium hydrogen sulfite (NaHSOs) product is analyzed as follows:
- acid value (Ph. Eur. 2.5.1): ≤ 0.1 mg KOH / g,
- hydroxyl value (Ph. Eur. 2.5.3): ≤ 0.1 mg KOH / g,
- triglyceride content (Ph. Eur. 2.2.28): ≥ 99.5 % based on tricaprylin,
- total content of MCPD fatty acid esters (DGF C-VI 18 (10)): ≤ 0.1 ppm based on tricaprylin,
- total content of glycidol esters of fatty acids (DGF C-VI 18 (10)): ≤ 0.1 ppm based on tricaprylin.

The aforedescribed production procedure is repeated in an analogous manner, however, using different starting materials, namely 4-octenoic acid and 7-octenoid acid and a triple mixture of 2-octenoid acid, 4-octenoic acid and 7-octenoid acid. In each approach comparable results are obtained.

In an analogous manner, also triheptanoin, on the one hand, and a medium-chain triglyceride (MCT) on the basis of caproic acid (C₆) / caprylic acid (Cs) / capric acid (C₁₀), on the other hand, are produced each with comparable results (characterization after treatment with hydrogen sulfite both for triheptanoin and for MCT each: acid value: ≤ 0.1 mg KOH / g; hydroxyl value: ≤ 0.1 mg KOH / g; triglyceride content: ≥ 99.5 %; total content of MCPD fatty acid esters: ≤ 0.1 ppm; total content of glycidol esters: ≤ 0.1 ppm).

### Inventive feature and/or measurement (i): Selective derivatization / Treatment with at least one nucleophilic agent

To show the effectiveness of the inventive measurement (i), especially the selective derivatization of halide-based impurities, i.e. the selective removal of halide-based impurities (especially MCPD and glycidol fatty acid esters), commercially available triglycerides are subjected to the treatment according to inventive measurement (i).

A commercial product on the basis of a MCT oil (triglyceride) has a glycidol ester content of 70 ppm. After 6 hours of treatment with aqueous sodium hydrogen sulfite at 80 °C the glycidol ester content is reduced to 9.11 ppm; thus, a reduction by a factor of about 7.5 is achieved.

Another commercial product on the basis of a MCT oil (triglyceride) (MCT 60/40) has a 3-MCPD fatty acid ester content of 8 ppm. After 6 hours of treatment with aqueous sodium hydrogen sulfite at 80 °C the 3-MCPD content is reduced to 2.21 ppm; thus, a reduction by a factor of about 3.5 is achieved.

A further commercial product (Miglyol^{®} 812N, Cs / C₁₀ - triglyceride) has a 3-MCPD fatty acid ester content of 1.56 ppm. After 15 hours of treatment with 12 % aqueous sodium hydrogen sulfite at 80 °C the 3-MCPD content is reduced to 0.16 ppm; thus, a reduction by a factor of about 9.7 is achieved.

Comparable results are reached when, instead of sodium hydrogen sulfite (NaHSOs), also other nucleophilic agents are used (i.e. sodium hydrogen thiosulfate NaHS₂O₃, sodium hydrogen phosphite NaH₂PO₃ or phosphines). The obtained results are comparable.

Thus, the inventive feature and/or measurement (i) effectively reduces the content of halide-based impurities, especially MCPD and glycidol fatty acid ester. However, only on the basis of inventive measurement (i) alone, it is not always sufficient to fulfil the overall product specifications comprising a fivefold purity / grade requirement with aforedefined items (1) to (5), especially not when starting form commercial or prior art triglycerides with low purity grades; however, especially at least the features of (4) the total content of MCPD fatty acid esters below 0.1 ppm and (5) the total content of glycidol esters below 0.1 ppm can be efficiently adjusted and/or effected by this measurement (i).

### Conclusions

Applicant has been able to provide a universal concept / process for producing high-purity tricaprylin (but also other triglycerides of fatty acids, which can then be used for producing mixtures with tricaprylin) with high yields and high conversion rates, which concept / process is independent from any specific synthesis route. Thus, applicant's concept is universal to be applied for this purpose.

The inventive process is at the same time highly flexible and compatible with known prior art production processes and plants for these substances since the aforenamed inventive features and/or measurements (i) to (iv) can be easily implemented in or used to modify known production processes and plants for these substances.

Above all, the inventive process produces tricaprylin (but also other triglycerides of fatty acids, which can then be used for producing mixtures with tricaprylin) with a high purity degree, which at the same time unify a combination of at least five purity properties, namely: (1) a very low acid value (AV), (2) a very low hydroxyl value (OHV), (3) a very high triglyceride content, (4) a very low total content of MCPD fatty acid esters and (5) a very low total content of glycidol esters of fatty acids, wherein this purity specification can be reached independently from any specific synthesis route. Such combination of purity properties (1) to (5) was deemed incompatible or non-realizable so far; however, as applicant has surprisingly found out, the inventive process renders such combination of purity properties possible for the very first time.

Furthermore, nucleophilic agents, such as e.g. hydrogen sulfites (bisulfites, HSO₃⁻), sulfites (SO₃²⁻), hydrogen thiosulfates (HS₂O₃⁻), thiosulfates (S₂O₃²⁻), hydrogen phosphites (H₂PO₃⁻), phosphites (HPO₃²⁻) and phosphines as well as combinations thereof, are an excellent reactant agent for purifying tricaprylin, especially for removing halide-based impurities, especially chlorine-based impurities, from tricaprylin, particularly chlorine-based impurities selected from the group consisting of MCPD fatty acid esters (fatty acid esters of monochloropropanediols), glycidol esters of fatty acids and combinations thereof (i.e. selective derivatization of halide-based impurities).

**The present invention is also represented and disclosed by the following aspects:**

### Aspects of the present invention:

### Aspect No. 1:

1. A process for producing tricaprylin (i.e. trioctanoylglyceride or tricaprylyl glycerol), especially with a high purity degree,
   wherein 1,2,3-propantriol (glycerol, glycerine) of formula (I)

      CH₂(OH) - CH(OH) - CH₂(OH) (I)
   or its protected form or precursor, preferably solketal (isopropylidene glycerol), is used as starting material and is reacted and/or converted to tricaprylin,
   so as to produce, as a reaction product, tricaprylin of formula (II)

      CH₂[O - C(O)R¹] - CH [O - C(O)R¹] - CH₂[O - C(O)R¹] (II)
   wherein in formula (II) the radical R¹ represents a linear saturated aliphatic C₇-alkyl radical of formula - (CH₂)₆ - CH₃ (i.e. a n-heptylic radical),
   wherein the process comprises at least one esterification step, especially an esterification step using C₈-fatty acids, especially linear saturated or mono- or poly-unsaturated C₈-fatty acids, and/or their anhydrides and/or esters (preferably C₁-C₄-alkyl esters), preferably at least one esterification step using caprylic acid (i.e. n-octylic acid or 1-heptanecarboxlic acid) of formula CH₃ - (CH₂)₆ - COOH and/or its anhydride or esters (preferably C₁-C₄-alkyl esters);
   wherein the process is characterized by at least one of the following features and/or measurements (i) to (iv), especially by a combination of at least two, preferably by a combination of at least three, more preferably by a combination of all four, of the following features and/or measurements (i) to (iv):
      (i) a preferably selective derivatization of halide-based impurities, especially of MCPD fatty acid esters (fatty acid esters of monochloropropanediols, particularly MCPD caprylic acid esters) and glycidol esters of fatty acids (particularly glycidol caprylic acid esters), present in the resulting reaction product is performed and/or the resulting reaction product is treated and/or contacted with at least one nucleophilic agent, especially with a nucleophilic agent capable of removing halide-based impurities, preferably with a nucleophilic agent selected from the group consisting of hydrogen sulfites (bisulfites, HSO₃⁻), sulfites (SO₃²⁻), hydrogen thiosulfates (HS₂O₃⁻), thiosulfates (S₂O₃²⁻), hydrogen phosphites (H₂PO₃⁻), phosphites (HPO₃²⁻) and phosphines as well as combinations thereof, more preferably with hydrogen sulfites (bisulfites, HSO₃⁻), in particular for a preferably selective derivatization of halide-based impurities, especially of MCPD fatty acid esters (fatty acid esters of monochloropropanediols) and glycidol esters of fatty acids;
      (ii) the at least one esterification step is run and/or performed at temperatures not exceeding 180 °C, especially not exceeding 150 °C, particularly not exceeding 120 °C, preferably not exceeding 100 °C, more preferably not exceeding 80 °C, even more preferably not exceeding 50 °C;
      (iii) the 1,2,3-propantriol of formula (I) or its protected form, preferably solketal (isopropylidene glycerol), used as starting material is at least essentially free of any chlorinated species and particularly comprises a total chlorinated species content of at most 1 ppm, especially at most 0.5 ppm, preferably at most 0.1 ppm, based on the 1,2,3-propantriol of formula (I) or its protected form, preferably solketal (isopropylidene glycerol);
      (iv) the at least one esterification step is run and/or performed in the absence of any metal-based catalyst.

### Aspect No. 2:

2. The process according to Aspect 1,
wherein the reaction product, especially tricaprylin of formula (II)

   CH₂[O - C(O)R¹] - CH [O - C(O)R¹] - CH₂[O - C(O)R¹] (II)
wherein in formula (II) the radical R¹ represents a linear saturated aliphatic C₇-alkyl radical of formula - (CH₂)₆ - CH₃ (i.e. a n-heptylic radical),
is characterized by and/or exhibits the following properties (1) to (5) in their combination and/or fulfils the following product specification:
   (1) acid value (AV), especially determined according to Ph. Eur. 2.5.1 (European Pharmacopoeia 10.0): ≤ 0.5 mg KOH / g, especially ≤ 0.2 mg KOH / g, preferably ≤ 0.1 mg KOH / g; and
   (2) hydroxyl value (OHV), especially determined according to Ph. Eur. 2.5.3 (European Pharmacopoeia 10.0): ≤ 0.5 mg KOH / g, especially ≤ 0.2 mg KOH / g, preferably ≤ 0.1 mg KOH / g; and
   (3) triglyceride content, especially determined according to Ph. Eur. 2.2.28 (European Pharmacopoeia 10.0): ≥ 99 %, especially ≥ 99.2 %, preferably ≥ 99.5 %, each based on the reaction product; and
   (4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.5 ppm, especially ≤ 0.2 ppm, preferably ≤ 0.1 ppm, each based on the reaction product; and
   (5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.5 ppm, especially ≤ 0.2 ppm, preferably ≤ 0.1 ppm, each based on the reaction product; and/or
wherein the process is performed such and/or the features and/or measurements (i) to (iv), especially the combination of at least two, preferably at least three, of the features and/or measurements (i) to (iv), are applied and/or performed with the proviso that the reaction product, especially tricaprylin of formula (II)

   CH₂[O - C(O)R¹] - CH [O - C(O)R¹] - CH₂[O - C(O)R¹] (II)
wherein in formula (II) the radical R¹ represents a linear saturated aliphatic C₇-alkyl radical of formula - (CH₂)₆ - CH₃ (i.e. a n-heptylic radical),
is characterized by and/or exhibits the following properties (1) to (5) in their combination and/or fulfils the following product specification:
   (1) acid value (AV), especially determined according to Ph. Eur. 2.5.1 (European Pharmacopoeia 10.0): ≤ 0.5 mg KOH / g, especially ≤ 0.2 mg KOH / g, preferably ≤ 0.1 mg KOH / g; and
   (2) hydroxyl value (OHV), especially determined according to Ph. Eur. 2.5.3 (European Pharmacopoeia 10.0): ≤ 0.5 mg KOH / g, especially ≤ 0.2 mg KOH / g, preferably ≤ 0.1 mg KOH / g; and
   (3) triglyceride content, especially determined according to Ph. Eur. 2.2.28 (European Pharmacopoeia 10.0): ≥ 99 %, especially ≥ 99.2 %, preferably ≥ 99.5 %, each based on the reaction product; and
   (4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.5 ppm, especially ≤ 0.2 ppm, preferably ≤ 0.1 ppm, each based on the reaction product; and
   (5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.5 ppm, especially ≤ 0.2 ppm, preferably ≤ 0.1 ppm, each based on the reaction product.

### Aspect No. 3:

3. A process for producing tricaprylin (i.e. trioctanoylglyceride or tricaprylyl glycerol), especially with a high purity degree, especially the process according to Aspect 1 or Aspect 2,
wherein 1,2,3-propantriol (glycerol, glycerine) of formula (I)

   CH₂(OH) - CH(OH) - CH₂(OH) (I)
or its protected form or precursor, preferably solketal (isopropylidene glycerol), is used as starting material and is reacted and/or converted to tricaprylin,
so as to produce, as a reaction product, tricaprylin of formula (II)

   CH₂[O - C(O)R¹] - CH [O - C(O)R¹] - CH₂[O - C(O)R¹] (II)
wherein in formula (II) the radical R¹ represents a linear saturated aliphatic C₇-alkyl radical of formula - (CH₂)₆ - CH₃ (i.e. a n-heptylic radical),
wherein the process comprises at least one esterification step, especially an esterification step using C₈-fatty acids, especially linear saturated or mono- or poly-unsaturated C₈-fatty acids, and/or their anhydrides and/or esters (preferably C₁-C₄-alkyl esters), preferably at least one esterification step using caprylic acid (i.e. n-octylic acid or 1-heptanecarboxlic acid) of formula CH₃- (CH₂)₆ - COOH and/or its anhydride or esters (preferably C₁-C₄-alkyl esters);
wherein the process is characterized by at least one of the following features and/or measurements (i) to (iv), especially by a combination of at least two, preferably by a combination of at least three, more preferably by a combination of all four, of the following features and/or measurements (i) to (iv):
   (i) a preferably selective derivatization of halide-based impurities, especially of MCPD fatty acid esters (fatty acid esters of monochloropropanediols, particularly MCPD caprylic acid esters) and glycidol esters of fatty acids (particularly glycidol caprylic acid esters), present in the resulting reaction product is performed and/or the resulting reaction product is treated and/or contacted with at least one nucleophilic agent, especially with a nucleophilic agent capable of removing halide-based impurities, preferably with a nucleophilic agent selected from the group consisting of hydrogen sulfites (bisulfites, HSO₃), sulfites (SO₃²), hydrogen thiosulfates (HS₂O₃⁻), thiosulfates (S₂O₃²⁻), hydrogen phosphites (H₂PO₃⁻), phosphites (HPO₃²⁻) and phosphines as well as combinations thereof, more preferably with hydrogen sulfites (bisulfites, HSO₃⁻), in particular for a preferably selective derivatization of halide-based impurities, especially of MCPD fatty acid esters (fatty acid esters of monochloropropanediols) and glycidol esters of fatty acids;
   (ii) the at least one esterification step is run and/or performed at temperatures not exceeding 180 °C, especially not exceeding 150 °C, particularly not exceeding 120 °C, preferably not exceeding 100 °C, more preferably not exceeding 80 °C, even more preferably not exceeding 50 °C;
   (iii) the 1,2,3-propantriol of formula (I) or its protected form, preferably solketal (isopropylidene glycerol), used as starting material is at least essentially free of any chlorinated species and particularly comprises a total chlorinated species content of at most 1 ppm, especially at most 0.5 ppm, preferably at most 0.1 ppm, based on the 1,2,3-propantriol of formula (I) or its protected form, preferably solketal (isopropylidene glycerol);
   (iv) the at least one esterification step is run and/or performed in the absence of any metal-based catalyst,
wherein the process is performed such and/or the features and/or measurements (i) to (iv), especially the combination of at least two, preferably at least three, of the features and/or measurements (i) to (iv), are applied and/or performed with the proviso that the reaction product, especially tricaprylin of above formula (II), is characterized by and/or exhibits the following properties (1) to (5) in their combination and/or fulfils the following product specification:
   (1) acid value (AV), especially determined according to Ph. Eur. 2.5.1 (European Pharmacopoeia 10.0): ≤ 0.5 mg KOH / g, especially ≤ 0.2 mg KOH / g, preferably ≤ 0.1 mg KOH / g; and
   (2) hydroxyl value (OHV), especially determined according to Ph. Eur. 2.5.3 (European Pharmacopoeia 10.0): ≤ 0.5 mg KOH / g, especially ≤ 0.2 mg KOH / g, preferably ≤ 0.1 mg KOH / g; and
   (3) triglyceride content, especially determined according to Ph. Eur. 2.2.28 (European Pharmacopoeia 10.0): ≥ 99 %, especially ≥ 99.2 %, preferably ≥ 99.5 %, each based on the reaction product; and
   (4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.5 ppm, especially ≤ 0.2 ppm, preferably ≤ 0.1 ppm, each based on the reaction product; and
   (5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.5 ppm, especially ≤ 0.2 ppm, preferably ≤ 0.1 ppm, each based on the reaction product.

### Aspect No. 4:

4. The process according to any of the preceding aspects,
wherein the process is performed such and/or the features and/or measurements (i) to (iv), especially the combination of at least two, preferably at least three, of the features and/or measurements (i) to (iv), are applied and/or performed with the proviso that the reaction product, especially tricaprylin of above formula (II), is characterized by and/or exhibits the following properties (1) to (5) in their combination and/or fulfils the following product specification:
(1) acid value (AV), especially determined according to Ph. Eur. 2.5.1 (European Pharmacopoeia 10.0): ≤ 0.1 mg KOH / g; and
(2) hydroxyl value (OHV), especially determined according to Ph. Eur. 2.5.3 (European Pharmacopoeia 10.0): ≤ 0.1 mg KOH / g; and
(3) triglyceride content, especially determined according to Ph. Eur. 2.2.28 (European Pharmacopoeia 10.0): ≥ 99.5 %, especially ≥ 99.6 %, preferably ≥ 99.8 %, each based on the reaction product; and
(4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.1 ppm based on the reaction product; and
(5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.1 ppm based on the reaction product.

### Aspect No. 5:

5. The process according to any of the preceding aspects,
wherein in the at least one esterification step caprylic acid of formula CH₃ - (CH₂)₆ - COOH and/or its anhydride or esters (preferably C₁-C₄-alkyl esters) is used.

### Aspect No. 6:

6. The process according to any of Aspects 1 to 5,
wherein the reaction product is tricaprylin; and/or
wherein in above formula (II) the radical R¹ represents a linear saturated aliphatic C₇-alkyl radical; and/or
wherein in the at least one esterification step octanoic acid (caprylic acid) and/or its anhydride and/or esters (preferably C₁-C₄-alkyl esters) is used.

### Aspect No. 7:

7. The process according to any of Aspects 1 to 6,
wherein the process is characterized by a combination of at least two, preferably by a combination of at least three, more preferably by a combination of all four, of the features and/or measurements (i) to (iv).

### Aspect No. 8:

8. The process according to any of Aspects 1 to 6,
wherein the process is characterized by a combination of at least two, preferably by a combination of at least three, of the features and/or measurements (ii) to (iv), optionally in further combination with feature and/or measurement (i).

### Aspect No. 9:

9. The process according to any of Aspects 1 to 6,
wherein the process is characterized by a combination of feature and/or measurement (i) with at least two of the features and/or measurements (ii) to (iv).

### Aspect No. 10:

10. The process according to any of Aspects 1 to 6,
wherein the process is characterized by a combination of all of features and/or measurements (i) to (iv).

### Aspect No. 11:

11. The process according to any of the preceding aspects,
wherein the whole and/or overall process, especially also including optional pre-treatment and/or post-treatment steps if any, is run and/or performed at temperatures not exceeding 180 °C, especially not exceeding 160 °C, particularly not exceeding 150 °C, preferably not exceeding 120 °C.

### Aspect No. 12:

12. The process according to any of the preceding aspects,
wherein the process is characterized by further feature and/or measurement (v):
(v) the whole and/or overall process, especially also including optional pre-treatment and/or post-treatment steps if any, is run and/or performed at temperatures not exceeding 180 °C, especially not exceeding 160 °C, particularly not exceeding 150 °C, preferably not exceeding 120 °C.

### Aspect No. 13:

13. The process according to any of the preceding aspects,
wherein the whole and/or overall process, especially also including optional pre-treatment and/or post-treatment steps if any, is run and/or performed in the absence of any metal-based catalyst.

### Aspect No. 14:

14. The process according to any of the preceding aspects,
wherein the process is characterized by further feature and/or measurement (vi):
(vi) the whole and/or overall process, especially also including optional pre-treatment and/or post-treatment steps if any, is run and/or performed in the absence of any metal-based catalyst.

### Aspect No. 15:

15. The process according to any of the preceding aspects,
wherein the at least one esterification step is performed and/or carried out as a one-stage esterification or, alternatively, as a two-stage or multistage esterification.

### Aspect No. 16:

16. The process according to any of the preceding aspects,
wherein the at least one esterification step is performed and/or carried out as a one-stage esterification, using anhydrides of C₈-fatty acids, especially caprylic acid anhydride, to be reacted with 1,2,3-propantriol of formula (I) or its protected form or precursor, preferably solketal, or, alternatively, wherein the at least one esterification step is performed and/or carried out as a two-stage or multistage esterification, in a first stage using C₈-fatty acids, especially caprylic acid, and/or their esters to be reacted with the 1,2,3-propantriol of formula (I) or its protected form or precursor, preferably solketal, followed in a subsequent stage by further esterification, especially further esterification of free and/or remaining OH-groups, preferably using anhydrides of C₈-fatty acids, especially caprylic acid anhydride.

### Aspect No. 17:

17. The process according to any of the preceding aspects,
wherein the at least one esterification step is performed and/or carried out in the absence of solvents and/or without any solvent.

### Aspect No. 18:

18. The process according to any of the preceding aspects,
wherein the at least one esterification step is performed and/or carried out at temperatures in the range of from 5 °C to 95 °C, especially in the range of from 10 °C to 85 °C, preferentially in the range of from 15 °C to 80 °C, more preferably in the range of from 20 °C to 75 °C, even more preferably in the range of from 25 °C to 70 °C; and/or
wherein the at least one esterification step is performed and/or carried out at a pressure in the range of from 0.0001 bar to 10 bar, especially in the range of from 0.001 bar to 5 bar, preferentially in the range of from 0.01 bar to 2 bar, more preferably in the range of from 0.05 bar to 1 bar, even more preferably at about 1 bar.

### Aspect No. 19:

19. The process according to any of the preceding aspects,
wherein the at least one esterification step is performed and/or carried out in the absence of any catalyst or, alternatively, wherein the at least one esterification step is performed and/or carried out at least partially in the presence of an enzyme as a catalyst and/or at least partially in the presence of an enzyme-based catalyst;
especially wherein the catalyst is recycled after the at least one esterification step.

### Aspect No. 20:

20. The process according to any of the preceding aspects,
wherein the at least one esterification step is performed and/or carried out in the absence of any catalyst when using anhydrides of C₈-fatty acids, especially caprylic acid anhydride, to be reacted with 1,2,3-propantriol of formula (I) or its protected form or precursor, preferably solketal, or, alternatively, wherein the at least one esterification step is performed and/or carried out at least partially in the presence of an enzyme as a catalyst and/or at least partially in the presence of an enzyme-based catalyst when using C₈-fatty acids, especially caprylic acid, and/or their esters to be reacted with the 1,2,3-propantriol of formula (I) or its protected form or precursor, preferably solketal;
especially wherein the catalyst is recycled after the at least one esterification step.

### Aspect No. 21:

21. The process according to any of the preceding aspects,
wherein the at least one esterification step is performed and/or carried out at least partially in the presence of an enzyme as a catalyst and/or at least partially in the presence of an enzyme-based catalyst;
especially wherein the enzyme is selected from synthetases (ligases), catalases, esterases, lipases and combinations thereof; and/or
especially wherein the enzyme is derived from Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar and Pseudomonas sp. and combinations thereof, preferably from Candida antarctica, Mucor miehei (Rhizomucor miehei) and Thermomyces lanuginosus, more preferably from Candida antarctica; and/or
especially wherein the enzyme is used in immobilized form, especially immobilized on a carrier, preferentially on a polymeric carrier, preferably on a polymeric organic carrier, more preferably with hydrophobic properties, even more preferably on a poly(meth)acrylic resin-based carrier; and/or
especially wherein the enzyme is recycled after the at least one esterification step; and/or
especially wherein the at least one esterification step is performed and/or carried out at temperatures in the range of from 5 °C to 95 °C, especially in the range of from 10 °C to 85 °C, preferentially in the range of from 15 °C to 80 °C, more preferably in the range of from 20 °C to 75 °C, even more preferably in the range of from 25 °C to 70 °C; and/or
especially wherein the enzyme is used in amounts, based on the total amount of all starting compounds, in the range of from 0.0001 to 25 % by weight, especially in the range of from 0.001 to 20 % by weight, preferentially in the range of from 0.01 to 15 % by weight, preferably in the range of from 0.05 to 10 % by weight; and/or
especially wherein the enzyme is used in amounts, based on the total amount of all starting compounds, in the range of from 1 • 10⁻⁶ to 5 mol-%, especially in the range of from 1 • 10⁻⁵ to 2.5 mol-%, preferentially in the range of from 1 • 10⁻⁴ to 1.5 mol-%, preferably in the range of from 1 • 10⁻³ to 1 mol-%; and/or
especially wherein the at least one esterification step is performed and/or carried out at a pressure in the range of from 0.0001 bar to 10 bar, especially in the range of from 0.001 bar to 5 bar, preferentially in the range of from 0.01 bar to 2 bar, more preferably in the range of from 0.05 bar to 1 bar, even more preferably at about 1 bar.

### Aspect No. 22:

22. The process of any of the preceding aspects,
wherein the reaction product, especially tricaprylin, is produced via one of the following synthesis routes (A) to (F):
(A) wherein, according to a (first) Synthesis Route (A), 1,2,3-propantriol (glycerol, glycerine) of formula (I)

   CH₂(OH) - CH(OH) - CH₂(OH) (I)

   is first reacted, preferably in the presence of an enzyme as a catalyst and/or in the presence of an enzyme-based catalyst (especially as defined hereinabove), with caprylic acid or its ester, preferably so as to produce a diglyceride, more preferably a 1,3-diglyceride,
   followed by subsequent reaction, preferably in the absence of any catalyst, with caprylic acid anhydride,
   so as to produce the reaction product of formula (II) as defined hereinabove, especially tricaprylin;
   or else
(B) wherein, according to a (second) Synthesis Route (B), 1,2,3-propantriol (glycerol, glycerine) of formula (I)

   CH₂(OH) - CH(OH) - CH₂(OH) (I)

   is first reacted, preferably in the presence of an enzyme as a catalyst and/or in the presence of an enzyme-based catalyst (especially as defined hereinabove), with caprylic acid or its ester, preferably so as to produce a diglyceride, more preferably a 1,3-diglyceride,
   followed by subsequent reaction, preferably in the presence of an enzyme as a catalyst and/or in the presence of an enzyme-based catalyst (more preferably an enzyme for regioselective esterification of the 2-position of 1,3-diglyceride), with caprylic acid or its ester,
   so as to produce the reaction product of formula (II) as defined hereinabove, especially tricaprylin;
   or else
(C) wherein, according to a (third) Synthesis Route (C), 1,2,3-propantriol (glycerol, glycerine) of formula (I)

   CH₂(OH) - CH(OH) - CH₂(OH) (I)

   is reacted, preferably in the absence of any catalyst, with caprylic acid anhydride,
   so as to produce the reaction product of formula (II) as defined hereinabove, especially tricaprylin;
   or else
(D) wherein, according to a (fourth) Synthesis Route (D), a protected form or precursor of 1,2,3-propantriol of above formula (I), preferably solketal (isopropylidene glycerol), is reacted, preferably in the absence of any catalyst, with caprylic acid anhydride,
   especially wherein a deprotection reaction, especially ring-opening (preferably acid-induced) and optionally removal of acetone, is performed after monoesterification of the free OH-group of the protected form or precursor of 1,2,3-propantriol of above formula (I), followed by further esterification with caprylic acid anhydride,
   so as to produce the reaction product of formula (II) as defined hereinabove, especially tricaprylin;
   or else
(E) wherein, according to a (fifth) Synthesis Route (E), a protected form or precursor of 1,2,3-propantriol of above formula (I), preferably solketal (isopropylidene glycerol), is reacted, preferably in the presence of an enzyme as a catalyst and/or in the presence of an enzyme-based catalyst (especially as defined hereinabove), with caprylic acid or its ester, followed by deprotection reaction, especially ring-opening (preferably acid-induced) and removal of acetone, preferably so as to produce a monoglyceride, more preferably a terminal monoglyceride and/or 1-monoglyceride,
   followed by subsequent reaction, preferably in the absence of any catalyst, with caprylic acid anhydride,
   so as to produce the reaction product of formula (II) as defined hereinabove, especially tricaprylin;
   or else
(F) wherein, according to a (sixth) Synthesis Route (F), 1,2,3-propantriol (glycerol, glycerine) of formula (I)

   CH₂(OH) - CH(OH) - CH₂(OH) (I)

   is reacted, optionally in the presence of an enzyme as a catalyst and/or in the presence of an enzyme-based catalyst (especially as defined hereinabove), with at least one linear mono- or poly-unsaturated (preferably mono-unsaturated) aliphatic C₈-fatty acid and/or its esters and/or anhydrides, preferably with at least one linear mono-unsaturated aliphatic C₈-fatty acid and/or its esters and/or anhydrides, preferably so as to produce triglycerides of linear mono- or poly-unsaturated (preferably mono-unsaturated) aliphatic C₈-fatty acids,
   followed by subsequent hydrogenation reaction, preferably in the presence of at least one hydrogenation catalyst, preferably so as to hydrogenate the double bonds of the linear mono- or poly-unsaturated (preferably mono-unsaturated) aliphatic C₈-fatty acid radicals of the triglycerides,
   so as to produce the reaction product of formula (II) as defined hereinabove, especially tricaprylin.

### Aspect No. 23:

23. A tricaprylin obtainable or obtained by the process according to any of the preceding aspects.

24. A tricaprylin, especially a tricaprylin according to Aspect 23,
wherein the tricaprylin corresponds to formula (II)

   CH₂[O - C(O)R¹] - CH[O - C(O)R¹] - CH₂[O - C(O)R¹] (II)
wherein in formula (II) the radical R¹ represents a linear saturated aliphatic C₇-alkyl radical of formula - (CH₂)₆ - CH₃ (i.e. a n-heptylic radical);
wherein the tricaprylin of formula (II) is characterized by and/or exhibits the following properties (1) to (5) in their combination and/or fulfils the following product specification:
   (1) acid value (AV), especially determined according to Ph. Eur. 2.5.1 (European Pharmacopoeia 10.0): ≤ 0.5 mg KOH / g, especially ≤ 0.2 mg KOH / g, preferably ≤ 0.1 mg KOH / g; and
   (2) hydroxyl value (OHV), especially determined according to Ph. Eur. 2.5.3 (European Pharmacopoeia 10.0): ≤ 0.5 mg KOH / g, especially ≤ 0.2 mg KOH / g, preferably ≤ 0.1 mg KOH / g; and
   (3) triglyceride content, especially determined according to Ph. Eur. 2.2.28 (European Pharmacopoeia 10.0): ≥ 99 %, especially ≥ 99.2 %, preferably ≥ 99.5 %, each based on the tricaprylin; and
   (4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.5 ppm, especially ≤ 0.2 ppm, preferably ≤ 0.1 ppm, each based on the tricaprylin; and
   (5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.5 ppm, especially ≤ 0.2 ppm, preferably ≤ 0.1 ppm, each based on the tricaprylin.

### Aspect No. 25:

25. The tricaprylin according to Aspect 23 or Aspect 24,
wherein the tricaprylin of formula (II) is characterized by and/or exhibits the following properties (1) to (5) in their combination and/or fulfils the following product specification:
(1) acid value (AV), especially determined according to Ph. Eur. 2.5.1 (European Pharmacopoeia 10.0): ≤ 0.1 mg KOH / g; and
(2) hydroxyl value (OHV), especially determined according to Ph. Eur. 2.5.3 (European Pharmacopoeia 10.0): ≤ 0.1 mg KOH / g; and
(3) triglyceride content, especially determined according to Ph. Eur. 2.2.28 (European Pharmacopoeia 10.0): ≥ 99.5 %, especially ≥ 99.6 %, preferably ≥ 99.8 %, each based on the tricaprylin; and
(4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.1 ppm based on the tricaprylin; and
(5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.1 ppm based on the tricaprylin.

### Aspect No. 26:

26. A pharmaceutical composition, especially a drug or medicament, comprising a tricaprylin according to any of Aspects 23 to 25, especially together with a physiologically acceptable excipient.

### Aspect No. 27:

27. The pharmaceutical composition according to Aspect 26 for the prophylactic and/or therapeutic treatment or for use in the prophylactic and/or therapeutic treatment of diseases of the human or animal body, especially diseases associated with a disorder of the energy metabolism, especially ketone body metabolism, such as especially craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, fat metabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, especially ulcerative colitis and Crohn's disease, lyosomal storage diseases such as sphingolipidosis, especially Niemann-Pick disease, diabetes mellitus, migraine and migraine headaches, epilepsy, especially infantile spasms as well as effects or side-effects of chemotherapy.

### Aspect No. 28:

28. The pharmaceutical composition according to Aspect 26 or Aspect 27 for the prophylactic and/or therapeutic treatment or for use in the prophylactic and/or therapeutic treatment of diseases of the human body selected among Alzheimer's disease, especially mild to moderate forms of Alzheimer's disease; migraine and migraine headaches; as well as epilepsy, especially infantile spasms.

### Aspect No. 29:

29. A tricaprylin according to any of Aspects 23 to 25 for the prophylactic and/or therapeutic treatment or for use in the prophylactic and/or therapeutic treatment of diseases of the human or animal body, especially diseases associated with a disorder of the energy metabolism, especially ketone body metabolism, such as especially craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, fat metabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, especially ulcerative colitis and Crohn's disease, lyosomal storage diseases such as sphingolipidosis, especially Niemann-Pick disease, diabetes mellitus, migraine and migraine headaches, epilepsy, especially infantile spasms as well as effects or side-effects of chemotherapy.

### Aspect No. 30:

30. A tricaprylin according to any of Aspects 23 to 25 for the prophylactic and/or therapeutic treatment or for use in the prophylactic and/or therapeutic treatment of diseases of the human body selected among Alzheimer's disease, especially mild to moderate forms of Alzheimer's disease; migraine and migraine headaches; as well as epilepsy, especially infantile spasms.

### Aspect No. 31:

31. Use of a tricaprylin according to any of Aspects 23 to 25 for the prophylactic and/or therapeutic treatment or for producing a medicament for the prophylactic and/or therapeutic treatment of diseases of the human or animal body, especially diseases associated with a disorder of the energy metabolism, especially ketone body metabolism, such as especially craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, fat metabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, especially ulcerative colitis and Crohn's disease, lyosomal storage diseases such as sphingolipidosis, especially Niemann-Pick disease, diabetes mellitus, migraine and migraine headaches, epilepsy, especially infantile spasms as well as effects or side-effects of chemotherapy.

### Aspect No. 32:

32. Use of a tricaprylin according to any of Aspects 23 to 25 for the prophylactic and/or therapeutic treatment or for producing a medicament for the prophylactic and/or therapeutic treatment of diseases of the human body selected among Alzheimer's disease, especially mild to moderate forms of Alzheimer's disease; migraine and migraine headaches; as well as epilepsy, especially infantile spasms.

### Aspect No. 33:

33. Use of a tricaprylin according to any of Aspects 23 to 25 for the prophylactic and/or therapeutic treatment or for producing a medicament for the prophylactic and/or therapeutic treatment of or for the application for catabolic metabolic states, such as hunger, diets or low-carbohydrate nutrition.

### Aspect No. 34:

34. A food and/or a nutritive or food composition and/or a food product and/or a medical food comprising a tricaprylin according to any of Aspects 23 to 25.

### Aspect No. 35:

35. The food and/or the nutritive or food composition and/or the food product and/or the medical food according to Aspect 34,
wherein food and/or the nutritive or food composition and/or the food product and/or the medical food is a dietary supplement, a functional food, a novel food, a food additive, a food supplement, a dietary food, a power snack, an appetite suppressant or a strength and/or endurance sports supplement.

### Aspect No. 36:

36. Use of a tricaprylin according to any of Aspects 23 to 25 in a food and/or in a nutritive or food composition and/or in a food product and/or in a medical food.

### Aspect No. 37:

37. The use according to Aspect 36,
wherein food and/or the nutritive or food composition and/or the food product and/or the medical food is a dietary supplement, a functional food, a novel food, a food additive, a food supplement, a dietary food, a power snack, an appetite suppressant or a strength and/or endurance sports supplement.

### Aspect No. 38:

38. Use of a tricaprylin according to any of Aspects 23 to 25 as an additive or aid, especially as a carrier or excipient, a solubilizer, a release agent, a surface-treatment agent, a transport agent, a lubricant, hydrophobic agent, a film-forming or protective agent or a viscosity regulator, preferably as a carrier or excipient, in pharmaceutical compositions, especially drugs or medicaments, in food, nutritive or food compositions, food products and/or medical foods as well as in cosmetic compositions.

### Aspect No. 39:

39. Use of a tricaprylin according to any of Aspects 23 to 25 as a carrier or excipient in pharmaceutical compositions, especially drugs or medicaments.

### Aspect No. 40:

40. Use of a tricaprylin according to any of Aspects 23 to 25 as an active substance (active ingredient) in pharmaceutical compositions, especially drugs or medicaments, in food, nutritive or food compositions, food products and/or medical foods or in cosmetic compositions, especially together with an excipient, particularly together with a physiologically acceptable excipient.

### Aspect No. 41:

41. Use of a tricaprylin according to any of Aspects 23 to 25 as an active substance (active ingredient, API) in pharmaceutical compositions, especially drugs or medicaments, especially together with a physiologically acceptable excipient.

### Aspect No. 42:

42. Use of a tricaprylin according to any of Aspects 23 to 25 as an active substance (active ingredient, API) in pharmaceutical compositions, especially drugs or medicaments, especially together with a physiologically acceptable excipient.

### Aspect No. 43:

43. Use of a nucleophilic agent for purifying a tricaprylin, especially tricaprylin of formula (II) as defined hereinabove, and/or for a preferably selective removal or derivatization of halide-based impurities present in a tricaprylin, especially a tricaprylin of formula (II) as defined hereinabove.

### Aspect No. 44:

44. The use according to Aspect 43,
wherein the nucleophilic agent is a nucleophilic agent capable of removing halide-based impurities, preferably selected from the group consisting of hydrogen sulfites (bisulfites, HSO₃), sulfites (SO₃²), hydrogen thiosulfates (HS₂O₃⁻), thiosulfates (S₂O₃²⁻), hydrogen phosphites (H₂PO₃⁻), phosphites (HPO₃²⁻) and phosphines as well as combinations thereof, more preferably hydrogen sulfites (bisulfites, HSO₃⁻); and/or
wherein the tricaprylin to be purified is treated and/or contacted with the nucleophilic agent; and/or
wherein the nucleophilic agent is used for purifying tricaprylin from halide-based, especially chlorine-based impurities and/or for removing halide-based, especially chlorine-based impurities from tricaprylin, particularly chlorine-based impurities selected from the group consisting of MCPD fatty acid esters (fatty acid esters of monochloropropanediols), glycidol esters of fatty acids and combinations thereof; and/or
wherein the halide-based impurities are chlorine-based impurities, especially chlorine-based impurities selected from the group consisting of MCPD fatty acid esters (fatty acid esters of monochloropropanediols), glycidol esters of fatty acids and combinations thereof.

### Aspect No. 45:

45. The use according to Aspect 43 or Aspect 44,
wherein the tricaprylin to be purified is treated and/or contacted with the nucleophilic agent, especially with a preferably water-based solution of the nucleophilic agent; and/or
wherein the tricaprylin to be purified is treated and/or contacted with the nucleophilic agent under reaction conditions, especially at reaction temperatures and/or for reaction durations, sufficient to remove the impurities; and/or
wherein the tricaprylin to be purified is treated and/or contacted with the nucleophilic agent at temperatures in the range of from 10 °C to 175 °C, preferably in the range of from 25 °C to 120 °C, more preferably in the range of from 50 °C to 100 °C, and/or for a duration in the range of from 0.001 to 50 hours, preferably in the range of from 0.01 to 30 hours, more preferably in the range of from 0.1 to 20 hours.

## Claims

1. A process for producing tricaprylin (i.e. trioctanoylglyceride or tricaprylyl glycerol), especially with a high purity degree,
wherein 1,2,3-propantriol (glycerol, glycerine) of formula (I)
CH₂(OH) - CH(OH) - CH₂(OH) (I)
or its protected form or precursor, preferably solketal (isopropylidene glycerol), is used as starting material and is reacted and/or converted to tricaprylin,
so as to produce, as a reaction product, tricaprylin of formula (II)
CH₂[O - C(O)R¹] - CH [O - C(O)R¹] - CH₂[O - C(O)R¹] (II)
wherein in formula (II) the radical R¹ represents a linear saturated aliphatic C₇-alkyl radical of formula - (CH₂)₆ - CH₃ (i.e. a n-heptylic radical),
wherein the process comprises at least one esterification step, especially an esterification step using C₈-fatty acids, especially linear saturated or mono- or poly-unsaturated C₈-fatty acids, and/or their anhydrides and/or esters (preferably C₁-C₄-alkyl esters), preferably at least one esterification step using caprylic acid (i.e. n-octylic acid or 1-heptanecarboxlic acid) of formula CH₃ - (CH₂)₆ - COOH and/or its anhydride or esters (preferably C₁-C₄-alkyl esters);
wherein the process is **characterized by** at least one of the following features and/or measurements (i) to (iv), especially by a combination of at least two, preferably by a combination of at least three, more preferably by a combination of all four, of the following features and/or measurements (i) to (iv):
(i) a preferably selective derivatization of halide-based impurities, especially of MCPD fatty acid esters (fatty acid esters of monochloropropanediols, particularly MCPD caprylic acid esters) and glycidol esters of fatty acids (particularly glycidol caprylic acid esters), present in the resulting reaction product is performed and/or the resulting reaction product is treated and/or contacted with at least one nucleophilic agent, especially with a nucleophilic agent capable of removing halide-based impurities, preferably with a nucleophilic agent selected from the group consisting of hydrogen sulfites (bisulfites, HSO₃⁻), sulfites (SO₃²⁻), hydrogen thiosulfates (HS₂O₃⁻), thiosulfates (S₂O₃²⁻), hydrogen phosphites (H₂PO₃⁻), phosphites (HPO₃²⁻) and phosphines as well as combinations thereof, more preferably with hydrogen sulfites (bisulfites, HSO₃⁻), in particular for a preferably selective derivatization of halide-based impurities, especially of MCPD fatty acid esters (fatty acid esters of monochloropropanediols) and glycidol esters of fatty acids;
(ii) the at least one esterification step is run and/or performed at temperatures not exceeding 180 °C, especially not exceeding 150 °C, particularly not exceeding 120 °C, preferably not exceeding 100 °C, more preferably not exceeding 80 °C, even more preferably not exceeding 50 °C;
(iii) the 1,2,3-propantriol of formula (I) or its protected form, preferably solketal (isopropylidene glycerol), used as starting material is at least essentially free of any chlorinated species and particularly comprises a total chlorinated species content of at most 1 ppm, especially at most 0.5 ppm, preferably at most 0.1 ppm, based on the 1,2,3-propantriol of formula (I) or its protected form, preferably solketal (isopropylidene glycerol);
(iv) the at least one esterification step is run and/or performed in the absence of any metal-based catalyst.

2. The process according to Claim 1,
wherein the reaction product, especially tricaprylin of formula (II)
CH₂[O - C(O)R¹] - CH [O - C(O)R¹] - CH₂[O - C(O)R¹] (II)
wherein in formula (II) the radical R¹ represents a linear saturated aliphatic C₇-alkyl radical of formula - (CH₂)₆ - CH₃ (i.e. a n-heptylic radical),
is **characterized by** and/or exhibits the following properties (1) to (5) in their combination and/or fulfils the following product specification:
(1) acid value (AV), especially determined according to Ph. Eur. 2.5.1 (European Pharmacopoeia 10.0): ≤ 0.5 mg KOH / g, especially ≤ 0.2 mg KOH / g, preferably ≤ 0.1 mg KOH / g; and
(2) hydroxyl value (OHV), especially determined according to Ph. Eur. 2.5.3 (European Pharmacopoeia 10.0): ≤ 0.5 mg KOH / g, especially ≤ 0.2 mg KOH / g, preferably ≤ 0.1 mg KOH / g; and
(3) triglyceride content, especially determined according to Ph. Eur. 2.2.28 (European Pharmacopoeia 10.0): ≥ 99 %, especially ≥ 99.2 %, preferably ≥ 99.5 %, each based on the reaction product; and
(4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.5 ppm, especially ≤ 0.2 ppm, preferably ≤ 0.1 ppm, each based on the reaction product; and
(5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.5 ppm, especially ≤ 0.2 ppm, preferably ≤ 0.1 ppm, each based on the reaction product; and/or
wherein the process is performed such and/or the features and/or measurements (i) to (iv), especially the combination of at least two, preferably at least three, of the features and/or measurements (i) to (iv), are applied and/or performed with the proviso that the reaction product, especially tricaprylin of formula (II)
CH₂[O - C(O)R¹] - CH [O - C(O)R¹] - CH₂[O - C(O)R¹] (II)
wherein in formula (II) the radical R¹ represents a linear saturated aliphatic C₇-alkyl radical of formula - (CH₂)₆ - CH₃ (i.e. a n-heptylic radical),
is **characterized by** and/or exhibits the following properties (1) to (5) in their combination and/or fulfils the following product specification:
(1) acid value (AV), especially determined according to Ph. Eur. 2.5.1 (European Pharmacopoeia 10.0): ≤ 0.5 mg KOH / g, especially ≤ 0.2 mg KOH / g, preferably ≤ 0.1 mg KOH / g; and
(2) hydroxyl value (OHV), especially determined according to Ph. Eur. 2.5.3 (European Pharmacopoeia 10.0): ≤ 0.5 mg KOH / g, especially ≤ 0.2 mg KOH / g, preferably ≤ 0.1 mg KOH / g; and
(3) triglyceride content, especially determined according to Ph. Eur. 2.2.28 (European Pharmacopoeia 10.0): ≥ 99 %, especially ≥ 99.2 %, preferably ≥ 99.5 %, each based on the reaction product; and
(4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.5 ppm, especially ≤ 0.2 ppm, preferably ≤ 0.1 ppm, each based on the reaction product; and
(5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.5 ppm, especially ≤ 0.2 ppm, preferably ≤ 0.1 ppm, each based on the reaction product.

3. A process for producing tricaprylin (i.e. trioctanoylglyceride or tricaprylyl glycerol), especially with a high purity degree, especially the process according to Claim 1 or Claim 2,
wherein 1,2,3-propantriol (glycerol, glycerine) of formula (I)
CH₂(OH) - CH(OH) - CH₂(OH) (I)
or its protected form or precursor, preferably solketal (isopropylidene glycerol), is used as starting material and is reacted and/or converted to tricaprylin,
so as to produce, as a reaction product, tricaprylin of formula (II)
CH₂[O - C(O)R¹] - CH [O - C(O)R¹] - CH₂[O - C(O)R¹] (II)
wherein in formula (II) the radical R¹ represents a linear saturated aliphatic C₇-alkyl radical of formula - (CH₂)₆ - CH₃ (i.e. a n-heptylic radical),
wherein the process comprises at least one esterification step, especially an esterification step using C₈-fatty acids, especially linear saturated or mono- or poly-unsaturated C₈-fatty acids, and/or their anhydrides and/or esters (preferably C₁-C₄-alkyl esters), preferably at least one esterification step using caprylic acid (i.e. n-octylic acid or 1-heptanecarboxlic acid) of formula CH₃- (CH₂)₆ - COOH and/or its anhydride or esters (preferably C₁-C₄-alkyl esters);
wherein the process is **characterized by** at least one of the following features and/or measurements (i) to (iv), especially by a combination of at least two, preferably by a combination of at least three, more preferably by a combination of all four, of the following features and/or measurements (i) to (iv):
(i) a preferably selective derivatization of halide-based impurities, especially of MCPD fatty acid esters (fatty acid esters of monochloropropanediols, particularly MCPD caprylic acid esters) and glycidol esters of fatty acids (particularly glycidol caprylic acid esters), present in the resulting reaction product is performed and/or the resulting reaction product is treated and/or contacted with at least one nucleophilic agent, especially with a nucleophilic agent capable of removing halide-based impurities, preferably with a nucleophilic agent selected from the group consisting of hydrogen sulfites (bisulfites, HSO₃), sulfites (SO₃²), hydrogen thiosulfates (HS₂O₃⁻), thiosulfates (S₂O₃²⁻), hydrogen phosphites (H₂PO₃⁻), phosphites (HPO₃²⁻) and phosphines as well as combinations thereof, more preferably with hydrogen sulfites (bisulfites, HSO₃⁻), in particular for a preferably selective derivatization of halide-based impurities, especially of MCPD fatty acid esters (fatty acid esters of monochloropropanediols) and glycidol esters of fatty acids;
(ii) the at least one esterification step is run and/or performed at temperatures not exceeding 180 °C, especially not exceeding 150 °C, particularly not exceeding 120 °C, preferably not exceeding 100 °C, more preferably not exceeding 80 °C, even more preferably not exceeding 50 °C;
(iii) the 1,2,3-propantriol of formula (I) or its protected form, preferably solketal (isopropylidene glycerol), used as starting material is at least essentially free of any chlorinated species and particularly comprises a total chlorinated species content of at most 1 ppm, especially at most 0.5 ppm, preferably at most 0.1 ppm, based on the 1,2,3-propantriol of formula (I) or its protected form, preferably solketal (isopropylidene glycerol);
(iv) the at least one esterification step is run and/or performed in the absence of any metal-based catalyst,
wherein the process is performed such and/or the features and/or measurements (i) to (iv), especially the combination of at least two, preferably at least three, of the features and/or measurements (i) to (iv), are applied and/or performed with the proviso that the reaction product, especially tricaprylin of above formula (II), is **characterized by** and/or exhibits the following properties (1) to (5) in their combination and/or fulfils the following product specification:
(1) acid value (AV), especially determined according to Ph. Eur. 2.5.1 (European Pharmacopoeia 10.0): ≤ 0.5 mg KOH / g, especially ≤ 0.2 mg KOH / g, preferably ≤ 0.1 mg KOH / g; and
(2) hydroxyl value (OHV), especially determined according to Ph. Eur. 2.5.3 (European Pharmacopoeia 10.0): ≤ 0.5 mg KOH / g, especially ≤ 0.2 mg KOH / g, preferably ≤ 0.1 mg KOH / g; and
(3) triglyceride content, especially determined according to Ph. Eur. 2.2.28 (European Pharmacopoeia 10.0): ≥ 99 %, especially ≥ 99.2 %, preferably ≥ 99.5 %, each based on the reaction product; and
(4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.5 ppm, especially ≤ 0.2 ppm, preferably ≤ 0.1 ppm, each based on the reaction product; and
(5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.5 ppm, especially ≤ 0.2 ppm, preferably ≤ 0.1 ppm, each based on the reaction product.

4. The process according to any of the preceding claims,
wherein the process is performed such and/or the features and/or measurements (i) to (iv), especially the combination of at least two, preferably at least three, of the features and/or measurements (i) to (iv), are applied and/or performed with the proviso that the reaction product, especially tricaprylin of above formula (II), is **characterized by** and/or exhibits the following properties (1) to (5) in their combination and/or fulfils the following product specification:
(1) acid value (AV), especially determined according to Ph. Eur. 2.5.1 (European Pharmacopoeia 10.0): ≤ 0.1 mg KOH / g; and
(2) hydroxyl value (OHV), especially determined according to Ph. Eur. 2.5.3 (European Pharmacopoeia 10.0): ≤ 0.1 mg KOH / g; and
(3) triglyceride content, especially determined according to Ph. Eur. 2.2.28 (European Pharmacopoeia 10.0): ≥ 99.5 %, especially ≥ 99.6 %, preferably ≥ 99.8 %, each based on the reaction product; and
(4) total content of MCPD fatty acid esters (fatty acid esters of monochloropropanediols), especially determined according to method of DGF C-VI 18 (10) (Einheitsmethode der Deutschen Gesellschaft für Fettwissenschaft [Standard method of German Society for Fat Science]): ≤ 0.1 ppm based on the reaction product; and
(5) total content of glycidol esters of fatty acids, especially determined according to method of DGF C-VI 18 (10) (Einheitsmethode der Deutschen Gesellschaft für Fettwissenschaft e.V. [Standard method of German Society for Fat Science]): ≤ 0.1 ppm based on the reaction product.

5. The process according to any of the preceding claims,
wherein in the at least one esterification step caprylic acid of formula CH₃- (CH₂)₆ - COOH and/or its anhydride or esters (preferably C₁-C₄-alkyl esters) is used.

6. The process according to any of Claims 1 to 5,
wherein the reaction product is tricaprylin; and/or
wherein in above formula (II) the radical R¹ represents a linear saturated aliphatic C₇-alkyl radical; and/or
wherein in the at least one esterification step octanoic acid (caprylic acid) and/or its anhydride and/or esters (preferably C₁-C₄-alkyl esters) is used.

7. The process according to any of Claims 1 to 6,
wherein the process is **characterized by** a combination of at least two, preferably by a combination of at least three, more preferably by a combination of all four, of the features and/or measurements (i) to (iv).

8. The process according to any of Claims 1 to 6,
wherein the process is **characterized by** a combination of at least two, preferably by a combination of at least three, of the features and/or measurements (ii) to (iv), optionally in further combination with feature and/or measurement (i).

9. The process according to any of Claims 1 to 6,
wherein the process is **characterized by** a combination of feature and/or measurement (i) with at least two of the features and/or measurements (ii) to (iv).

10. The process according to any of Claims 1 to 6,
wherein the process is **characterized by** a combination of all of features and/or measurements (i) to (iv).

11. The process according to any of the preceding claims,
wherein the whole and/or overall process, especially also including optional pre-treatment and/or post-treatment steps if any, is run and/or performed at temperatures not exceeding 180 °C, especially not exceeding 160 °C, particularly not exceeding 150 °C, preferably not exceeding 120 °C.

12. The process according to any of the preceding claims,
wherein the process is **characterized by** further feature and/or measurement (v):
(v) the whole and/or overall process, especially also including optional pre-treatment and/or post-treatment steps if any, is run and/or performed at temperatures not exceeding 180 °C, especially not exceeding 160 °C, particularly not exceeding 150 °C, preferably not exceeding 120 °C.

13. The process according to any of the preceding claims,
wherein the whole and/or overall process, especially also including optional pre-treatment and/or post-treatment steps if any, is run and/or performed in the absence of any metal-based catalyst.

14. The process according to any of the preceding claims,
wherein the process is **characterized by** further feature and/or measurement (vi):
(vi) the whole and/or overall process, especially also including optional pre-treatment and/or post-treatment steps if any, is run and/or performed in the absence of any metal-based catalyst.

15. The process according to any of the preceding claims,
wherein the at least one esterification step is performed and/or carried out as a one-stage esterification or, alternatively, as a two-stage or multistage esterification.

16. The process according to any of the preceding claims,
wherein the at least one esterification step is performed and/or carried out as a one-stage esterification, using anhydrides of C₈-fatty acids, especially caprylic acid anhydride, to be reacted with 1,2,3-propantriol of formula (I) or its protected form or precursor, preferably solketal, or, alternatively, wherein the at least one esterification step is performed and/or carried out as a two-stage or multistage esterification, in a first stage using C₈-fatty acids, especially caprylic acid, and/or their esters to be reacted with the 1,2,3-propantriol of formula (I) or its protected form or precursor, preferably solketal, followed in a subsequent stage by further esterification, especially further esterification of free and/or remaining OH-groups, preferably using anhydrides of C₈-fatty acids, especially caprylic acid anhydride.

17. The process according to any of the preceding claims,
wherein the at least one esterification step is performed and/or carried out in the absence of solvents and/or without any solvent.

18. The process according to any of the preceding claims,
wherein the at least one esterification step is performed and/or carried out at temperatures in the range of from 5 °C to 95 °C, especially in the range of from 10 °C to 85 °C, preferentially in the range of from 15 °C to 80 °C, more preferably in the range of from 20 °C to 75 °C, even more preferably in the range of from 25 °C to 70 °C; and/or
wherein the at least one esterification step is performed and/or carried out at a pressure in the range of from 0.0001 bar to 10 bar, especially in the range of from 0.001 bar to 5 bar, preferentially in the range of from 0.01 bar to 2 bar, more preferably in the range of from 0.05 bar to 1 bar, even more preferably at about 1 bar.

19. The process according to any of the preceding claims,
wherein the at least one esterification step is performed and/or carried out in the absence of any catalyst or, alternatively, wherein the at least one esterification step is performed and/or carried out at least partially in the presence of an enzyme as a catalyst and/or at least partially in the presence of an enzyme-based catalyst;
especially wherein the catalyst is recycled after the at least one esterification step.

20. The process according to any of the preceding claims,
wherein the at least one esterification step is performed and/or carried out in the absence of any catalyst when using anhydrides of C₈-fatty acids, especially caprylic acid anhydride, to be reacted with 1,2,3-propantriol of formula (I) or its protected form or precursor, preferably solketal, or, alternatively, wherein the at least one esterification step is performed and/or carried out at least partially in the presence of an enzyme as a catalyst and/or at least partially in the presence of an enzyme-based catalyst when using C₈-fatty acids, especially caprylic acid, and/or their esters to be reacted with the 1,2,3-propantriol of formula (I) or its protected form or precursor, preferably solketal;
especially wherein the catalyst is recycled after the at least one esterification step.

21. The process according to any of the preceding claims,
wherein the at least one esterification step is performed and/or carried out at least partially in the presence of an enzyme as a catalyst and/or at least partially in the presence of an enzyme-based catalyst;
especially wherein the enzyme is selected from synthetases (ligases), catalases, esterases, lipases and combinations thereof; and/or
especially wherein the enzyme is derived from Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar and Pseudomonas sp. and combinations thereof, preferably from Candida antarctica, Mucor miehei (Rhizomucor miehei) and Thermomyces lanuginosus, more preferably from Candida antarctica; and/or
especially wherein the enzyme is used in immobilized form, especially immobilized on a carrier, preferentially on a polymeric carrier, preferably on a polymeric organic carrier, more preferably with hydrophobic properties, even more preferably on a poly(meth)acrylic resin-based carrier; and/or
especially wherein the enzyme is recycled after the at least one esterification step; and/or
especially wherein the at least one esterification step is performed and/or carried out at temperatures in the range of from 5 °C to 95 °C, especially in the range of from 10 °C to 85 °C, preferentially in the range of from 15 °C to 80 °C, more preferably in the range of from 20 °C to 75 °C, even more preferably in the range of from 25 °C to 70 °C; and/or
especially wherein the enzyme is used in amounts, based on the total amount of all starting compounds, in the range of from 0.0001 to 25 % by weight, especially in the range of from 0.001 to 20 % by weight, preferentially in the range of from 0.01 to 15 % by weight, preferably in the range of from 0.05 to 10 % by weight; and/or
especially wherein the enzyme is used in amounts, based on the total amount of all starting compounds, in the range of from 1 • 10⁻⁶ to 5 mol-%, especially in the range of from 1 • 10⁻⁵ to 2.5 mol-%, preferentially in the range of from 1 • 10⁻⁴ to 1.5 mol-%, preferably in the range of from 1 • 10⁻³ to 1 mol-%; and/or
especially wherein the at least one esterification step is performed and/or carried out at a pressure in the range of from 0.0001 bar to 10 bar, especially in the range of from 0.001 bar to 5 bar, preferentially in the range of from 0.01 bar to 2 bar, more preferably in the range of from 0.05 bar to 1 bar, even more preferably at about 1 bar.

22. The process of any of the preceding claims,
wherein the reaction product, especially tricaprylin, is produced via one of the following synthesis routes (A) to (F):
(A) wherein, according to a (first) Synthesis Route (A), 1,2,3-propantriol (glycerol, glycerine) of formula (I)
CH₂(OH) - CH(OH) - CH₂(OH) (I)
is first reacted, preferably in the presence of an enzyme as a catalyst and/or in the presence of an enzyme-based catalyst (especially as defined hereinabove), with caprylic acid or its ester, preferably so as to produce a diglyceride, more preferably a 1,3-diglyceride,
followed by subsequent reaction, preferably in the absence of any catalyst, with caprylic acid anhydride,
so as to produce the reaction product of formula (II) as defined hereinabove, especially tricaprylin;
or else
(B) wherein, according to a (second) Synthesis Route (B), 1,2,3-propantriol (glycerol, glycerine) of formula (I)
CH₂(OH) - CH(OH) - CH₂(OH) (I)
is first reacted, preferably in the presence of an enzyme as a catalyst and/or in the presence of an enzyme-based catalyst (especially as defined hereinabove), with caprylic acid or its ester, preferably so as to produce a diglyceride, more preferably a 1,3-diglyceride,
followed by subsequent reaction, preferably in the presence of an enzyme as a catalyst and/or in the presence of an enzyme-based catalyst (more preferably an enzyme for regioselective esterification of the 2-position of 1,3-diglyceride), with caprylic acid or its ester,
so as to produce the reaction product of formula (II) as defined hereinabove, especially tricaprylin;
or else
(C) wherein, according to a (third) Synthesis Route (C), 1,2,3-propantriol (glycerol, glycerine) of formula (I)
CH₂(OH) - CH(OH) - CH₂(OH) (I)
is reacted, preferably in the absence of any catalyst, with caprylic acid anhydride,
so as to produce the reaction product of formula (II) as defined hereinabove, especially tricaprylin;
or else
(D) wherein, according to a (fourth) Synthesis Route (D), a protected form or precursor of 1,2,3-propantriol of above formula (I), preferably solketal (isopropylidene glycerol), is reacted, preferably in the absence of any catalyst, with caprylic acid anhydride,
especially wherein a deprotection reaction, especially ring-opening (preferably acid-induced) and optionally removal of acetone, is performed after monoesterification of the free OH-group of the protected form or precursor of 1,2,3-propantriol of above formula (I), followed by further esterification with caprylic acid anhydride,
so as to produce the reaction product of formula (II) as defined hereinabove, especially tricaprylin;
or else
(E) wherein, according to a (fifth) Synthesis Route (E), a protected form or precursor of 1,2,3-propantriol of above formula (I), preferably solketal (isopropylidene glycerol), is reacted, preferably in the presence of an enzyme as a catalyst and/or in the presence of an enzyme-based catalyst (especially as defined hereinabove), with caprylic acid or its ester, followed by deprotection reaction, especially ring-opening (preferably acid-induced) and removal of acetone, preferably so as to produce a monoglyceride, more preferably a terminal monoglyceride and/or 1-monoglyceride,
followed by subsequent reaction, preferably in the absence of any catalyst, with caprylic acid anhydride,
so as to produce the reaction product of formula (II) as defined hereinabove, especially tricaprylin;
or else
(F) wherein, according to a (sixth) Synthesis Route (F), 1,2,3-propantriol (glycerol, glycerine) of formula (I)
CH₂(OH)-CH(OH)-CH₂(OH) (I)
is reacted, optionally in the presence of an enzyme as a catalyst and/or in the presence of an enzyme-based catalyst (especially as defined hereinabove), with at least one linear mono- or poly-unsaturated (preferably mono-unsaturated) aliphatic Cs-fatty acid and/or its esters and/or anhydrides, preferably with at least one linear mono-unsaturated aliphatic Cs-fatty acid and/or its esters and/or anhydrides, preferably so as to produce triglycerides of linear mono- or poly-unsaturated (preferably mono-unsaturated) aliphatic Cs-fatty acids,
followed by subsequent hydrogenation reaction, preferably in the presence of at least one hydrogenation catalyst, preferably so as to hydrogenate the double bonds of the linear mono- or poly-unsaturated (preferably mono-unsaturated) aliphatic Cs-fatty acid radicals of the triglycerides,
so as to produce the reaction product of formula (II) as defined hereinabove, especially tricaprylin.
